# EUROPÄISCHE PATENTSCHRIFT

(11) **EP 1 392 660 B1**
(45) Veröffentlichungstag und Bekanntmachung des Hinweises auf die Patenterteilung: **04.08.2010**
(21) Anmeldenummer: 02730231.4
(22) Anmeldetag: 03.05.2002
(51) Int. Cl.: C07D 233/32, A01N 37/18, C07D 239/10, C07D 307/14, C07D 285/08, C07D 333/38, C07D 261/18, C07D 261/04, C07D 261/02, C07D 277/46, C07D 265/02, C07D 257/04, C07C 233/76, C07C 275/38, C07D 295/20, C07C 333/08

(54) **SUBSTITUIERTE BENZOYLZYKLOHEXENONE UND DEREN VERWENDUNG ALS HERBIZIDE MITTEL**
SUBSTITUTED BENZOYLCYCLOHEXENONES AND THEIR USE AS HERBICIDAL AGENTS
BENZOYLCYCLOHEXENONE SUBSTITUEES ET LEUR UTILISATION EN TANT QU'HERBICIDES

(30) Priorität: 16.05.2001 DE 10123887; 06.08.2001 DE 10138576
(43) Veröffentlichungstag der Anmeldung: 03.03.2004
(73) Patentinhaber: Bayer Aktiengesellschaft, 51368 Leverkusen (DE)
(72) Erfinder: SCHWARZ, Hans-Georg, 40764 Langenfeld (DE); MÜLLER, Klaus-Helmut, 40593 Düsseldorf (DE); HERRMANN, Stefan, 40764 Langenfeld (DE); HOISCHEN, Dorothee, 40474 Düsseldorf (DE); KATHER, Kristian, 40764 Langenfeld (DE); LEHR, Stefan, 65835 Liederbach (DE); SCHALLNER, Otto, 40789 Monheim (DE); DREWES, Mark, Wilhelm, 40764 Langenfeld (DE); DAHMEN, Peter, 41470 Neuss (DE); FEUCHT, Dieter, 40789 Monheim (DE); PONTZEN, Rolf, 42799 Leichlingen (DE); GOTO, Toshio, Shimotsuga-gun, Tochigi 329-0414 (JP); Shirakura, Shinichi, Tochigi 323-0022 (JP)
(74) Vertreter: Schwenk, Norbert
(86) Internationale Anmeldenummer: PCT/EP2002/004851
(87) Internationale Veröffentlichungsnummer: WO 2002/092574

(56) Entgegenhaltungen:
- WO-A-00/05221
- US-A- 4 780 127

## Beschreibung

Die Erfindung betrifft neue substituierte Benzoylcyclohexenone, Verfahren zu ihrer Herstellung und ihre Verwendung als Herbizide.

Es ist bereits bekannt, dass bestimmte substituierte Benzoylcyclohexenone oder Benzoylcyclohexandione, wie z.B. die Verbindungen N-[2,6-Dichlor-3-[(3,3-dimethyl-2,6-dioxo-cyclohexyl)-carbonyl]-phenyl]-acetamid, N-[2-Chlor-3-[(2,6-dioxo-cyclohexyl)-carbonyl]-4-nitro-phenyl]-acetamid, N-[2-Chlor-3-[(2,6-dioxo-cyclohexyl)-carbonyl]-phenyl]-acetamid, N-[2,6-Dichlor-3-[(2,6-dioxo-cyclohexyl)-carbonyl]-phenyl]-acetamid (vgl. US-A-4 780 127), herbizide Eigenschaften aufweisen (vgl. auch EP-A-090 262, EP-A-135 191, EP-A-137 963, EP-A-186-118, EP-A-186 119, EP-A-186 120, EP-A-319 075, WO-A-96/26200, WO-A-97/46530, WO-A-99/07688, WO-A-99/10327, WO-A-00/05221, WO-A-00/21924). Die Wirkung dieser Verbindungen ist jedoch nicht in allen Belangen zufriedenstellend.

Aufgabe der vorliegenden Erfindung war die Bereitstellung weiterer Herbizide.

Es wurden nun die neuen substituierten Benzoylcyclohexenone der allgemeinen Formel (I) 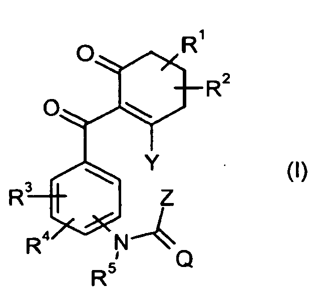 in welcher
- Q: für O (Sauerstoff) oder S (Schwefel) steht,
- R¹: steht für Wasserstoff, Fluor, Chlor oder Brom, für jeweils gegebenenfalls durch Fluor, Chlor, Methoxy, Ethoxy, n- oder i-Propoxy, Methylthio, Ethyl- thio, n- oder i-Propylthio, Methylsulfinyl, Ethylsulfinyl, Methylsulfonyl oder Ethylsulfonyl substituiertes Methyl, Ethyl, n- oder i-Propyl, n-, i-, s- oder t- Butyl, Methylthio, Ethylthio, n- oder i-Propylthio, n-, i-, s- oder t-Butylthio, oder für Phenyl.
- R²: steht für Wasserstoff, Fluor, Chlor oder Brom, für jeweils gegebenenfalls durch Fluor oder Chlor substituiertes Methyl, Ethyl, n- oder i-Propyl, n-, i-, s- oder t-Butyl, oder gegebenenfalls auch zusammen mit R¹ für O (Sauerstoff), Ethan-1,2-diyl, Propan-1,3-diyl oder Butan-1,4-diyl.
- R³, R⁴: stehen unabhängig voneinander für Wasserstoff, Nitro, Cyano, Carboxy, Carbamoyl, Thiocarbamoyl, Fluor, Chlor, Brom, Iod, für jeweils gegebenen- falls durch Fluor, Chlor, Methoxy, Ethoxy, Methylthio, Ethylthio, Methyl- sulfinyl, Ethylsulfinyl, Methylsulfonyl oder Ethylsulfonyl substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n- oder i- Propylthio, n-, i-, s- oder t-Butylthio, Methylsulfinyl, Ethylsulfinyl, n- oder i- Propylsulfinyl, Methylsulfonyl, Ethylsulfonyl, n- oder i-Propylsulfonyl, für Methylamino, Ethylamino, n- oder i-Propylamino, n-, i-, s- oder t-Butyl- amino, Dimethylamino, Diethylamino, Dimethylaminocarbonyl, Diethyl- aminocarbonyl, Dimethylaminosulfonyl oder Diethylaminosulfonyl.
- R⁵: steht für Wasserstoff, für jeweils gegebenenfalls durch Cyano, Fluor, Chlor, Brom, Methoxy, Ethoxy, n- oder i-Propoxy substituiertes Methyl, Ethyl, n- oder i-Propyl, n-, i-, s- oder t-Butyl, n-, i-, s- oder t-Pentyl, Methoxy, Ethoxy, n- oder i-Propoxy, n-, i-, s- oder t-Butoxy, Methylthio, Ethylthio, n- oder i- Propylthio, n-, i-, s- oder t-Butylthio, Methylsulfinyl, Ethylsulfinyl, n- oder i- Propylsulfinyl, Methylsulfonyl, Ethylsulfonyl, Methylamino, Ethylamino, n- oder i-Propylamino, n-, i-, s- oder t-Butylamino, für Dimethylamino oder Di- ethylamino, für jeweils gegebenenfalls durch Fluor, Chlor und/oder Brom substituiertes Ethenyl, Propenyl, Butenyl, Pentenyl, Ethinyl, Propinyl, Butinyl oder Pentinyl, für jeweils gegebenenfalls durch Cyano, Fluor, Chlor, Brom, Methyl oder Ethyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, oder für jeweils gegebenenfalls durch Nitro, Cyano, Fluor, Chlor, Brom, Methyl, Ethyl, n- oder i-Propyl, n-, i-, s- oder t-Butyl, Trifluor- methyl, Methoxy, Ethoxy, n- oder i-Propoxy, Difluormethoxy oder Trifluor- methoxy substituiertes Phenyl, Phenylthio, Phenylsulfinyl, Phenylsulfonyl, Naphthyl, Phenylmethyl, Phenylethyl, Naphthylmethyl oder Naphthylethyl oder für Gruppierung -C(Q)-Z.
- Y: steht für Hydroxy, Formyloxy, Fluor, Chlor oder Brom, für jeweils gege- benenfalls durch Cyano, Fluor, Chlor, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl substituiertes Methoxy, Ethoxy, n- oder i- Propoxy, Methylthio, Ethylthio, n- oder i-Propylthio, Methylsulfinyl, Ethyl- sulfinyl, Methylsulfonyl, Ethylsulfonyl, Acetyloxy, Propionyloxy, n- oder i- Butyroyloxy, Methoxycarbonyloxy, Ethoxycarbonyloxy, n- oder i-Propoxy- carbonyloxy, Methylaminocarbonyloxy, Ethylaminocarbonyloxy, n- oder i- Propylaminocarbonyloxy, Methylsulfonyloxy, Ethylsulfonyloxy, n- oder i- Propylsulfonyloxy, für jeweils gegebenenfalls durch Fluor, Chlor und/oder Brom substituiertes Propenyloxy, Butenyloxy, Propinyloxy oder Butinyloxy, für jeweils gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy, Trifluormethoxy, Methylthio, Ethylthio, n- oder i-Propylthio, Difluormethylthio, Trifluormethylthio, Methylsulfinyl, Ethylsulfinyl, Trifluormethylsulfinyl, Methylsulfonyl, Ethyl- sulfonyl oder Trifluormethylsulfonyl substituiertes Phenyloxy, Phenylthio, Phenylsulfinyl, Phenylsulfonyl, Phenylcarbonyloxy, Phenylcarbonylmethoxy, Phenylsulfonyloxy, Phenylmethoxy, Phenylmethylthio, Phenylmethylsulfinyl oder Phenylmethylsulfonyl.
- Z: steht für Wasserstoff, Amino, Cyanoamino, Nitroamino, Hydroxyamino, Hydrazino,
für durch Cyano, Fluor, Chlor, Methoxy, Ethoxy, n- oder i-Propoxy, Methyl- thio, Ethylthio, n- oder i-Propylthio, Methylsulfinyl oder Ethylsulfonyl substituiertes Methyl,
für jeweils gegebenenfalls durch Fluor, Chlor und/oder Brom substituiertes Ethenyl, Propenyl, Butenyl, Pentenyl, Ethinyl, Propinyl, Butinyl, Pentinyl, Propenyloxy, Butenyloxy, Pentenyloxy, Propenylthio, Butenylthio, Pentenyl- thio, Propenylamino, Butenylamino, Pentenylamino, Propenyloxyamino, Butenyloxyamino, Pentenyloxyamino, Ethinyl, Propinyl, Butinyl, Pentinyl, Propinyloxy, Butinyloxy, Pentinyloxy, Propinylamino, Butinylamino oder Pentinylamino,
für jeweils gegebenenfalls durch Cyano, Fluor, Chlor, Brom, Methyl oder Ethyl substituiertes Cyclopropyl, Cyclobutyl, Cyclopentyl, Cyclohexyl, Cyclopropyloxy, Cyclobutyloxy, Cyclopentyloxy, Cyclohexyloxy, Cyclo- propylamino, Cyclobutylamino, Cyclopentylamino, Cyclohexylamino, Cyclo- propylhydrazino, Cyclobutylhydrazino, Cyclopentylhydrazino, Cyclohexylhy- drazino, Cyclopropylmethyl, Cyclobutylmethyl, Cyclopentylmethyl, Cyclo- hexylmethyl, Cyclopropylmethoxy, Cyclobutylmethoxy, Cyclopentylmethoxy, Cyclohexylmethoxy, Cyclopropylmethylamino, Cyclobuylmethylamino, Cyclopentylmethylamino oder Cyclohexylmethylamino,
für jeweils gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy, Trifluormethoxy, Methoxy- carbonyl, Ethoxycarbonyl, n- oder i-Propoxycarbonyl substituiertes Phenyl, Phenylcarbonyl, Phenoxy, Phenoxycarbonyl, Phenylthio, Phenylamino, Phenylhydrazino, Naphthyl, Naphthyloxy, Naphtylthio, Naphthylamino, Phenylmethyl, Phenylethyl, Phenylmethoxy, Phenylethoxy, Phenylmethylthio, Phenylethylthio, Phenylmethylamino, Phenylethylamino, Naphthylmethyl, Naphthylethyl, Naphthylmethoxy, Naphthylethoxy, Naphthylmethylamino oder Naphthylethylamino, oder
für jeweils gegebenenfalls durch Nitro, Cyano, Fluor, Chlor, Brom, Methyl, Ethyl, n- oder i-Propyl, n-, i-, s- oder t-Butyl, Difluormethyl, Trifluormethyl, Dichlormethyl, Trichlormethyl, Chlordifluormethyl, Fluordichlormethyl, Methoxy, Ethoxy, n- oder i-Propoxy, n-, i-, s- oder t-Butoxy, Difluormethoxy, Trifluormethoxy, Methylthio, Ethylthio, n- oder i-Propylthio, n-, i-, s- oder t- Butylthio, Difluormethylthio, Trifluormethylthio, Methoxycarbonyl, Ethoxy- carbonyl, n- oder i-Propoxycarbonyl substituiertes monocyclisches oder bi- cyclisches Heterocyclyl, Heterocyclyloxy, Heterocyclylamino, die Gruppierung -N=(Heterocyclyl), Heterocyclylalkyl, Heterocyclylalkoxy oder Heterocyclylalkylamino aus der Reihe Furyl, Tetrahydrofuryl, Furyloxy, Tetrahydrofuryloxy, Furylamino, Tetrahydrofurylamino, Furylmethyl, Tetra- hydrofurylmethyl, Furylmethoxy, Tetrahydrofurylmethoxy, Furylmethyl- amino, Tetrahydrofurylmethylamino, Dioxolanyl, Dioxolanylmethyl, Di- oxolanylmethoxy, Dioxolanylmethylamino, Thienyl, Thienylamino, Thienyl- methyl, Thenylmethylamino, Dithiolanyl, Dithiolanylmethyl, Dithiolanyl- methoxy, Dithiolanylmethylamino, Pyrrolidinyl, Pyrrolidinylamino, Oxo- pyrrolidinyl, Pyrrolyl, Indolyl, Pyrrolylmethyl, Pyrazolyl, Pyrazolyloxy, Pyrazolylamino, Pyrazolylmethyl, Imidazolyl, Imidazolinyl, Imidazolyl- methyl, Imidazolinylmethyl, Oxoimidazolinyl, 2-Oxo-1,3-diaza-cyclopentyl, Oxazolyl, Oxazolylamino, Dihydrooxazolyl (Oxazolinyl), Tetrahydro- oxazolyl, (Oxazolidinyl), Isoxazolyl, Isoxazolylamino, Dihydroisoxazolyl (Isoxazolinyl), Tetrahydroisoxazolyl (Isoxazolidinyl), Tetrahydro-(2H)-1,2- oxazin-2-yl, Oxazolylmethyl, Thiazolyl, Thiazolylamino, Thiazolylmethyl, Dihydrothiazolyl (Thiazolinyl), Tetrahydrothiazolyl (Thiazolidinyl), Thiazolimino, Thiazolinylthio, Thiazolinylamino, Oxothiazolidinyl, Cyano- iminothiazolidinyl, Oxadiazolylamino, Thiadiazolylamino, Triazolylamino, Oxotriazolinyl, Thioxotriazolinyl, Oxotetrazolinyl, Oxotetrazolinylmethyl, Tetrazolylmethyl, Dioxanyl, Dioxanylmethyl, Dioxanylmethoxy, Dioxanyl- methylamino, Dithianyl, Dithianylmethyl, Dithianylmethoxy, Dithianyl- methylamino, Piperidinyl, Piperidinylamino, Oxopiperidinyl, 2-Oxo-1,3- diaza-cyclohexyl, 2-Oxo-1-aza-cycloheptyl, 2-Oxo-1,3-diaza-cycloheptyl, Morpholinyl, Morpholinylamino, Piperazinyl, Pyridinyl, Pyridinyloxy, Pyridinylthio, Pyridinylamino, 2-(1H)-Pyridinimino, Pyridinylmethyl, Pyridinylmethoxy, Pyrimidinyl, Pyrimidinyloxy, Pyrimidinylthio, Pyri- midinylamino, Pyrimidinylmethyl oder Pyrimidinylmethoxy, - einschließlich aller möglichen tautomeren Formen der Verbindungen der all- gemeinen Formel (I) und der möglichen Salze der Verbindungen der allge- meinen Formel (I) -
gefunden.
- R¹: steht ganz besonders bevorzugt für Wasserstoff, für jeweils gegebenenfalls durch Fluor oder Chlor substituiertes Methyl, Ethyl, n- oder i-Propyl, Methyl- thio, Ethylthio, n- oder i-Propylthio, oder für Phenyl.
- R²: steht ganz besonders bevorzugt für Wasserstoff, für jeweils gegebenenfalls durch Fluor oder Chlor, substituiertes Methyl, Ethyl, n- oder i-Propyl, oder ge- gebenenfalls auch zusammen mit R¹ für Ethan-1,2-diyl, Propan-1,3-diyl oder Butan-1,4-diyl.
- R³,R⁴: stehen ganz besonders bevorzugt unabhängig voneinander für Wasserstoff, Nitro, Cyano, Fluor, Chlor, Brom, Iod, Methyl, Ethyl, n- oder i-Propyl, Di- fluormethyl, Trifluormethyl, Dichlormethyl, Trichlormethyl, Methoxymethyl, Methylthiomethyl, Methylsulfinylmethyl, Methylsulfonylmethyl, Methoxy, Ethoxy, Difluormethoxy, Trifluormethoxy, Methylthio, Ethylthio, Methyl- sulfinyl, Ethylsulfinyl, Methylsulfonyl, Ethylsulfonyl oder Dimethylamino- sulfonyl.
- R⁵: steht ganz besonders bevorzugt für Wasserstoff, für jeweils gegebenenfalls durch Cyano, Fluor, Chlor, Methoxy oder Ethoxy substituiertes Methyl, Ethyl, n- oder i-Propyl, n- oder i-Butyl, für Methoxy, Ethoxy, n- oder i-Propoxy, Methylthio, Ethylthio, n- oder i-Propylthio, Methylsulfinyl, Ethylsulfinyl, n- oder i-Propylsulfinyl, Methylsulfonyl, Ethylsulfonyl, Methylamino, Ethyl- amino, n- oder i-Propylamino oder Dimethylamino, für jeweils gegebenen- falls durch Fluor und/oder Chlor substituiertes Propenyl, Butenyl, Propinyl oder Butinyl, für jeweils gegebenenfalls durch Cyano, Fluor, Chlor oder Methyl substituiertes Cyclopropyl, Cyclobutyl, Cyclopentyl, Cyclohexyl, Cyclopropylmethyl, Cyclobutylmethyl, Cyclopentylmethyl oder Cyclohexyl- methyl, oder für jeweils gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes Phenyl, Phenylthio, Phenylsulfinyl, Phenylsulfonyl, Phenyl- methyl oder Phenylethyl, oder für Gruppierung -C(Q)-Z.
- Y: steht ganz besonders bevorzugt für Hydroxy, für jeweils gegebenenfalls durch Fluor und/oder Chlor substituiertes Methoxy, Ethoxy, n- oder i-Propoxy, Methylthio, Ethylthio, n- oder i-Propylthio, Methylsulfinyl, Ethylsulfinyl, Methylsulfonyl, Ethylsulfonyl, Acetyloxy, Propionyloxy, n- oder i-Butyroyl- oxy, Methoxycarbonyloxy, Ethoxycarbonyloxy, n- oder i-Propoxycarbonyl- oxy, Methylaminocarbonyloxy, Ethylaminocarbonyloxy, n- oder i-Propyl- aminocarbonyloxy, Methylsulfonyloxy, Ethylsulfonyloxy, n- oder i-Propyl- sulfonyloxy,
für jeweils gegebenenfalls durch Fluor, Chlor und/oder Brom substituiertes Propenyloxy, Butenyloxy, Propinyloxy oder Butinyloxy,
für jeweils gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy, Trifluormethoxy, Methylthio, Ethylthio, n- oder i-Propylthio, Difluormethylthio, Trifluormethylthio, Methylsulfinyl, Ethylsulfinyl, Trifluormethylsulfinyl, Methylsulfonyl, Ethyl- sulfonyl oder Trifluormethylsulfonyl substituiertes Phenyloxy, Phenylthio, Phenylsulfinyl, Phenylsulfonyl, Phenylcarbonyloxy, Phenylcarbonylmethoxy, Phenylsulfonyloxy, Phenylmethoxy, Phenylmethylthio, Phenylmethylsulfinyl oder Phenylmethylsulfonyl.
- Z: steht ganz besonders bevorzugt für Amino, Cyanoamino, Hydrazino,
für durch Cyano, Fluor, Chlor, Methoxy, Ethoxy, n- oder i-Propoxy, Methyl- thio, Ethylthio, n- oder i-Propylthio, Methylsulfinyl, Ethylsulfinyl, n- oder i- Propylsulfinyl, Methylsulfonyl oder Ethylsulfonyl substituiertes Methyl,
für jeweils gegebenenfalls durch Cyano, Fluor, Chlor, Methoxy, Ethoxy, n- oder i-Propoxy, Methylthio, Ethylthio, n- oder i-Propylthio, Methylsulfinyl, Ethylsulfinyl, n- oder i-Propylsulfinyl, Methylsulfonyl oder Ethylsulfonyl sub- stituiertes Ethyl, n- oder i-Propyl, n-, i-, s- oder t-Butyl, Methoxy, Ethoxy, n- oder i-Propoxy, Methylthio, Ethylthio, n- oder i-Propylthio, Methylamino, Ethylamino, n- oder i-Propylamino, Methoxyamino, Ethoxyamino, n- oder i- Propoxyamino, Methylhydrazino, Ethylhydrazino, n- oder i-Propylhydrazino, n-, i-, s- oder t-Butylhydrazino, Dimethylamino, N-Methyl-methoxyamino oder Dimethylhydrazino,
für jeweils gegebenenfalls durch Fluor und/oder Chlor substituiertes Ethenyl, Propenyl, Butenyl, Ethinyl, Propinyl, Butinyl, Propenyloxy, Butenyloxy, Propenylthio, Butenylthio, Propenylamino, Butenylamino, Propenyloxy- amino, Butenyloxyamino, Ethinyl, Propinyl, Butinyl, Propinyloxy, Butinyl- oxy, Propinylamino oder Butinylamino,
für jeweils gegebenenfalls durch Cyano, Fluor, Chlor oder Methyl substitu- iertes Cyclopropyl, Cyclobutyl, Cyclopentyl, Cyclohexyl, Cyclopropyloxy, Cyclobutyloxy, Cyclopentyloxy, Cyclohexyloxy, Cyclopropylamino, Cyclo- butylamino, Cyclopentylamino, Cyclohexylamino, Cyclopentylhydrazino, Cyclohexylhydrazino, Cyclopropylmethyl, Cyclobutylmethyl, Cyclopentyl- methyl, Cyclohexylmethyl, Cyclopropylmethoxy, Cyclobutylmethoxy, Cyclo- pentylmethoxy, Cyclohexylmethoxy, Cyclopropylmethylamino, Cyclobuyl- methylamino, Cyclopentylmethylamino oder Cyclohexylmethylamino,
für jeweils gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy, Trifluormethoxy, Methoxy- carbonyl, Ethoxycarbonyl, n- oder i-Propoxycarbonyl substituiertes Phenyl, Phenylcarbonyl, Phenoxy, Phenoxycarbonyl, Phenylthio, Phenylamino, Phenylhydrazino, Phenylmethyl, Phenylethyl, Phenylmethoxy, Phenylethoxy, Phenylmethylthio, Phenylethylthio, Phenylmethylamino oder Phenylethyl- amino, oder für jeweils gegebenenfalls durch Nitro, Cyano, Fluor, Chlor, Brom, Methyl, Ethyl, n- oder i-Propyl, n-, i-, s- oder t-Butyl, Difluormethyl, Trifluormethyl, Dichlormethyl, Trichlormethyl, Chlordifluormethyl, Fluor- dichlormethyl, Methoxy, Ethoxy, n- oder i-Propoxy, Difluormethoxy, Tri- fluormethoxy, Methylthio, Ethylthio, n- oder i-Propylthio, Difluormethylthio, Trifluormethylthio, Methoxycarbonyl, Ethoxycarbonyl, n- oder i-Propoxy- carbonyl substituiertes monocyclisches oder bicyclisches Heterocyclyl, Heterocyclyloxy, Heterocyclylamino, die Gruppierung -N=(Heterocyclyl), Heterocyclylalkyl, Heterocyclylalkoxy oder Heterocyclylalkylamino aus der Reihe Furyl, Tetrahydrofuryl, Furyloxy, Tetrahydrofuryloxy, Furylamino, Tetrahydrofurylamino, Furylmethyl, Tetrahydrofurylmethyl, Furylmethoxy, Tetrahydrofurylmethoxy, Furylmethylamino, Tetrahydrofurylmethylamino, Dioxolanyl, Dioxolanylmethyl, Dioxolanylmethoxy, Dioxolanylmethylamino, Thienyl, Thienylmethyl, Dithiolanyl, Dithiolanylmethyl, Dithiolanylmethoxy, Dithiolanylmethylamino, Pyrrolidinyl, Pyrrolidinylamino, Oxopyrrolidinyl, Pyrrolyl, Indolyl, Pyrrolylmethyl, Pyrazolyl, Pyrazolyloxy, Pyrazolylamino, Pyrazolylmethyl, Imidazolyl, Imidazolinyl, Imidazolylmethyl, Imidazolinyl- methyl, Oxoimidazolinyl, 2-Oxo-1,3-diaza-cyclopentyl, Oxazolyl, Oxazolyl- methyl, Dihydrooxazolyl (Oxazolinyl), Tetrahydrooxazolyl (Oxazolidinyl), Is- oxazolyl, Dihydroisoxazolyl (Isoxazolinyl), Tetrahydroi-(2H)-1,2-oxazin-z-yl, Tetrahydroisoxazolyl (Isoxazolidinyl), Thiazolyl, Thiazolylmethyl, Dihydro- thiazolyl (Thiazolinyl), Tetrahydrothiazolyl (Thiazolidinyl), Thiazolimino, Oxothiazolidinyl, Cyanoiminothiazolidinyl, Oxadiazolylamino, Thiadiazolylamino, Oxotriazolinyl, Oxatetrazolinylmethyl, Tetrazolylmethyl, Oxotetrazolinyl, Oxotetrazolinylmethyl, Tetrazolylmethyl, Dioxanyl, Di- oxanylmethyl, Dioxanylmethoxy, Dioxanylmethylamino, Dithianyl, Di- thianylmethyl, Dithianylmethoxy, Dithianylmethylamino, Triazolylamino, Piperidinyl, Piperidinylamino, 2-(1H)-Pyridinimino, Oxopiperidinyl, 2-Oxo- 1,3-diaza-cyclohexyl, 2-Oxo-1-aza-cycloheptyl, 2-Oxo-1,3-diaza-cycloheptyl, Morpholinyl, Morpholinylamino, Piperazinyl, Pyridinyl, Pyridinyloxy, Pyri- dinylamino, Pyridinylmethyl, Pyridinylmethoxy, Pyrimidinyl, Pyrimidinyloxy, Pyrimidinylmethyl oder Pyrimidinylmethoxy.
- R¹ und R²: stehen am meisten bevorzugt für Wasserstoff.
- R³: steht am meisten bevorzugt für Wasserstoff, Fluor, Chor, Methyl, Ethyl, Trifluormethyl, Trichlormethyl, Methoxy, Ethoxy, Methylsulfonyl oder Ethylsulfonyl.
- R⁴: steht am meisten bevorzugt für Wassestoff, Fluor, Chlor, Methyl, Ethyl, Trifluormethyl, Trichlormethyl, Methoxy, Ethoxy, Methylsulfonyl oder Ethylsulfonyl.
- R⁵: steht am meisten bevorzugt für Wasserstoff.
- Y: steht am meisten bevorzugt für Hydroxy.

Erfindungsgemäß bevorzugt sind die Verbindungen der Formel (I), in welchen eine Kombination der vorstehend als bevorzugt aufgeführten Bedeutungen vorliegt.

Erfindungsgemäß besonders bevorzugt sind die Verbindungen der Formel (I), in welchen eine Kombination der vorstehend als besonders bevorzugt aufgeführten Bedeutungen vorliegt.

Erfindungsgemäß ganz besonders bevorzugt sind die Verbindungen der Formel (I), in welchen eine Kombination der vorstehend als ganz besonders bevorzugt aufgeführten Bedeutungen vorliegt.

Erfindungsgemäß am meisten bevorzugt sind die Verbindungen der Formel (I) in welchen eine Kombination der vorstehend als am meisten bevorzugt aufgeführten Bedeutungen vorliegt.

Eine ganz besonders bevorzugte Gruppe sind diejenigen Verbindungen der allgemeinen Formel (I), in welcher
- Q: für O oder S steht,
- R¹: für Wasserstoff, für jeweils gegebenenfalls durch Fluor oder Chlor substitu- iertes Methyl, Ethyl, n- oder i-Propyl, Methylthio, Ethylthio, n- oder i-Propyl- thio, oder für Phenyl steht,
- R²: für Wasserstoff, für jeweils gegebenenfalls durch Fluor oder Chlor substitu- iertes Methyl, Ethyl, n- oder i-Propyl, oder gegebenenfalls auch zusammen mit R¹ für Ethan-1,2-diyl, Propan-1,3-diyl oder Butan-1,4-diyl steht,
- R³ R⁴: unabhängig voneinadner für Wasserstoff, Nitro, Cyano, Fluor, Chlor, Brom, Iod, Methyl, Ethyl, n- oder i-Propyl, Difluormethyl, Trifluormethyl, Dichlor- methyl, Trichlormethyl, Methoxymethyl, Methylthiomethyl, Methylsulfinyl- methyl, Methylsulfonylmethyl, Methoxy, Ethoxy, Difluormethoxy, Trifluor- methoxy, Methylthio, Ethylthio, Methylsulfinyl, Ethylsulfinyl, Methyl- sulfonyl, Ethylsulfonyl oder Dimethylaminosulfonyl steht,
- R⁵: für Wasserstoff, für jeweils gegebenenfalls durch Cyano, Fluor, Chlor, Methoxy oder Ethoxy substituiertes Methyl, Ethyl, n- oder i-Propyl, n- oder i- Butyl, für Methoxy, Ethoxy, n- oder i-Propoxy, Methylthio, Ethylthio, n- oder i-Propylthio, Methylsulfinyl, Ethylsulfinyl, n- oder i-Propylsulfinyl, Methyl- sulfonyl, Ethylsulfonyl, Methylamino, Ethylamino, n- oder i-Propylamino oder Dimethylamino, für jeweils gegebenenfalls durch Fluor und/oder Chlor substituiertes Propenyl, Butenyl, Propinyl oder Butinyl, für jeweils gege- benenfalls durch Cyano, Fluor, Chlor oder Methyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, oder für jeweils gegebenenfalls durch Nitro, Cyano, Fluor, Chlor, Brom, Methyl, Ethyl, n- oder i-Propyl, n-, i- , s- oder t-Butyl, Trifluormethyl, Methoxy, Ethoxy, n- oder i-Propoxy, Di- fluormethoxy oder Trifluormethoxy substituiertes Phenyl, Phenylthio, Phenyl- sulfinyl, Phenylsulfonyl, Phenylmethyl oder Phenylethyl, oder für Gruppierung -C(Q)-Z steht,
- Y: für Hydroxy, für jeweils gegebenenfalls durch Fluor und/oder Chlor substitu- iertes Methoxy, Ethoxy, n- oder i-Propoxy, Methylthio, Ethylthio, n- oder i- Propylthio, Methylsulfinyl, Ethylsulfinyl, Methylsulfonyl, Ethylsulfonyl, Acetyloxy, Propionyloxy, n- oder i-Butyroyloxy, Methoxycarbonyloxy, Ethoxycarbonyloxy, n- oder i-Propoxycarbonyloxy, Methylaminocarbonyl- oxy, Ethylaminocarbonyloxy, n- oder i-Propylaminocarbonyloxy, Methyl- sulfonyloxy, Ethylsulfonyloxy, n- oder i-Propylsulfonyloxy,
für jeweils gegebenenfalls durch Fluor, Chlor und/oder Brom substituiertes Propenyloxy, Butenyloxy, Propinyloxy oder Butinyloxy,
für jeweils gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy, Trifluormethoxy, Methylthio, Ethylthio, n- oder i-Propylthio, Difluormethylthio, Trifluormethylthio, Methylsulfinyl, Ethylsulfinyl, Trifluormethylsulfinyl, Methylsulfonyl, Ethyl- sulfonyl oder Trifluormethylsulfonyl substituiertes Phenyloxy, Phenylthio, Phenylsulfinyl, Phenylsulfonyl, Phenylcarbonyloxy, Phenylcarbonylmethoxy, Phenylsulfonyloxy, Phenylmethoxy, Phenylmethylthio, Phenylmethylsulfinyl oder Phenylmethylsulfonyl steht, und
- Z: für Amino, Cyanoamino, Hydrazino,
für durch Cyano, Fluor, Chlor, Methoxy, Ethoxy, n- oder i-Propoxy, Methyl- thio, Ethylthio, n- oder i-Propylthio, Methylsulfinyl, Ethylsulfinyl, n- oder i- Propylsulfinyl, Methylsulfonyl oder Ethylsulfonyl substituiertes Methyl, für jeweils gegebenenfalls durch Fluor und/oder Chlor substituiertes Ethenyl, Propenyl, Butenyl, Ethinyl, Propinyl, Butinyl, Propenyloxy, Butenyloxy, Propenylthio, Butenylthio, Propenylamino, Butenylamino, Propenyloxy- amino, Butenyloxyamino, Ethinyl, Propinyl, Butinyl, Propinyloxy, Butinyl- oxy, Propinylamino oder Butinylamino,
für jeweils gegebenenfalls durch Cyano, Fluor, Chlor oder Methyl substitu- iertes Cyclopropyl, Cyclobutyl, Cyclopentyl, Cyclohexyl, Cyclopropyloxy, Cyclobutyloxy, Cyclopentyloxy, Cyclohexyloxy, Cyclopropylamino, Cyclo- butylamino, Cyclopentylamino, Cyclohexylamino, Cyclopentylhydrazino, Cyclohexylhydrazino, Cyclopropylmethyl, Cyclobutylmethyl, Cyclopentyl- methyl, Cyclohexylmethyl, Cyclopropylmethoxy, Cyclobutylmethoxy, Cyclo- pentylmethoxy, Cyclohexylmethoxy, Cyclopropylmethylamino, Cyclobuyl- methylamino, Cyclopentylmethylamino oder Cyclohexylmethylamino,
für jeweils gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy, Trifluormethoxy, Methoxy- carbonyl, Ethoxycarbonyl, n- oder i-Propoxycarbonyl substituiertes Phenyl, Phenylcarbonyl, Phenoxy, Phenoxycarbonyl, Phenylthio, Phenylamino, Phenylhydrazino, Phenylmethyl, Phenylethyl, Phenylmethoxy, Phenylethoxy, Phenylmethylthio, Phenylethylthio, Phenylmethylamino oder Phenylethyl- amino, oder
für jeweils gegebenenfalls durch Nitro, Cyano, Fluor, Chlor, Brom, Methyl, Ethyl, n- oder i-Propyl, n-, i-, s- oder t-Butyl, Difluormethyl, Trifluormethyl, Dichlormethyl, Trichlormethyl, Chlordifluormethyl, Fluordichlormethyl, Methoxy, Ethoxy, n- oder i-Propoxy, Difluormethoxy, Trifluormethoxy, Methylthio, Ethylthio, n- oder i-Propylthio, Difluormethylthio, Trifluor- methylthio, Methoxycarbonyl, Ethoxycarbonyl, n- oder i-Propoxycarbonyl substituiertes monocyclisches oder bicyclisches Heterocyclyl, Heterocyclyl- oxy, Heterocyclylamino, Heterocyclylalkyl, Heterocyclylalkoxy oder Hetero- cyclylalkylamino aus der Reihe Furyl, Tetrahydrofuryl, Furyloxy, Tetrahydro- furyloxy, Furylamino, Tetrahydrofurylamino, Furylmethyl, Tetrahydrofuryl- methyl, Furylmethoxy, Tetrahydrofurylmethoxy, Furylmethylamino, Tetra- hydrofurylmethylamino, Dioxolanyl, Dioxolanylmethyl, Dioxolanylmethoxy, Dioxolanylmethylamino, Thienyl, Thienylmethyl, Dithiolanyl, Dithiolanyl- methyl, Dithiolanylmethoxy, Dithiolanylmethylamino, Pyrrolidinyl, Pyrrolidinylamino, Oxopyrrolidinyl, Pyrrolyl, Indolyl, Pyrrolylmethyl, Pyrazolyl, Pyrazolyloxy, Pyrazolylamino, Pyrazolylmethyl, Imidazolyl, Imid- azolinyl, Imidazolylmethyl, Imidazolinylmethyl, Oxoimidazolinyl, 2-Oxo-1,3- diaza-cyclopentyl, Oxazolyl, Oxazolylmethyl, Oxotetrazolinylmethyl, Tetra- zolylmethyl, Dihydrooxazolyl (Oxazolinyl), Tetrahydrooxazolyl (Oxazolidinyl), Isoxazolyl, Dihydroisoxazolyl (Isoxazolinyl), Tetrahydro- isoxazolyl (Isoxazolidinyl), Thiazolyl, Thiazolylmethyl, Dihydrothiazolyl (Thiazolinyl), Tetrahydrothiazolyl (Thiazolidinyl), Oxothiazolidinyl, Cyano- iminothiazolidinyl, Oxotriazolinyl, Oxotetrazolinyl, Dioxanyl, Dioxanyl- methyl, Dioxanylmethoxy, Dioxanylmethylamino, Dithianyl, Dithianyl- methyl, Dithianylmethoxy, Dithianylmethylamino, Piperidinyl, Piperidinyl- amino, Oxopiperidinyl, 2-Oxo-1,3-diaza-cyclohexyl, 2-Oxo-1-aza-cyclo- heptyl, 2-Oxo-1,3-diaza-cycloheptyl, Morpholinyl, Morpholinylamino, Piper- azinyl, Pyridinyl, Pyridinyloxy, Pyridinylamino, Pyridinylmethyl, Pyridinyl- methoxy, Pyrimidinyl, Pyrimidinyloxy, Pyrimidinylmethyl oder Pyrimidinyl- methoxy steht.

Die Verbindungen der Formeln (I-1) bis (I-3) werden besonders hervorgehoben:

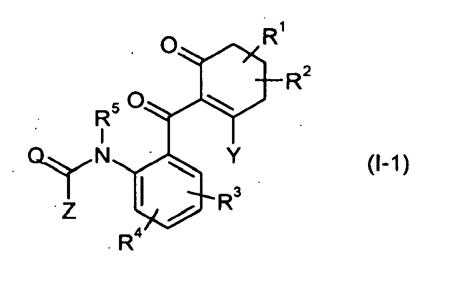

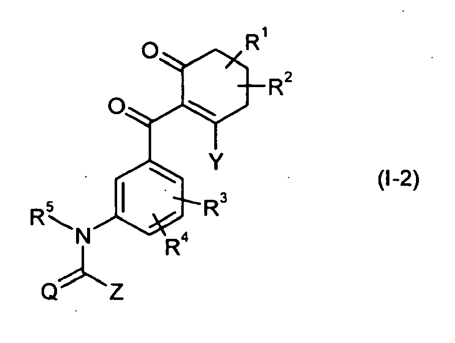

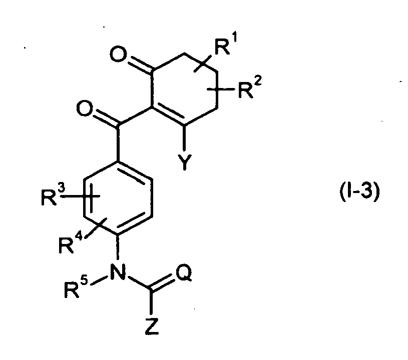

Q, R¹, R², R³, R⁴, R⁵, Y und Z haben hierbei jeweils die oben als ganz besonders bevorzugt angegebenen Bedeutungen.

Die Reste R³ bzw. R⁴ stehen wiederum bevorzugt an Position (2) bzw. (4) des Phenylringes.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangs- oder Zwischenprodukte. Diese Restedefinitionen können untereinander, also auch zwischen den angegebenen bevorzugten Bereichen beliebig kombiniert werden.

In den vorstehenden und nachfolgenden Definitionen gelten sofern nicht anders ausgeführt, die nachfolgenden Definitionen:
Gesättigte oder ungesättigte Kohlenwasserstoffketten, wie Alkyl, Alkandiyl, Alkenyl oder Alkinyl, auch in Verknüpfung mit Hetroatomen, wie beispielsweise Alkoxy, Alkylthio oder Alkylamino sind jeweils geradkettig oder verzweigt. Bevorzugt sind, falls nicht anders angegeben, Kohlenwasserstoffketten mit 1 bis 6 Kohlenstoffatomen.

Aryl steht für aromatische, mono- oder polycyclische Kohlenwasserstoffringe, wie z.B. Phenyl, Naphthyl, Anthranyl, Phenanthryl, vorzugsweise Phenyl oder Naphthyl, insbesondere Phenyl.

Heterocyclyl steht für gesättigte, ungesättigte oder aromatische, ringförmige Verbindungen, in denen mindestens ein Ringglied ein Heteroatom, d.h. ein von Kohlenstoff verschiedenes Atom, ist. Enthält der Ring mehrere Heteroatome, können diese gleich oder verschieden sein. Heteroatome sind bevorzugt Sauerstoff, Stickstoff oder Schwefel. Enthält diese Ring mehrere Sauerstoffatome, stehen diese nicht benachbart. Gegebenenfalls bilden die ringförmigen Verbindungen mit weiteren carbocyclischen oder heterocyclischen, ankondensierten oder überbrückten Ringen gemeinsam ein polycyclisches Ringsystem, ankondensierten oder überbrückten Ringen gemeinsam ein polycyclisches Ringsystem. Ein polycyclisches Ringsystem kann über den heterocyclischen Ring oder einen ankondensierten carbocyclischen Ring verknüpft sein. Bevorzugt sind mono- oder bicyclische Ringsysteme, insbesondere monocyclische Ringsysteme mit 5 bis 6 Ringgliedern und bicyclischen Ringsysteme mit 7 bis 9 Ringgliedern.

Cycloalkyl steht für gesättigte, carbocyclische, ringförmige Verbindungen, die gegebenenfalls mit weiteren carbocyclischen, ankondensierten oder überbrückten Ringen ein polycyclisches Ringsystem bilden. Bevorzugt sind, falls nicht anders angegeben, Cyclopropyl, Cyclopentyl und Cyclohexyl.

Die neuen substituierten Benzoylcyclohexenone der allgemeinen Formel (I) zeichnen sich durch starke und selektive herbizide Wirksamkeit aus.

Man erhält die neuen substituierten Benzoylcyclohexenone der allgemeinen Formel (I), wenn man

Cyclohexandione ("Hydroxycyclohexenone") der allgemeinen Formel (II) 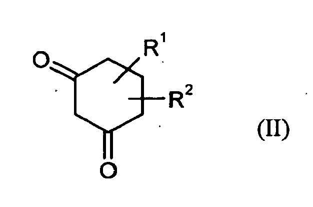 in welcher
- R¹ und R²: die oben angegebene Bedeutung haben,
mit substituierten Benzoesäuren der allgemeinen Formel (III), 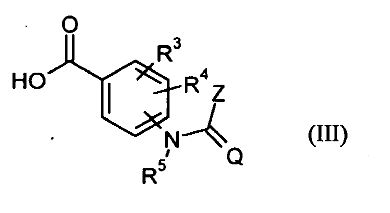 in welcher
- Q, R³, R⁴, R⁵ und Z: die oben angegebene Bedeutung haben,

- oder mit reaktionsfähigen Derivaten hiervon, wie z.B. mit entsprechenden Säurehalogeniden, Säureanhydriden, Säurecyaniden oder Estern -
gegebenenfalls in Gegenwart eines Dehydratisierungsmittels, gegebenenfalls in Gegenwart eines oder mehrerer Reaktionshilfsmittel und gegebenenfalls in Gegenwart eines oder mehrerer Verdünnungsmittel umsetzt,
und gegebenenfalls im Anschluß daran an den so erhaltenen Verbindungen der Formel (I) im Rahmen der Substituentendefinition auf übliche Weise elektrophile oder nucleophile Substitutionsreaktionen bzw. Oxidations- oder Reduktionsreaktionen durchführt oder die Verbindungen der Formel (I) auf übliche Weise in Salze überführt.

Die neuen substituierten Benzoylcyclohexenone der allgemeinen Formel (I) können prinzipiell auch wie im Folgenden schematisch dargestellt erhalten werden:

Umsetzung von Aminobenzoylcyclohexenonen der allgemeinen Formel (IV) mit Halogen(thio)carbonylverbindungen der allgemeinen Formel (V) oder gegebenenfalls mit entsprechenden Iso(thio)cyanaten (Q, R¹, R², R³, R⁴, R⁵, Y und Z haben hierbei die oben angegebenen Bedeutungen, X steht für Halogen):

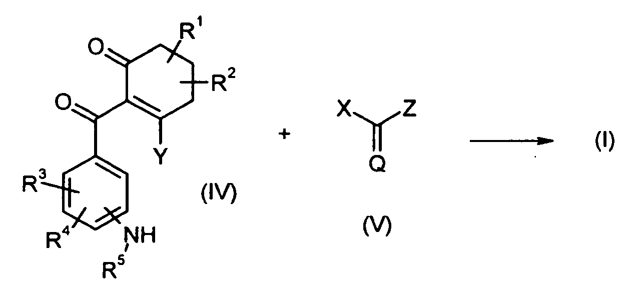

Die bei den erfindungsgemäßen Verfahren (α) und (β) als Ausgangsstoffe zu verwendenden Verbindungen sind durch die Formeln (IV) und (V) allgemein definiert. In den Formeln (IV) und (V) haben Q, R¹, R², R³, R⁴, R⁵, Y und Z vorzugsweise diejenige Bedeutungen, die bereits oben im Zusammenhang mit der Beschreibung der erfindungsgemäßen Verbindungen der Formel (I) als bevorzugt, besonders bevorzugt, ganz besonders bevorzugt oder als die meisten bevorzugt für Q, R¹, R², R³, R⁴, R⁵, Y und Z angegeben wurden.

Die Ausgangsstoffe der Formel (V) sind dem Fachmann bekannte Verbindungen.

Die Ausgangsstoffe der Formel (IV) können in dem Fachmann bekannter Weise erhalten werden.

Umsetzung von Iso(thio)cyanatobenzoylcyclohexenonen der allgemeinen Formel (VI) mit nucleophilen Verbindungen der allgemeinen Formel (VII); (Q, R¹, R², R³, R⁴, Y und Z haben hierbei die oben angegebenen Bedeutungen):

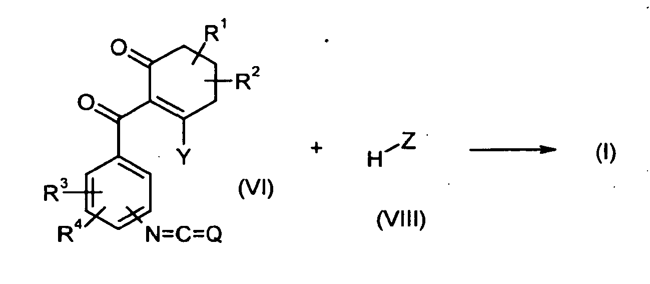

Verwendet man beispielsweise Cyclohexan-1,3-dion und 2-Chlor-4-[(Dimethylaminocarbonyl)-(methylamino)]-benzoesäure als Ausgangsstoffe, so kann der Reaktionsablauf beim erfindungsgemäßen Verfahren durch das folgende Formelschema skizziert werden:

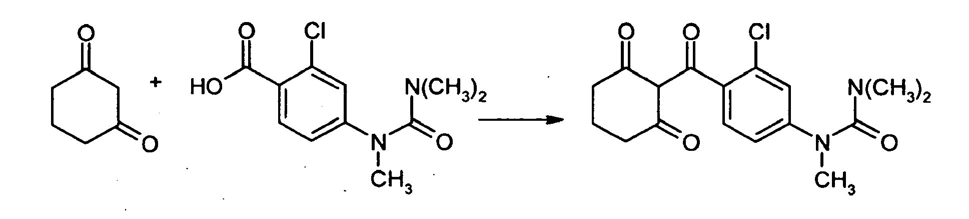

Die beim erfindungsgemäßen Verfahren zur Herstellung von Verbindungen der allgemeinen Formel (I) als Ausgangsstoffe zu verwendenden Cyclohexandione sind durch die Formel (II) allgemein definiert. In der allgemeinen Formel (II) haben R¹ und R² vorzugsweise diejenigen Bedeutungen, die bereits oben im Zusammenhang mit der Beschreibung der erfindungsgemäßen Verbindungen der allgemeinen Formel (I) als bevorzugt, besonders bevorzugt, ganz besonders bevorzugt oder am meisten bevorzugt für R¹ und R² angegeben worden sind.

Die Ausgangsstoffe der allgemeinen Formel (II) sind bekannt und/oder können nach an sich bekannten Verfahren hergestellt werden.

Die beim erfindungsgemäßen Verfahren weiter als Ausgangsstoffe zu verwendenden substituierten Benzoesäuren sind durch die Formel (III) allgemein definiert. In der Formel (III) haben Q, R³, R⁴, R⁵ und Z vorzugsweise diejenigen Bedeutungen, die bereits oben im Zusammenhang mit der Beschreibung der erfindungsgemäßen Verbindungen der Formel (I) als bevorzugt, besonders bevorzugt, ganz besonders bevorzugt oder am meisten bevorzugt für Q, R³, R⁴, R⁵ und Z angegeben wurden.

Die Ausgangsstoffe der allgemeinen Formel (III) sind bekannt und/oder können nach an sich bekannten Verfahren hergestellt werden (vgl. JP-A-11292849, Herstellungbeispiele).

Man erhält die substituierten Benzoesäuren der allgemeinen Formel (III), wenn man Benzoesäureester der allgemeinen Formel (IIIa), 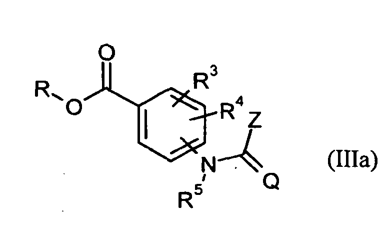 in welcher
- Q, R³, R⁴, R⁵ und Z: die oben angegebene Bedeutung haben und
- R: für Alkyl, insbesondere für Methyl oder Ethyl steht,
mit Wasser, gegebenenfalls in Gegenwart eines Hydrolysehilfsmittels, wie z.B. Natronlauge, und gegebenenfalls in Gegenwart eines Verdünnungsmittels, wie z.B. Tetrahydrofuran, bei Temperaturen zwischen 0°C und 100°C umsetzt (vgl. die Herstellungsbeispiele).

Die als Vorprodukte benötigten Benzoesäureester der allgemeinen Formel (IIIa) sind bekannt und/oder können nach an sich bekannten Verfahren hergestellt werden (vgl. JP-A-11292849, Herstellungsbeispiele).

Man erhält die Benzoesäureester der allgemeinen Formel (IIIa), wenn man
(α) Aminobenzoesäureester der allgemeinen Formel (IX) 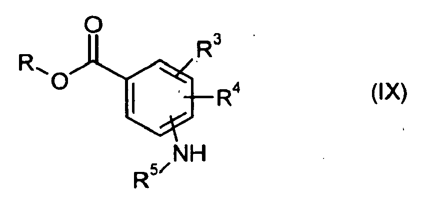 in welcher
- Q, R³, R⁴, R⁵ und Z: die oben angegebene Bedeutung haben und
- R: für Alkyl, insbesondere für Methyl oder Ethyl steht,
mit Halogen(thio)carbonylverbindungen der allgemeinen Formel (VI) 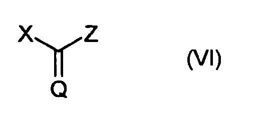 in welcher
- Q und Z: die oben angegebene Bedeutung haben und

- X: für Halogen, insbesondere Fluor, Chlor oder Brom steht,

- oder gegebenenfalls mit entsprechenden Iso(thio)cyanaten -
gegebenenfalls in Gegenwart eines Säureakzeptors, wie z.B. Kaliumcarbonat oder Triethylamin, und gegebenenfalls in Gegenwart eines Verdünnungsmittels, wie z.B. Methylisobutylketon oder Acetonitril, bei Temperaturen zwischen 0°C und 100°C umsetzt (vgl. die Herstellungsbeispiele),
oder wenn man
(β) Iso(thio)cyanatobenzoesäureester der allgemeinen Formel (X) 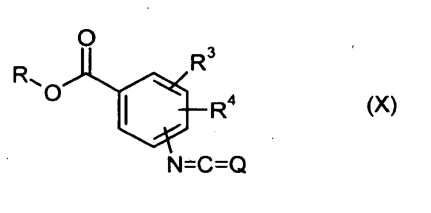 in welcher
- Q, R³ und R⁴: die oben angegebene Bedeutung haben und
- R: für Alkyl, insbesondere für Methyl oder Ethyl steht,
mit nucleophilen Verbindungen der allgemeinen Formel (VIII) 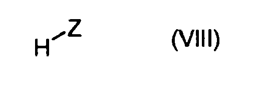 in welcher
- Z: die oben angegebene Bedeutung hat,
gegebenenfalls in Gegenwart eines Reaktionshilfsmittels, wie z.B. Triethylamin, und gegebenenfalls in Gegenwart eines Verdünnungsmittels, wie z.B. Acetonitril oder Toluol, bei Temperaturen zwischen 10°C und 120°C umsetzt (vgl. die Herstellungsbeispiele).

Die bei den erfindungsgemäßen Verfahren (α) und (β) als Ausgangsstoffe zu verwendenden Verbindungen sind durch die Formeln (IX), (VI), (X) und (VIII) allgemein definiert. In den Formeln (IX), (VI), (X) und (VIII) haben Q, R³, R⁴, R⁵ und Z vorzugsweise diejenige Bedeutungen, die bereits oben im Zusammenhang mit der Beschreibung der erfindungsgemäßen Verbindungen der Formel (I) als bevorzugt, besonders bevorzugt, ganz besonders bevorzugt oder als am meisten bevorzugt für Q, R³, R⁴, R⁵ und Z angegeben wurden.

Die Ausgangsstoffe der Formeln (VI) und (VIII) sind dem Fachmann bekannte Verbindungen.

Die Ausgangsstoffe der Formeln (IX) und (X) können in dem Fachmann bekannter Weise erhalten werden.

Das erfindungsgemäße Verfahren zur Herstellung der neuen substituierten Benzoylcyclohexenone der allgemeinen Formel (I) wird vorzugsweise unter Verwendung eines Dehydratisierungsmittels durchgeführt. Es kommen hierbei die üblichen zur Bindung von Wasser geeigneten Chemikalien in Betracht.

Als Beispiele hierfür seien Dicyclohexylcarbodiimid, Propanphosphonsäureanhydrid und Carbonyl-bis-imidazol genannt.

Als besonders gut geeignete Dehydratisierungsmittel seien Dicyclohexylcarbodiimid und Propanphosphonsäureanhydrid genannt.

Das erfindungsgemäße Verfahren zur Herstellung der neuen substituierten Benzoylcyclohexenone der allgemeinen Formel (I) wird gegebenenfalls unter Verwendung eines Reaktionshilfsmittels durchgeführt.

Als Beispiele hierfür seien Natriumcyanid, Kaliumcyanid, Acetoncyanhydrin, 2-Cyano-2-(trimethylsilyloxy)-propan und Trimethylsilylcyanid genannt.

Als besonders gut geeignetes Reaktionshilfsmittel sei Trimethylsilylcyanid genannt.

Das erfindungsgemäße Verfahren wird vorzugsweise unter Verwendung eines oder mehrerer Reaktionshilfsmittel durchgeführt. Als Reaktionshilfsmittel für das erfindungsgemäße Verfahren kommen im allgemeinen die üblichen anorganischen oder organischen Basen oder Säureakzeptoren in Betracht. Hierzu gehören vorzugsweise Alkalimetall- oder Erdalkalimetall- -acetate, -amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calcium-amid, Natrium-, Kalium- oder Calcium-carbonat, Natrium-, Kalium- oder Calcium-hydrogencarbonat, Lithium-, Natrium-, Kalium- oder Calcium-hydrid, Lithium-, Natrium-, Kalium- oder Calcium-hydroxid, Natrium- oder Kalium- -methanolat, -ethanolat, -n- oder -i-propanolat, -n-, -i-, -s- oder -t-butanolat; weiterhin auch basische organische Stickstoffverbindungen, wie beispielsweise Trimethylamin, Triethylamin, Tripropylamin, Tributylamin, Ethyl-diisopropylamin, N,N-Dimethyl-cyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl- und 3,5-Dimethyl-pyridin, 5-Ethyl-2-methyl-pyridin, 4-Dimethylaminopyridin, N-Methyl-piperidin, N-Ethyl-piperidin, N-Methyl-morpholin, N-Ethylmorpholin, 1,4-Diazabicyclo[2.2.2]-octan (DABCO), 1,5-Diazabicyclo[4.3.0]-non-5-en (DBN), oder 1,8-Diazabicyclo[5.4.0]-undec-7-en (DBU).

Als weitere Reaktionshilfsmittel für das erfindungsgemäße Verfahren kommen auch Phasentransfer-Katalysatoren in Betracht. Als Beispiele für solche Katalysatoren seien genannt:

Tetrabutylammonium-bromid, Tetrabutylammonium-chlorid, Tetraoctylammoniumchlorid, Tetrabutylammonium-hydrogensulfat, Methyl-trioctylammonium-chlorid, Hexadecyl-trimethylammonium-chlorid, Hexadecyl-trimethylammonium-bromid, Benzyl-trimethylammonium-chlorid, Benzyl-triethylammonium-chlorid, Benzyl-trimethylammonium-hydroxid, Benzyl-triethylammonium-hydroxid, Benzyl-tributylammonium-chlorid, Benzyl-tributylammonium-bromid, Tetrabutylphosphoniumbromid, Tetrabutylphosphonium-chlorid, Tributyl-hexadecylphosphonium-bromid, Butyl-triphenylphosphonium-chlorid, Ethyl-trioctylphosphonium-bromid, Tetraphenylphosphonium-bromid.

Das erfindungsgemäße Verfahren zur Herstellung der Verbindungen der allgemeinen Formel (I) wird vorzugsweise jeweils unter Verwendung eines oder mehrerer Verdünnungsmittel durchgeführt. Als Verdünnungsmittel zur Durchführung des erfindungsgemäßen Verfahrens kommen vor allem inerte organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Butyronitril; Amide, wie N,N-Dimethylformamid, N,N-Dimethylacetamid, N-Methyl-formanilid, N-Methyl-pyrrolidon oder Hexamethylphosphorsäuretriamid; Ester wie Essigsäuremethylester oder Essigsäureethylester, Sulfoxide, wie Dimethylsulfoxid, Alkohole, wie Methanol, Ethanol, n- oder i-Propanol, Ethylenglykolmonomethylether, Ethylenglykolmonoethylether, Diethylenglykolmonomethylether, Diethylenglykolmonoethylether.

Die Reaktionstemperaturen können bei der Durchführung des erfindungsgemäßen Verfahrens in einem größeren Bereich variiert werden. Im allgemeinen arbeitet man bei Temperaturen zwischen 0°C und 150°C, vorzugsweise zwischen 10°C und 120°C.

Das erfindungsgemäße Verfahren wird im allgemeinen unter Normaldruck durchgeführt. Es ist jedoch auch möglich, das erfindungsgemäße Verfahren unter erhöhtem oder vermindertem Druck - im allgemeinen zwischen 0,1 bar und 10 bar - durchzuführen.

Zur Durchführung des erfindungsgemäßen Verfahrens werden die Ausgangsstoffe im allgemeinen in angenähert äquimolaren Mengen eingesetzt. Es ist jedoch auch möglich, eine der Komponenten in einem größeren Überschuß zu verwenden. Die Umsetzung wird im allgemeinen in einem geeigneten Verdünnungsmittel in Gegenwart eines Reaktionshilfsmittels durchgeführt und das Reaktionsgemisch wird im allgemeinen mehrere Stunden bei der erforderlichen Temperatur gerührt. Die Aufarbeitung wird nach üblichen Methoden durchgeführt (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab. Die erfindungsgemäßen Wirkstoffe können z.B. bei den folgenden Pflanzen verwendet werden:
Dikotyle Unkräuter der Gattungen: Abutilon, Amaranthus, Ambrosia, Anoda, Anthemis, Aphanes, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.
Dikotyle Kulturen der Gattungen: Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia.
Monokotyle Unkräuter der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.
Monokotyle Kulturen der Gattungen: Allium, Ananas, Asparagus, Avena, Hordeum, Oryza, Panicum, Saccharum, Secale, Sorghum, Triticale, Triticum, Zea.

Die Verwendung der erfindungsgemäßen Wirkstoffe ist jedoch keineswegs auf diese Gattungen beschränkt, sondern erstreckt sich in gleicher Weise auch auf andere Pflanzen.

Die erfindungsgemäßen Wirkstoffe eignen sich in Abhängigkeit von der Konzentration zur Totalunkrautbekämpfung, z.B. auf Industrie- und Gleisanlagen und auf Wegen und Plätzen mit und ohne Baumbewuchs. Ebenso können die erfindungsgemäßen Wirkstoffe zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen sowie zur selektiven Unkrautbekämpfung in einjährigen Kulturen eingesetzt werden.

Die erfindungsgemäßen Verbindungen der Formel (I) zeigen starke herbizide Wirksamkeit und ein breites Wirkungsspektrum bei Anwendung auf dem Boden und auf oberirdische Pflanzenteile. Sie eignen sich in gewissem Umfang auch zur selektiven Bekämpfung von monokotylen und dikotylen Unkräutern in monokotylen und dikotylen Kulturen, sowohl im Vorauflauf- als auch im Nachauflauf-Verfahren.

Die erfindungsgemäßen Wirkstoffe können in bestimmten Konzentrationen bzw. Aufwandmengen auch zur Bekämpfung von tierischen Schädlingen und pilzlichen oder bakteriellen Pflanzenkrankheiten verwendet werden. Sie lassen sich gegebenenfalls auch als Zwischen- oder Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden und/oder mit Stoffen, welche die Kulturpflanzen-Verträglichkeit verbessern ("Safenern") zur Unkrautbekämpfung verwendet werden, wobei Fertigformulierungen oder Tankmischungen möglich sind. Es sind also auch Mischungen mit Unkrautbekämpfungsmitteln möglich, welche ein oder mehrere bekannte Herbizide und einen Safener enthalten.

Für die Mischungen kommen bekannte Herbizide infrage, beispielsweise Acetochlor, Acifluorfen (-sodium), Aclonifen, Alachlor, Alloxydim (-sodium), Ametryne, Amicarbazone, Amidochlor, Amidosulfuron, Anilofos, Asulam, Atrazine, Azafenidin, Azimsulfuron, Beflubutamid, Benazolin (-ethyl), Benfuresate, Bensulfuron (-methyl), Bentazon, Benzfendizone, Benzobicyclon, Benzofenap, Benzoylprop (-ethyl), Bialaphos, Bifenox, Bispyribac (-sodium), Bromobutide, Bromofenoxim, Bromoxynil, Butachlor, Butafenacil (-allyl), Butroxydim, Butylate, Cafenstrole, Caloxydim, Carbetamide, Carfentrazone (-ethyl), Chlomethoxyfen, Chloramben, Chloridazon, Chlorimuron (-ethyl), Chlornitrofen, Chlorsulfuron, Chlortoluron, Cinidon (-ethyl), Cinmethylin, Cinosulfuron, Clefoxydim, Clethodim, Clodinafop (propargyl), Clomazone, Clomeprop, Clopyralid, Clopyrasulfuron (-methyl), Cloransulam (-methyl), Cumyluron, Cyanazine, Cybutryne, Cycloate, Cyclosulfamuron, Cycloxydim, Cyhalofop (-butyl), 2,4-D, 2,4-DB, Desmedipham, Diallate, Dicamba, Dichlorprop (-P), Diclofop (-methyl), Diclosulam, Diethatyl (-ethyl), Difenzoquat, Diflufenican, Diflufenzopyr, Dimefuron, Dimepiperate, Dimethachlor, Dimethametryn, Dimethenamid, Dimexyflam, Dinitramine, Diphenamid, Diquat, Dithiopyr, Diuron, Dymron, Epropodan, EPTC, Esprocarb, Ethalfluralin, Ethametsulfuron (-methyl), Ethofumesate, Ethoxyfen, Ethoxysulfuron, Etobenzanid, Fenoxaprop (-P-ethyl), Fentrazamide, Flamprop (-isopropyl, -isopropyl-L, -methyl), Flazasulfuron, Florasulam, Fluazifop (-P-butyl), Fluazolate, Flucarbazone (-sodium), Flufenacet, Flumetsulam, Flumiclorac (-pentyl), Flumioxazin, Flumipropyn, Flumetsulam, Fluometuron, Fluoröchloridone, Fluoroglycofen (-ethyl), Flupoxam, Flupropacil, Flurpyrsulfuron (-methyl, -sodium), Flurenol (-butyl), Fluridone, Fluroxypyr (-butoxypropyl, -meptyl), Flurprimidol, Flurtamone, Fluthiacet (-methyl), Fluthiamide, Fomesafen, Foramsulfuron, Glufosinate (-ammonium), Glyphosate (-isopropylammonium), Halosafen, Haloxyfop (-ethoxyethyl, -P-methyl), Hexazinone, Imazamethabenz (-methyl), Imazamethapyr, Imazamox, Imazapic, Imazapyr, Imazaquin, Imazethapyr, Imazosulfuron, Iodosulfuron (-methyl, -sodium), Ioxynil, Isopropalin, Isoproturon, Isouron, Isoxaben, Isoxachlortole, Isoxaflutole, Isoxapyrifop, Lactofen, Lenacil, Linuron, MCPA, Mecoprop, Mefenacet, Mesotrione, Metamitron, Metazachlor, Methabenzthiazuron, Metobenzuron, Metobromuron, (alpha-) Metolachlor, Metosulam, Metoxuron, Metribuzin, Metsulfuron (-methyl), Molinate, Monolinuron, Naproanilide, Napropamide, Neburon, Nicosulfuron, Norflurazon, Orbencarb, Oryzalin, Oxadiargyl, Oxadiazon, Oxasulfuron, Oxaziclomefone, Oxyfluorfen, Paraquat, Pelargonsäure, Pendimethalin, Pendralin, Pentoxazone, Phenmedipham, Picolinafen, Piperophos, Pretilachlor, Primisulfuron (-methyl), Profluazol, Prometryn, Propachlor, Propanil, Propaquizafop, Propisochlor, Propoxycarbazone (-sodium), Propyzamide, Prosulfocarb, Prosulfuron, Pyraflufen (-ethyl), Pyrazogyl, Pyrazolate, Pyrazosulfuron (-ethyl), Pyrazoxyfen, Pyribenzoxim, Pyributicarb, Pyridate, Pyridatol, Pyriftalid, Pyriminobac (-methyl), Pyrithiobac (-sodium), Quinchlorac, Quinmerac, Quinoclamine, Quizalofop (-P-ethyl, -P-tefuryl), Rimsulfuron, Sethoxydim, Simazine, Simetryn, Sulcotrione, Sulfentrazone, Sulfometuron (-methyl), Sulfosate, Sulfosulfuron, Tebutam, Tebuthiuron, Tepraloxydim, Terbuthylazine, Terbutryn, Thenylchlor, Thiafluamide, Thiazopyr, Thidiazimin, Thifensulfuron (-methyl), Thiobencarb, Tiocarbazil, Tralkoxydim, Triallate, Triasulfuron, Tribenuron (-methyl), Triclopyr, Tridiphane, Trifluralin, Trifloxysulfuron, Triflusulfuron (-methyl), Tritosulfuron.

Für die Mischungen kommen weiterhin bekannte Safener in Frage, beispielsweise

AD-67, BAS-145138, Benoxacor, Cloquintocet (-mexyl), Cyometrinil, 2,4-D, DKA-24, Dichlormid, Dymron, Fenclorim, Fenchlorazol (-ethyl), Flurazole, Fluxofenim, Furilazole, Isoxadifen (-ethyl), MCPA, Mecoprop (-P), Mefenpyr (-diethyl), MG-191, Oxabetrinil, PPG-1292, R-29148.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit bestimmten Eigenschaften ("Traits"), die sowohl durch konventionelle Züchtung, durch Mutagenese oder durch rekombinante DNA-Techniken erhalten worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel - auch in Kombination mit anderen agrochemischen Wirkstoffen - , besseres Pflanzenwachstum der Kulturpflanzen, erhöhte Toleranz der Kulturpflanzen gegenüber hohen oder niedrigen Temperaturen, erhöhte Toleranz der Kulturpflanzen gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Raps sowie Obstpflanzen (mit den Früchten Äpfel, Birnen, Zitrusfrüchten und Weintrauben) erwähnt, wobei Mais, Soja, Kartoffel, Baumwolle und Raps besonders hervorgehoben werden. Als Eigenschaften ("Traits") werden besonders hervorgehoben die erhöhte Abwehr der Pflanzen gegen Insekten durch in den Pflanzen entstehende Toxine, insbesondere solche, die durch das genetische Material aus *Bacillus thuringiensis* (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja); Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel I bzw. den erfindungsgemäßen Wirkstoffmischungen behandelt werden, wobei zusätzlich der guten Bekämpfung der Unkrautpflanzen die obengenannten synergistischen Effekte mit den transgenen Pflanzen oder Pflanzensorten auftreten. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die Herstellung und die Verwendung der erfindungsgemäßen Wirkstoffe geht aus den nachfolgenden Beispielen hervor.

### Herstellungsbeispiele:

### Beispiel 1-1

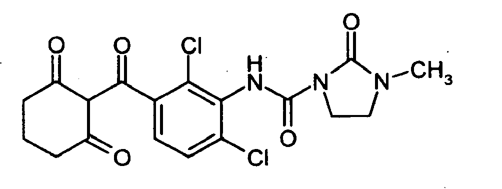

Eine Mischung aus 2,80 g (8,43 mMol) 2,4-Dichlor-3-[[(3-methyl-2-oxo-1-imidazolidinyl)-carbonyl]-amino]-benzoesäure, 0,945 g (8,43 mMol) Cyclohexan-1,3-dion, 2,10 g (10,1 mMol) Dicyclohexylcarbodiimid und 30 ml Acetonitril wird 18 Stunden bei Raumtemperatur (ca. 20°C) gerührt und dann filtriert. Das Filtrat wird mit 0,335 g (3,37 mMol) Trimethylsilylcyanid und 1,70 g (16,9 mMol) Triethylamin versetzt, die Mischung wird 18 Stunden bei Raumtemperatur gerührt und anschließend unter vermindertem Druck eingeengt. Der Rückstand wird mit 10%iger wässriger Natriumcarbonat-Lösung verrührt und dann mit Diethylether geschüttelt. Nach Abtrennen (und Verwerfen) der organischen Phase wird die wässrige Lösung mit konz. Salzsäure angesäuert und das hierbei kristallin angefallene Produkt durch Absaugen isoliert.

Man erhält 2,20 g (49 % der Theorie) N-[2,6-Dichlor-3-[(2,6-dioxo-cyclohexyl)-carbonyl]-phenyl]-3-methyl-2-oxo-1-imidazolidincarboxamid.
logP (pH=2,3): 2,07.

Analog zu Beispiel 1 sowie entsprechend der allgemeinen Beschreibung des erfindungsgemäßen Herstellungsverfahrens können beispielsweise auch die in den nachstehenden Tabellen I-1 bis I-5 aufgeführten Verbindungen der allgemeinen Formel (I) - bzw. der Formeln (I-1), (I-2) oder (I-3) - hergestellt werden.

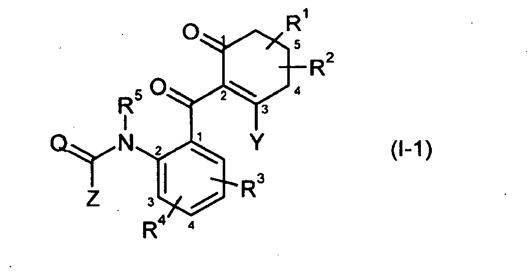

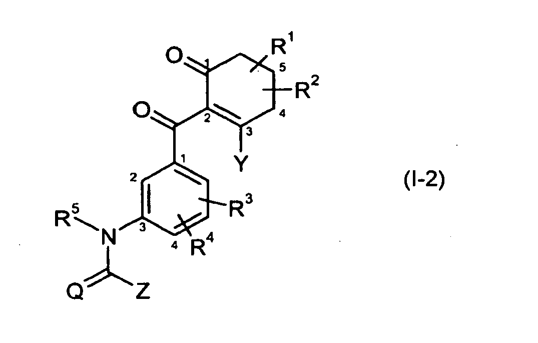

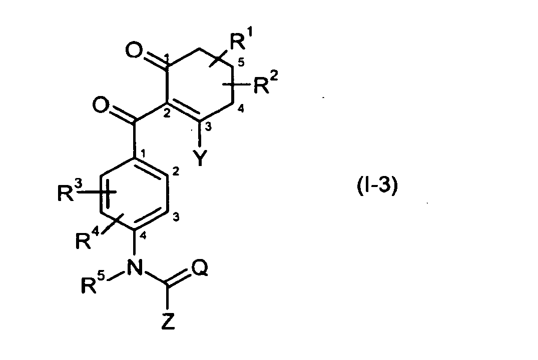

**Tabelle 1-1: Beispiele für die Verbindungen der Formel (I) Hierbei stehen jeweils R¹ und R² für Wasserstoff und Y für Hydroxy.**

| Bsp.-Nr. | Q | (Position) R³ | (Position) R⁴ | R⁵ | Z | Formel Physikal. Daten |
|---|---|---|---|---|---|---|
| 1-2 | O | (2) Cl | (4) Cl | H | 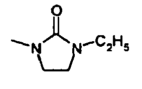 | (I-2) logP = 2,33 ^{a)} |
| 1-4 | O | (2) Cl | (4) Cl | H | 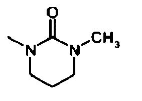 | (I-2) logP = 2,33 ^{a)} |
| 1-5 | O | (2) Cl | (4) Cl | H | 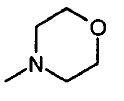 | (I-2) logP = 1,60 ^{a)} |
| 1-11 | O | (2) Cl | (4) Cl | H | 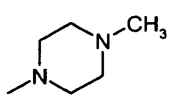 | (I-2) |
| 1-13 | O | (2) Cl | (4) Cl | H | 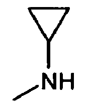 | (I-2) logP = 1,77 ^{a)} |
| 1-14 | O | (2) Cl | (4) Cl | H | 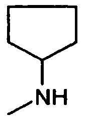 | (I-2) |
| 1-15 | O | (2) Cl | (4) Cl | H | 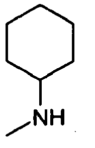 | (I-2) |
| 1-16 | O | (2) Cl | (4) Cl | H | 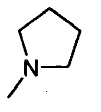 | (I-2) logP = 1,90 ^{a)} |
| 1-17 | O | (2) Cl | (4) Cl | H | 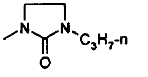 | (I-2) |
| 1-18 | O | (2) Cl | (4) Cl | H | 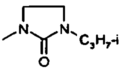 | (I-2) |
| 1-19 | O | (2) Cl | (4) Cl | H | 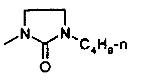 | (I-2) |
| 1-20 | O | (2) Cl | (4) Cl | H | 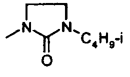 | (I-2) |
| 1-21 | O | (2) Cl | (4) Cl | H | 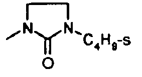 | (I-2) |
| 1-22 | O | (2) Cl | (4) Cl | H | 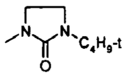 | (I-2) |
| 1-23 | O | (2) Cl | (4) Cl | H | 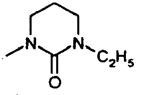 | (I-2) logP = 2,65 a) |
| 1-24 | O | (2) Cl | (4) Cl | H | 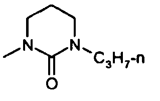 | (I-2) |
| 1-25 | O | (2) Cl | (4) Cl | H | 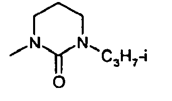 | (I-2) logP = 2,98 a) |
| 1-26 | O | (2) Cl | (4) Cl | H | 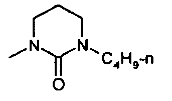 | (I-2) |
| 1-27 | O | (2) Cl | (4) Cl | H | 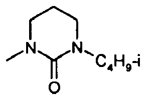 | (I-2) |
| 1-28 | O | (2) Cl | (4) Cl | H | 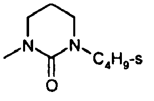 | (I-2) |
| 1-29 | O | (2) Cl | (4) Cl | H | 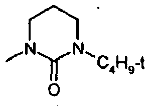 | (I-2) |
| 1-30 | O | (2) Cl | (4) Cl | H | 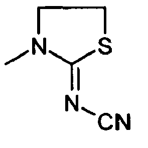 | (I-2) |
| 1-31 | O | (2) Cl | (4) Cl | H | 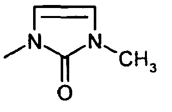 | (I-2) |
| 1-32 | O | (2) Cl | (4) Cl | H | 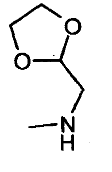 | (I-2) |
| 1-33 | O | (2) Cl | (4) Cl | H | 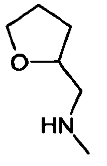 | (I-2) logP = 1,81 a) |
| 1-34 | O | (2) Cl | (4) Cl | H | 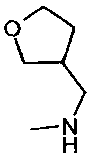 | (I-2) |
| 1-36 | O | (2) Cl | (4) Cl | H | 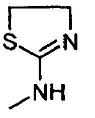 | (I-2) |
| 1-37 | O | (2) Cl | (4) Cl | H | 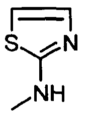 | (I-2) |
| 1-38 | O | (2) Cl | (4) Cl | H | 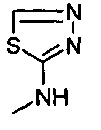 | (I-2) |
| 1-39 | O | (2) Cl | (4) Cl | H | 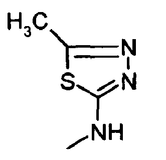 | (I-2) |
| 1-40 | O | (2) Cl | (4) Cl | H | 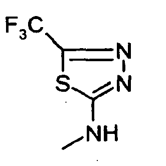 | (I-2) logP = 2,78 a) |
| 1-41 | O | (2) Cl | (4) Cl | H | 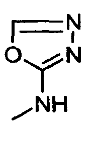 | (I-2) |
| 1-42 | O | (2) Cl | (4) Cl | H | 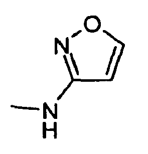 | (I-2) |
| 1-43 | O | (2) Cl | (4) Cl | H | 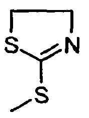 | (I-2) |
| 1-49 | O | (2) Cl | (4) Cl | H | 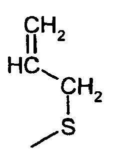 | (I-2) |
| 1-51 | O | (2) Cl | (4) SO₂CH₃ | H | 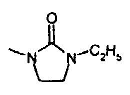 | (I-2) |
| 1-53 | O | (2) Cl | (4) SO₂CH₃ | H | 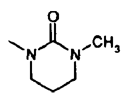 | (I-2) |
| 1-54 | O | (2) Cl | (4) SO₂CH₃ | H | 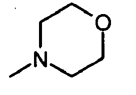 | (I-2) |
| 1-60 | O | (2) Cl | (4) SO₂CH₃ | H | 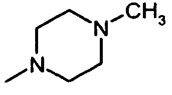 | (I-2) |
| 1-62 | O | (2) Cl | (4) SO₂CH₃ | H | 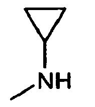 | (I-2) |
| 1-63 | O | (2) Cl | (4) SO₂CH₃ | H | 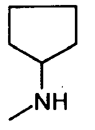 | (I-2) |
| 1-64 | O | (2) Cl | (4) SO₂CH₃ | H | 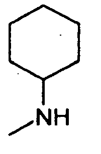 | (I-2) |
| 1-65 | O | (2) Cl | (4) SO₂CH₃ | H | 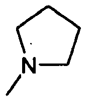 | (I-2) |
| 1-66 | O | (2) Cl | (4) SO₂CH₃ | H | 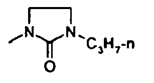 | (I-2) |
| 1-67 | O | (2) Cl | (4) SO₂CH₃ | H | 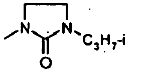 | (I-2) |
| 1-68 | O | (2) Cl | (4) SO₂CH₃ | H | 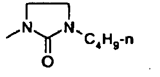 | (I-2) |
| 1-69 | O | (2) Cl | (4) SO₂CH₃ | H | 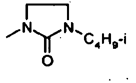 | (I-2) |
| 1-70 | O | (2) Cl | (4) SO₂CH₃ | H | 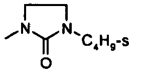 | (I-2) |
| 1-71 | O | (2) Cl | (4) H SO₂CH₃ | H | 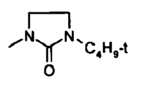 | (I-2) |
| 1-72 | O | (2) Cl | (4) SO₂CH₃ | H | 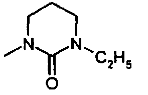 | (I-2) |
| 1-73 | O | (2) Cl | (4) SO₂CH₃ | H | 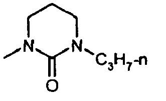 | (I-2) |
| 1-74 | O | (2) Cl | (4) SO₂CH₃ | H | 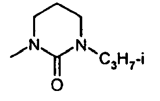 | (I-2) |
| 1-75 | O | (2) Cl | (4) SO₂CH₃ | H | 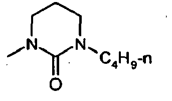 | (I-2) |
| 1-76 | O | (2) Cl | (4) SO₂CH₃ | H | 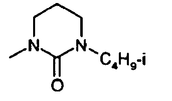 | (I-2) |
| 1-77 | O | (2) Cl | (4) SO₂CH₃ | H | 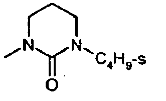 | (I-2) |
| 1-78 | O | (2) Cl | (4) SO₂CH₃ | H | 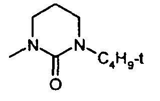 | (I-2) |
| 1-79 | O | (2) Cl | (4) SO₂CH₃ | H | 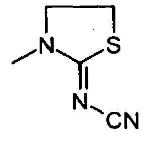 | (I-2) |
| 1-80 | O | (2) Cl | (4) SO₂CH₃ | H | 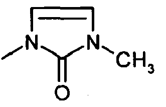 | (I-2) |
| 1-81 | O | (2) Cl | (4) SO₂CH₃ | H | 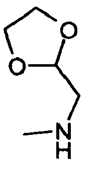 | (I-2) |
| 1-82 | O | (2) Cl | (4) SO₂CH₃ | H | 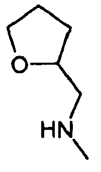 | (I-2) |
| 1-83 | O | (2) Cl | (4) SO₂CH₃ | H | 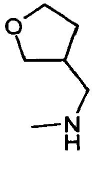 | (I-2) |
| 1-85 | O | (2) Cl | (4) SO₂CH₃ | H | 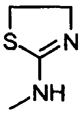 | (I-2) |
| 1-86 | O | (2) Cl | (4) SO₂CH₃ | H | 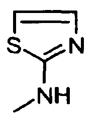 | (I-2) |
| 1-87 | O | (2) Cl | (4) SO₂CH₃ | H | 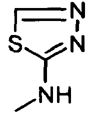 | (I-2) |
| 1-88 | O | (2) Cl | (4) SO₂CH₃ | H | 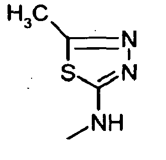 | (I-2) |
| 1-89 | O | (2) Cl | (4) SO₂CH₃ | H | 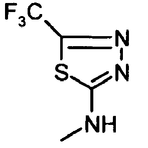 | (I-2) |
| 1-90 | O | (2) Cl | (4) SO₂CH₃ | H | 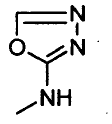 | (I-2) |
| 1-91 | O | (2) Cl | (4) SO₂CH₃ | H | 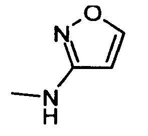 | (I-2) |
| 1-92 | O | (2) Cl | (4) SO₂CH₃ | H | 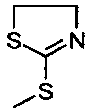 | (I-2) |
| 1-98 | O | (2) Cl | (4) SO₂CH₃ | H | 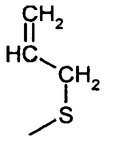 | (I-2) |
| 1-100 | O | (4) CF₃ | - | H | 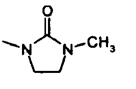 | (I-1) logP = 2,57 a) |
| 1-101 | O | (2) Cl | (4) Cl | H | 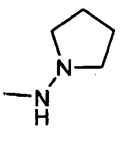 | (I-2) logP = 1,94 a) |
| 1-102 | O | (2) Cl | (4) Cl | H | 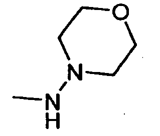 | (I-2) |
| 1-103 | O | (2) Cl | (4) Cl | H | 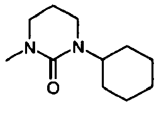 | (I-2) logP = 3,86 a) |
| 1-104 | O | (4) CF₃ | - | CH₃ | 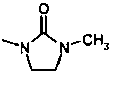 | (I-1) logP = 2,04 a) |
| 1-105 | O | (2) Cl | (4) Cl | H | 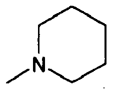 | (I-2) logP = 2,26 a) |
| 1-106 | O | (2) Cl | (4) Cl | H | NH₂ | (I-2) logP = 1,29 a) |
| 1-110 | O | (2) OCH₃ | - | H | 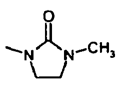 | (I-3) logP = 1,93 a) |
| 1-111 | O | (2) Cl | (4) Cl | H | 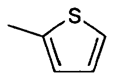 | (I-2) logP = 2,34 a) |
| 1-112 | O | (2) Cl | (4) Cl | H | 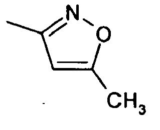 | (I-2) logP = 2,36 a) |
| 1-114 | O | (2) Cl | (4) Cl | H | CH₂OCH₃ | (I-2) |
| 1-121 | O | (2) Cl | (4) Cl | H | CH₂Cl | (I-2) |
| 1-122 | O | (2) Cl | (4) Cl | H | CHCl₂ | (I-2) |
| 1-123 | O | (2) Cl | (4) Cl | H | CCl₃ | (I-2) |
| 1-124 | O | (2) Cl | (4) Cl | H | CF₃ | (I-2) |
| 1-125 | O | (2) Cl | (4) Cl | H | 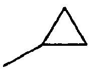 | (I-2) |
| 1-126 | O | (2) Cl | (4) Cl | H | 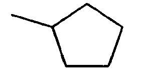 | (I-2) |
| 1-127 | O | (2) Cl | (4) Cl | H | 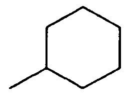 | (I-2) |
| 1-128 | O | (2) Cl | (4) Cl | H | 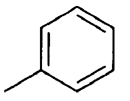 | (I-2) |
| 1-129 | O | (2) Cl | (4) Cl | H | 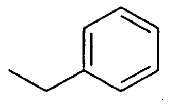 | (I-2) |
| 1-130 | O | (2) Cl | (4) Cl | H | 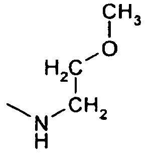 | (I-2) |
| 1-131 | O | (2) Cl | (4) Cl | CH₃ | 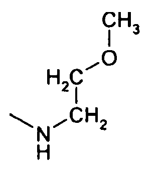 | (I-2) |
| 1-132 | O | (2) Cl | (4) Cl | H | 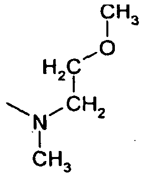 | (I-2) logP = 2,04^{a)} |
| 1-135 | O | (2) Cl | (4) Cl | H | 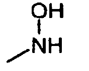 | (I-2) |
| 1-141 | O | (2) Cl | (4) Cl | H | 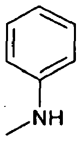 | (I-2) |
| 1-142 | O | (2) Cl | (4) Cl | H | 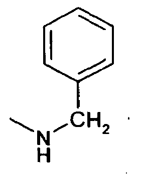 | (I-2) logP = 2,37 a) |
| 1-144 | O | (2) Cl | (4) Cl | H | 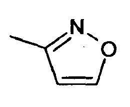 | (I-2) |
| 1-145 | O | (2) Cl | (4) Cl | H | 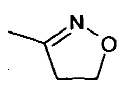 | (I-2) logP = 2,02 a) |
| 1-146 | O | (2) Cl | (4) Cl | H | 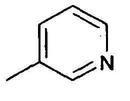 | (I-2) |
| 1-147 | O | (2) Cl | (4) Cl | H | 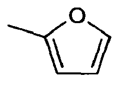 | (I-2) logP = 2,08 a) |
| 1-148 | O | (2) Cl | (4) Cl | H | 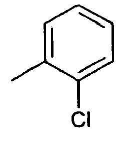 | (I-2) |
| 1-149 | O | (2) Cl | (4) Cl | H | 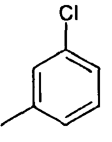 | (I-2) |
| 1-150 | O | (2) Cl | (4) Cl | H | 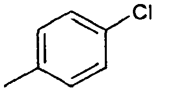 | (I-2) logP = 2,96 a) |
| 1-151 | O | (2) Cl | (4) Cl | H | 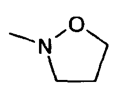 | (I-2) logP = 1,93 a) |
| 1-152 | O | (2) Cl | (4) SO₂CH₃ | H | 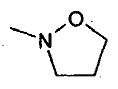 | (I-2) |
| 1-153 | O | (4) CF₃ | - | H | 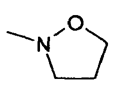 | (I-1) |
| 1-154 | O | (2) OCH₃ | - | H | 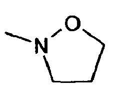 | (I-3) |
| 1-155 | O | (2) NO₂ | - | H | 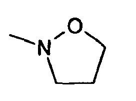 | (I-3) |
| 1-160 | O | (2) Cl | (4) Cl | H | 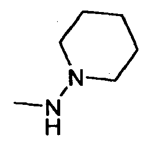 | (I-2) logP = 2,52 a) |
| 1-162 | O | (2) Cl | (4) Cl | H | 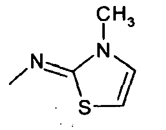 | (I-2) logP = 1,96 a) |
| 1-163 | O | (2) Cl | (4) Cl | H | 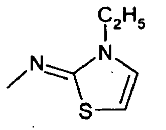 | (I-2) logP = 2,22 a) |
| 1-164 | O | (2) Cl | (4) Cl | H | 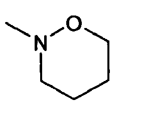 | (I-2) logP = 2,17 a) |
| 1-171 | O | (2) Cl | (4) Cl | H | 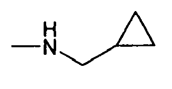 | (I-2) logP = 2,05 a) |
| 1-181 | O | (2) Cl | (4) SO₂CH₃ | H | 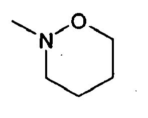 | (I-2) |
| 1-182 | O | (2) Cl | (4) Cl | H | 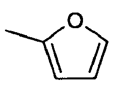 | (I-2) |
| 1-183 | O | (2) Cl | (4) SO₂CH₃ | H | 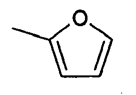 | (I-2) |
| 1-184 | O | (2) Cl | (4) SO₂CH₃ | H | 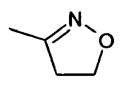 | (I-2) |
| 1-185 | O | (2) Cl | (4) SO₂CH₃ | H | 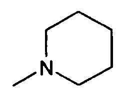 | (I-2) |
| 1-186 | O | (2) Cl | (4) SO₂CH₃ | H | 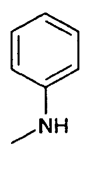 | (I-2) |
| 1-187 | O | (2) Cl | (4) Cl | H | 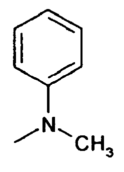 | (I-2) |
| 1-188 | O | (2) Cl | (4) SO₂CH₃ | H | 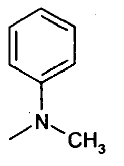 | (I-2) |
| 1-189 | O | (2) Cl | (4) Cl | H | 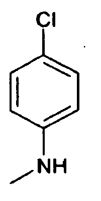 | (I-2) |
| 1-190 | O | (2) Cl | (4) SO₂CH₃ | H | 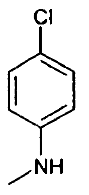 | (I-2) |
| 1-191 | O | (2) Cl | (4) Cl | H | 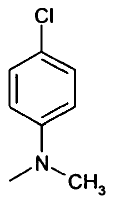 | (I-2) |
| 1-192 | O | (2) Cl | (4) SO₂CH₃ | H | 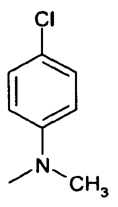 | (I-2) |
| 1-193 | O | (2) Cl | (4) Cl | H | 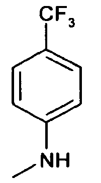 | (I-2) |
| 1-194 | O | (2) Cl | (4) SO₂CH₃ | H | 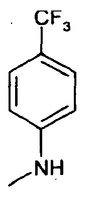 | (I-2) |
| 1-195 | O | (2) Cl | (4) Cl | H | 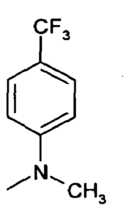 | (I-2) |
| 1-196 | O | (2) Cl | (4) SO₂CH₃ | H | 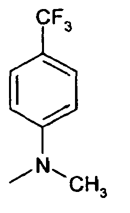 | (I-2) |
| 1-197 | O | (2) Cl | (4) Cl | H | 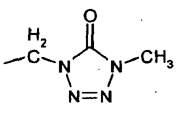 | (I-2) Fp.: 195°C |
| 1-198 | O | (2) Cl | (4) Cl | CH₃ | 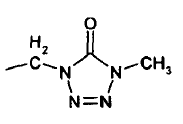 | (I-2) |
| 1-190 | O | (2) Cl | (4) Cl | H | 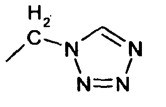 | (I-2) |
| 1-200 | O | (2) Cl | (4) Cl | H | 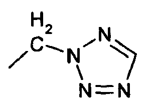 | (I-2) |
| 1-201 | O | (2) Cl | (4) Cl | CH₃ | 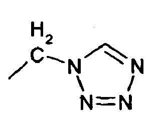 | (I-2) |
| 1-202 | O | (2) Cl | (4) Cl | CH₃ | 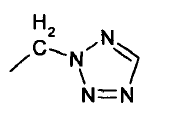 | (I-2) |
| 1-203 | O | (2) Cl | (4) SO₂CH₃ | H | 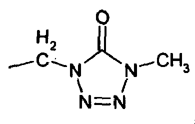 | (I-2) |
| 1-204 | O | (2) Cl | (4) SO₂CH₃ | CH₃ | 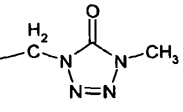 | (I-2) |
| 1-205 | O | (2) Cl | (4) SO₂CH₃ | H | 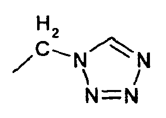 | (I-2) |
| 1-206 | O | (2) Cl | (4) SO₂CH₃ | H | 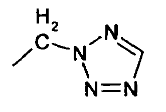 | (I-2) |
| 1-207 | O | (2) Cl | (4) SO₂CH₃ | CH₃ | 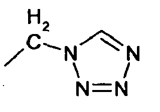 | (I-2) |
| 1-208 | O | (2) Cl | (4) SO₂CH₃ | CH₃ | 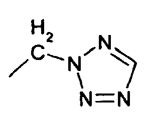 | (I-2) |

**Tabelle 1-2: Beispiele für die Verbindungen der Formel (I) Hierbei stehen jeweils R¹ für Methyl in 4-Position, R² für Methyl in 4-Position und Y für Hydroxy.**

| Bsp.- Nr. | Q | (Position) R³ | (Position) R⁴ | R⁵ | Z | Formel Physikal. Daten |
|---|---|---|---|---|---|---|
| 2-2 | O | (2) Cl | (4) Cl | H | 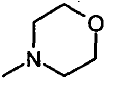 | (I-2) |
| 2-3 | O | (2) Cl | (4) Cl | H | 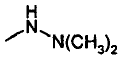 | (I-2) |
| 2-8 | O | (2) Cl | (4) Cl | H | 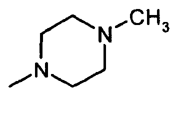 | (I-2) |
| 2-10 | O | (2) Cl | (4) Cl | H | 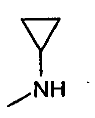 | (I-2) |
| 2-11 | O | (2) Cl | (4) Cl | H | 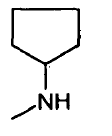 | (I-2) |
| 2-12 | O | (2) Cl | (4) Cl | H | 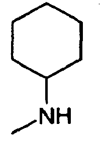 | (I-2) |
| 2-13 | O | (2) Cl | (4) Cl | H | 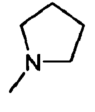 | (I-2) |
| 2-14 | O | (2) Cl | (4) Cl | H | 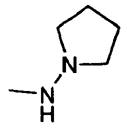 | (I-2) |
| 2-15 | O | (2) Cl | (4) Cl | H | 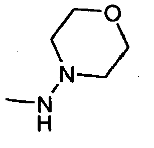 | (I-2) |
| 2-16 | O | (2) Cl | (4) Cl | H | 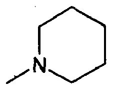 | (I-2) |
| 2-17 | O | (2) Cl | (4) Cl | H | NH₂ | (I-2) |
| 2-21 | O | (2) Cl | (4) Cl | H | 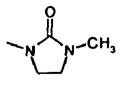 | (I-3) |
| 2-22 | O | (2) Cl | (4) Cl | H | 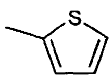 | (I-2) |
| 2-23 | O | (2) Cl | (4) Cl | H | 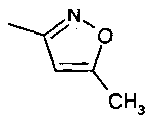 | (I-2) |
| 2-25 | O | (2) Cl | (4) Cl | H | CH₂OCH₃ | (I-2) |
| 2-31 | O | (2) Cl | (4) Cl | H | CH₂Cl | (I-2) |
| 2-32 | O | (2) Cl | (4) Cl | H | CHCl₂ | (I-2) |
| 2-33 | O | (2) Cl | (4) Cl | H | CCl₃ | (I-2) |
| 2-34 | O | (2) Cl | (4) Cl | H | CF₃ | (I-2) |
| 2-35 | O | (2) Cl | (4) Cl | H | 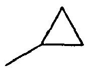 | (I-2) |
| 2-36 | O | (2) Cl | (4) Cl | H | 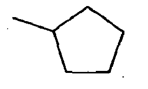 | (I-2) |
| 2-37 | O | (2) Cl | (4) Cl | H | 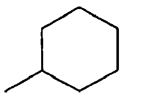 | (I-2) |
| 2-38 | O | (2) Cl | (4) Cl | H | 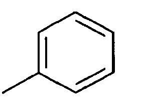 | (I-2) |
| 2-39 | O | (2) Cl | (4) Cl | H | 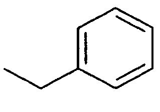 | (I-2) |
| 2-45 | O | (2) Cl | (4) Cl | H | 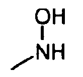 | (I-2) |
| 2-50 | O | (2) Cl | (4) Cl | H | 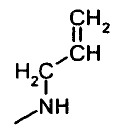 | (I-2) |
| 2-51 | O | (2) Cl | (4) Cl | H | 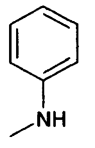 | (I-2) |
| 2-52 | O | (2) Cl | (4) Cl | H | 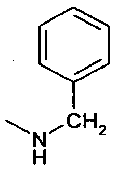 | (I-2) |
| 2-54 | O | (2) Cl | (4) Cl | H | 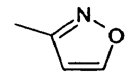 | (I-2) |
| 2-55 | O | (2) Cl | (4) Cl | H | 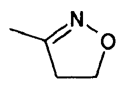 | (I-2) |
| 2-56 | O | (2) Cl | (4) Cl | H | 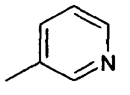 | (I-2) |
| 2-57 | O | (2) Cl | (4) Cl | H | 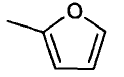 | (I-2) |
| 2-58 | O | (2) Cl | (4) Cl | H | 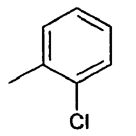 | (I-2) |
| 2-59 | O | (2) Cl | (4) Cl | H | 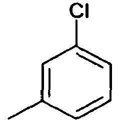 | (I-2) |
| 2-60 | O | (2) Cl | (4) Cl | H | 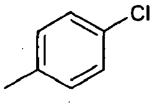 | (I-2) |
| 2-61 | O | (2) Cl | (4) Cl | H | 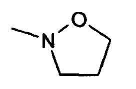 | (I-2) logP = 2,57 ^{a)} |
| 2-62 | O | (2) Cl | (4) SO₂CH₃ | H | 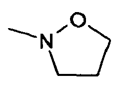 | (I-2) |
| 2-63 | O | (4) CF₃ | - | H | 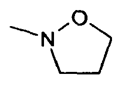 | (I-1) |
| 2-64 | O | (2) OCH₃ | - | H | 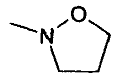 | (I-3) |
| 2-65 | O | (2) NO₂ | - | H | 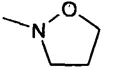 | (I-3) |
| 2-69 | O | (2) Cl | (4) Cl | H | 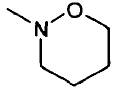 | (I-2) logP = 2,83 ^{a)} |
| 2-70 | O | (2) Cl | (4) SO₂CH₃ | H | 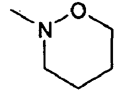 | (I-2) |
| 2-71 | O | (2) Cl | (4) SO₂CH₃ | H | 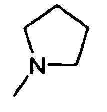 | (I-2) |
| 2-73 | O | (2) Cl | (4) SO₂CH₃ | H | 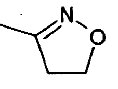 | (I-2) |
| 2-74 | O | (2) Cl | (4) SO₂CH₃ | H | 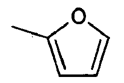 | (I-2) |

**Tabelle 1-3: Beispiele für die Verbindungen der Formel (I) Hierbei stehen jeweils R¹ für Methyl in 5-Position, R² für Wasserstoff und Y für Hydroxy.**

| Bsp.-Nr. | Q | (Position) R³ | (Position) R⁴ | R⁵ | Z | Formel Physikal. Daten |
|---|---|---|---|---|---|---|
| 3-2 | O | (2) Cl | (4) Cl | H | 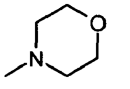 | (I-2) |
| 3-8 | O | (2) Cl | (4) Cl | H | 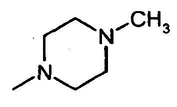 | (I-2) |
| 3-9 | O | (2) Cl | (4) Cl | H | NHC₃H₇-i | (I-2) |
| 3-10 | O | (2) Cl | (4) Cl | H | 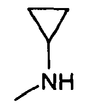 | (I-2) |
| 3-11 | O | (2) Cl | (4) Cl | H | 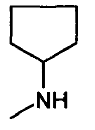 | (I-2) |
| 3-12 | O | (2) Cl | (4) Cl | H | 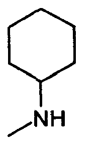 | (I-2) |
| 3-13 | O | (2) Cl | (4) Cl | H | 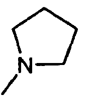 | (I-2) |
| 3-14 | O | (2) Cl | (4) Cl | H | 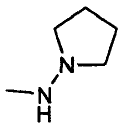 | (I-2) |
| 3-15 | O | (2) Cl | (4) Cl | H | 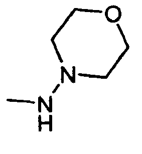 | (I-2) |
| 3-16 | O | (2) Cl | (4) Cl | H | 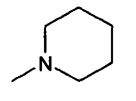 | (I-2) |
| 3-17 | O | (2) Cl | (4) Cl | H | NH₂ | (I-2) |
| 3-21 | O | (2) Cl | (4) Cl | H | 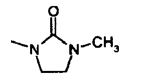 | (I-3) |
| 3-22 | O | (2) Cl | (4) Cl | H | 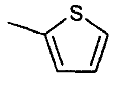 | (I-2) |
| 3-23 | O | (2) Cl | (4) Cl | H | 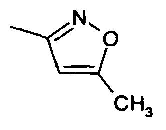 | (I-2) |
| 3-25 | O | (2) Cl | (4) Cl | H | CH₂OCH₃ | (I-2) |
| 3-31 | O | (2) Cl | (4) Cl | H | CH₂Cl | (I-2) |
| 3-32 | O | (2) Cl | (4) Cl | H | CHCl₂ | (I-2) |
| 3-33 | O | (2) Cl | (4) Cl | H | CCl₃ | (I-2) |
| 3-34 | O | (2) Cl | (4) Cl | H | CF₃ | (I-2) |
| 3-35 | O | (2) Cl | (4) Cl | H | 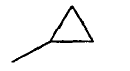 | (I-2) |
| 3-36 | O | (2) Cl | (4) Cl | H | 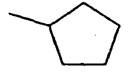 | (I-2) |
| 3-37 | O | (2) Cl | (4) Cl | H | 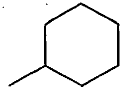 | (I-2) |
| 3-38 | O | (2) Cl | (4) Cl | H | 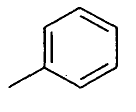 | (I-2) |
| 3-39 | O | (2) Cl | (4) Cl | H | 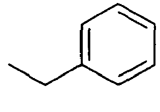 | (I-2) |
| 3-45 | O | (2) Cl | (4) Cl | H | 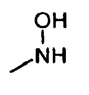 | (I-2) |
| 3-50 | O | (2) Cl | (4) Cl | H | 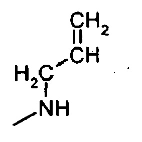 | (I-2) |
| 3-51 | O | (2) Cl | (4) Cl | H | 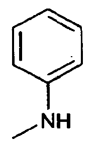 | (I-2) |
| 3-52 | O | (2) Cl | (4) Cl | H | 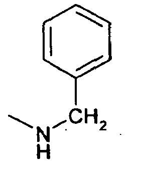 | (I-2) |
| 3-54 | O | (2) Cl | (4) Cl | H | 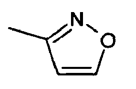 | (I-2) |
| 3-55 | O | (2) Cl | (4) Cl | H | 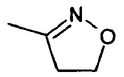 | (I-2) |
| 3-56 | O | (2) Cl | (4) Cl | H | 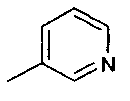 | (I-2) |
| 3-57 | O | (2) Cl | (4) Cl | H | 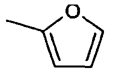 | (I-2) |
| 3-58 | O | (2) Cl | (4) Cl | H | 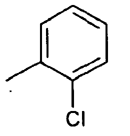 | (I-2) |
| 3-59 | O | (2) Cl | (4) Cl | H | 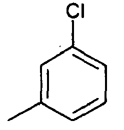 | (I-2) |
| 3-60 | O | (2) Cl | (4) Cl | H | 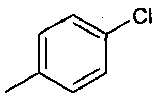 | (I-2) |
| 3-61 | O | (2) Cl | (4) Cl | H | 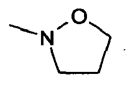 | (I-2) logP = 2,25 ^{a)} |
| 3-62 | O | (2) Cl | (4) SO₂CH₃ | H | 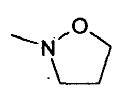 | (I-2) |
| 3-62 | O | (4) CF₃ | - | H | 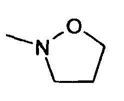 | (I-1) |
| 3-64 | O | (2) OCH₃ | - | H | 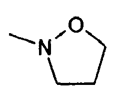 | (I-3) |
| 3-65 | O | (2) NO₂ | - | H | 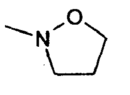 | (I-3) |
| 3-69 | O | (2) Cl | (4) Cl | H | 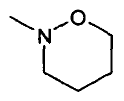 | (I-2) logP = 2,52 ^{a)} |
| 3-70 | O | (2) Cl | (4) SO₂CH₃ | H | 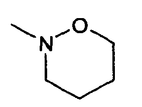 | (I-2) |
| 3-71 | O | (2) Cl | (4) SO₂CH₃ | H | 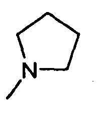 | (I-2) |
| 3-73 | O | (2) Cl | (4) SO₂CH₃ | H | 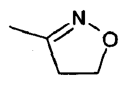 | (I-2) |
| 3-74 | O | (2) Cl | (4) SO₂CH₃ | H | 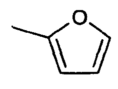 | (I-2) |

**Tabelle 1-4: Beispiele für die Verbindungen der Formel (I) Hierbei stehen jeweils R¹ für Methyl in 5-Position, R² für Methyl in 5-Position und Y für Hydroxy.**

| Bsp.-Nr. | Q | (Position) R³ | (Position) R⁴ | R⁵ | Z | Formel Physikal. Daten |
|---|---|---|---|---|---|---|
| 4-2 | O | (2) Cl | (4) Cl | H | 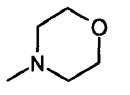 | (I-2) |
| 4-8 | O | (2) Cl | (4) Cl | H | 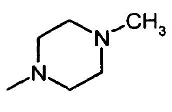 | (I-2) |
| 4-10 | O | (2) Cl | (4) Cl | H | 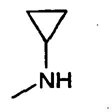 | (I-2) |
| 4-11 | O | (2) Cl | (4) Cl | H | 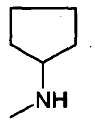 | (I-2) |
| 4-12 | O | (2) Cl | (4) Cl | H | 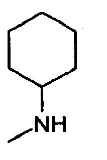 | (I-2) |
| 4-13 | O | (2) Cl | (4) Cl | H | 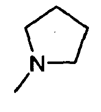 | (I-2) |
| 4-14 | O | (2) Cl | (4) Cl | H | 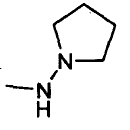 | (I-2) |
| 4-15 | O | (2) Cl | (4) Cl | H | 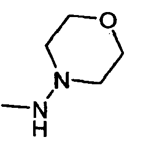 | (I-2) |
| 4-16 | O | (2) Cl | (4) Cl | H | 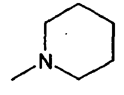 | (I-2) |
| 4-17 | O | (2) Cl | (4) Cl | H | NH₂ | (I-2) |
| 4-21 | O | (2) Cl | (4) Cl | H | 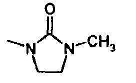 | (I-3) |
| 4-22 | O | (2) Cl | (4) Cl | H | 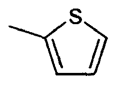 | (I-2) |
| 4-23 | O | (2) Cl | (4) Cl | H | 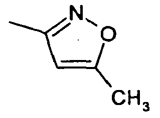 | (I-2) |
| 4-25 | O | (2) Cl | (4) Cl | H | CH₂OCH₃ | (I-2) |
| 4-31 | O | (2) Cl | (4) Cl | H | CH₂Cl | (I-2) |
| 4-32 | O | (2) Cl | (4) Cl | H | CHCl₂ | (I-2) |
| 4-33 | O | (2) Cl | (4) Cl | H | CCl₃ | (I-2) |
| 4-34 | O | (2) Cl | (4) Cl | H | CF₃ | (I-2) |
| 4-35 | O | (2) Cl | (4) Cl | H | 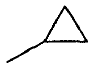 | (I-2) |
| 4-36 | O | (2) Cl | (4) Cl | H | 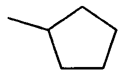 | (I-2) |
| 4-37 | O | (2) Cl | (4) Cl | H | 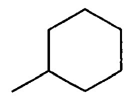 | (I-2) |
| 4-38 | O | (2) Cl | (4) Cl | H | 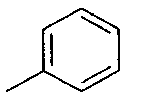 | (I-2) |
| 4-39 | O | (2) Cl | (4) Cl | H | 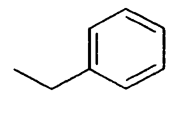 | (I-2) |
| 4-45 | O | (2) Cl | (4) Cl | H | 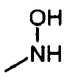 | (I-2) |
| 4-50 | O | (2) Cl | (4) Cl | H | 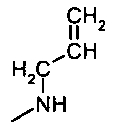 | (I-2) |
| 4-51 | O | (2) Cl | (4) Cl | H | 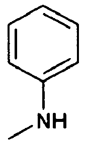 | (I-2) |
| 4-52 | O | (2) Cl | (4) Cl | H | 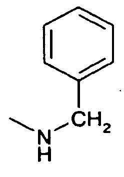 | (I-2) |
| 4-54 | O | (2) Cl | (4) Cl | H | 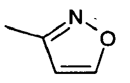 | (I-2) |
| 4-55 | O | (2) Cl | (4) Cl | H | 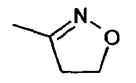 | (I-2) logP = 2,59 ^{a)} |
| 4-56 | O | (2) Cl | (4) Cl | H | 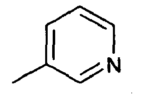 | (I-2) |
| 4-57 | O | (2) Cl | (4) Cl | H | 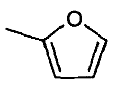 | (I-2) |
| 4-58 | O | (2) Cl | (4) Cl | H | 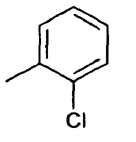 | (I-2) |
| 4-59 | O | (2) Cl | (4) Cl | H | 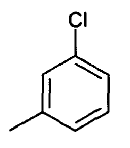 | (I-2) |
| 4-60 | O | (2) Cl | (4) Cl | H | 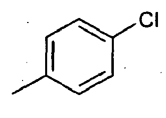 | (I-2) |
| 4-61 | O | (2) Cl | (4) Cl | H | 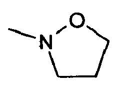 | (I-2) logP = 2,51 ^{a)} |
| 4-62 | O | (2) Cl | (4) SO₂CH₃ | H | 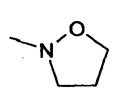 | (I-2) |
| 4-63 | O | (4) CF₃ | - | H | 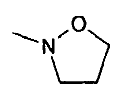 | (I-1) |
| 4-64 | O | (2) OCH₃ | - | H | 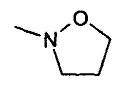 | (I-3) |
| 4-65 | O | (2) NO₂ | - | H | 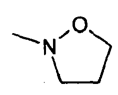 | (I-3) |
| 4-69 | O | (2) Cl | (4) Cl | H | 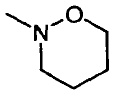 | (I-2) logP = 2,76 ^{a)} |
| 4-70 | O | (2) Cl | (4) SO₂CH₃ | H | 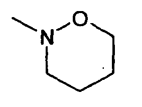 | (I-2) |
| 4-71 | O | (2) Cl | (4) SO₂CH₃ | H | 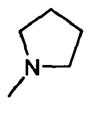 | (I-2) |
| 4-73 | O | (2) Cl | (4) SO₂CH₃ | H | 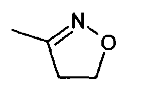 | (I-2) |
| 4-74 | O | (2) Cl | (4) SO₂CH₃ | H | 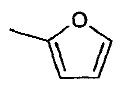 | (I-2) |

**Tabelle 1-5: Beispiele für die Verbindungen der Formel (I) Hierbei stehen jeweils R¹ und R² für eine Dimethylen-Gruppierung zwischen den Positionen 4 und 6 - "(4)-CH₂CH₂-(6)" und Y für Hydroxy.**

| Bsp.-Nr. | Q | (Position) R³ | (Position) R⁴ | R⁵ | Z | Formel Physikal. Daten |
|---|---|---|---|---|---|---|
| 5-2 | O | (2) Cl | (4) Cl | H | 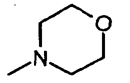 | (I-2) |
| 5-8 | O | (2) Cl | (4) Cl | H | 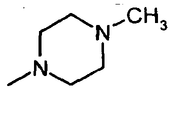 | (I-2) |
| 5-10 | O | (2) Cl | (4) Cl | H | 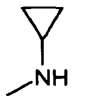 | (I-2) |
| 5-11 | O | (2) Cl | (4) Cl | H | 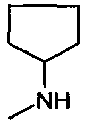 | (1-2) |
| 5-12 | O | (2) Cl | (4) Cl | H | 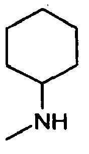 | (1-2) |
| 5-13 | O | (2) Cl | (4) Cl | H | 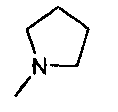 | (I-2) |
| 5-14 | O | (2) Cl | (4) Cl | H | 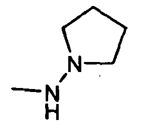 | (1-2) |
| 5-15 | O | (2) Cl | (4) Cl | H | 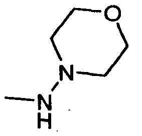 | (I-2) |
| 5-16 | O | (2) Cl | (4) Cl | H | 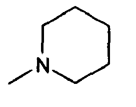 | (1-2) |
| 5-17 | O | (2) Cl | (4) Cl | H | NH₂ | (1-2) |
| 5-21 | O | (2) Cl | (4) Cl | H | 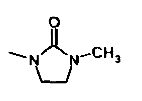 | (I-3) |
| 5-22 | O | (2) Cl | (4) Cl | H | 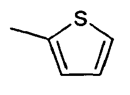 | (I-2) |
| 5-23 | O | (2) Cl | (4) Cl | H | 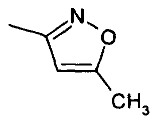 | (1-2) |
| 5-25 | O | (2) Cl | (4) Cl | H | CH₂OCH₃ | (1-2) |
| 5-31 | O | (2) Cl | (4) Cl | H | CH₂Cl | (1-2) |
| 5-32 | O | (2) Cl | (4) Cl | H | CHCl₂ | (I-2) |
| 5-33 | O | (2) Cl | (4) Cl | H | CCl₃ | (1-2) |
| 5-34 | O | (2) Cl | (4) Cl | H | CF₃ | (1-2) |
| 5-35 | O | (2) Cl | (4) Cl | H | 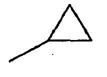 | (I-2) |
| 5-36 | O | (2) Cl | (4) Cl | H | 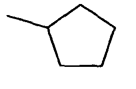 | (I-2) |
| 5-37 | O | (2) Cl | (4) Cl | H | 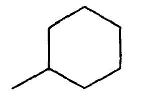 | (I-2) |
| 5-38 | O | (2) Cl | (4) Cl | H | 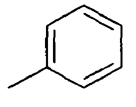 | (I-2) |
| 5-39 | O | (2) Cl | (4) Cl | H | 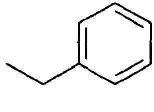 | (I-2) |
| 5-45 | O | (2) Cl | (4) Cl | H | 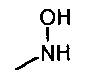 | (I-2) |
| 5-50 | O | (2) Cl | (4) Cl | H | 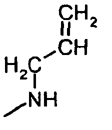 | (I-2) |
| 5-51 | O | (2) Cl | (4) Cl | H | 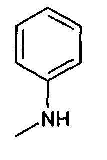 | (I-2) |
| 5-52 | O | (2) Cl | (4) Cl | H | 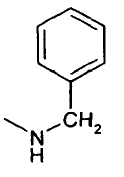 | (1-2) |
| 5-54 | O | (2) Cl | (4) Cl | H | 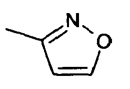 | (I-2) |
| 5-55 | O | (2) Cl | (4) Cl | H | 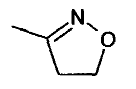 | (I-2) |
| 5-56 | O | (2) Cl | (4) Cl | H | 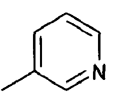 | (I-2) |
| 5-57 | O | (2) Cl | (4) Cl | H | 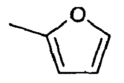 | (I-2) |
| 5-58 | O | (2) Cl | (4) Cl | H | 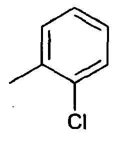 | (I-2) |
| 5-59 | O | (2) Cl | (4) Cl | H | 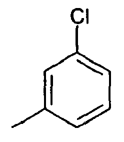 | (I-2) |
| 5-60 | O | (2) Cl | (4) Cl | H | 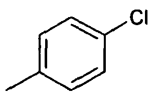 | (I-2) |
| 5-61 | O | (2) Cl | (4) Cl | H | 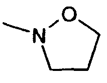 | (I-2) |
| 5-62 | O | (2) Cl | (4) SO₂CH₃ | H | 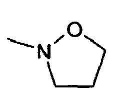 | (I-2) |
| 5-63 | O | (4) CF₃ | - | H | 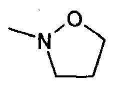 | (I-1) |
| 5-64 | O | (2) OCH₃ | - | H | 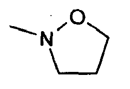 | (I-3) |
| 5-65 | O | (2) NO₂ | - | H | 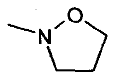 | (I-3) |
| 5-69 | O | (2) Cl | (4) Cl | H | 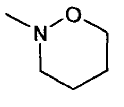 | (I-2) |
| 5-70 | O | (2) Cl | (4) SO₂CH₃ | H | 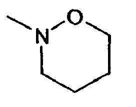 | (I-2) |
| 5-71 | O | (2) Cl | (4) SO₂CH₃ | H | 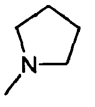 | (I-2) |
| 5-73 | O | (2) Cl | (4) SO₂CH₃ | H | 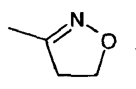 | (1-2) |
| 5-74 | O | (2) Cl | (4) SO₂CH₃ | H | 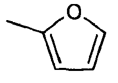 | (I-2) |

Die Bestimmung der in den Tabellen 1-1, 1-2, 1-3, 1-4 angegebenen logP-Werte erfolgte gemäß EEC-Directive 79/831 Annex V.A8 durch HPLC (High Performance Liquid Chromatography) an einer Phasenumkehrsäule (C 18). Temperatur: 43°C.
(a) Eluenten für die Bestimmung im sauren Bereich: 0,1% wässrige Phosphorsäure, Acetonitril; linearer Gradient von 10% Acetonitril bis 90% Acetonitril - entsprechende Messergebnisse sind in Tabelle 1 mit ^{a)} markiert.
(b) Eluenten für die Bestimmung im neutralen Bereich: 0,01-molare wässrige Phosphatpuffer-Lösung, Acetonitril; linearer Gradient von 10% Acetonitril bis 90% Acetonitril - entsprechende Messergebnisse sind in Tabelle 1 mit ^{b)} markiert.

Die Eichung erfolgte mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinanderfolgenden Alkanonen).

Die lambda-max-Werte wurden an Hand der UV-Spektren von 200 nm bis 400 nm in den Maxima der chromatographischen Signale ermittelt.

### Ausgangsstoffe der Formel (III):

### Beispiel (III-1)

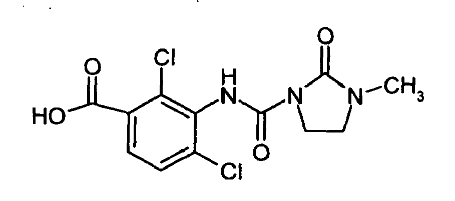

Eine Mischung aus 11,3 g (32,9 mMol) 2,4-Dichlor-3-[[(3-methyl-2-oxo-1-imidazolidinyl)-carbonyl]-amino]-benzoesäure-methylester, 50 ml Wasser, 50 ml Tetrahydrofuran und 1,3 g Natriumhydroxid wird 18 Stunden bei Raumtemperatur (ca. 20°C) gerührt und anschließend unter vermindertem Druck auf etwa das halbe Volumen eingeengt. Dann wird mit Diethylether geschüttelt, die organische Phase abgetrennt (und verworfen) und die wässrige Phase mit konz. Salzsäure angesäuert. Das hierbei kristallin angefallene Produkt wird durch Absaugen isoliert.

Man erhält 9,1 g (81,5 % der Theorie) 2,4-Dichlor-3-[[(3-methyl-2-oxo-1-imidazolidinyl)-carbonyl]-amino]-benzoesäure.
logP (pH=2,3): 1,35.

Analog zu Beispiel (III-1) können beispielsweise auch die in der nachstehenden Tabelle 2 aufgeführten Verbindungen der allgemeinen Formel (III) hergestellt werden.

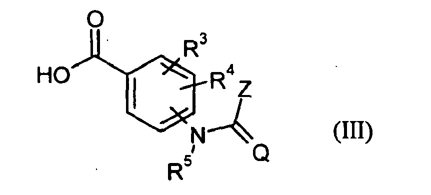

**Tabelle 2: Beispiele für die Verbindungen der Formel (III)**

| Bsp.- Nr. | (Position R³ | (Position) R⁴ | (Position) 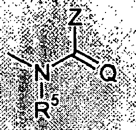 | Physikal Daten |
|---|---|---|---|---|
| III-3 | (2) Cl | (4) Cl | 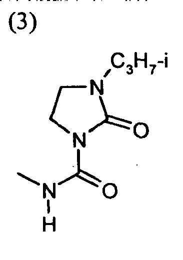 | |
| III-4 | (2) Cl | (4) Cl | 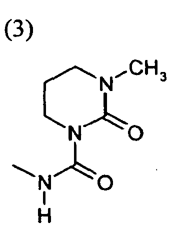 | logP=1,58 ^{a)} |
| III-5 | (2) Cl | (4) Cl | 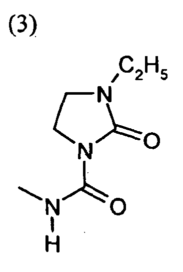 | |
| III-6 | (2) Cl | (4) Cl | 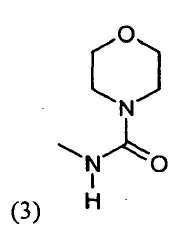 | logP = 0,78 ^{a)} |
| III-8 | (2) Cl | (4) Cl | 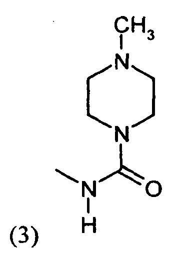 | |
| III-11 | (4) CF₃ | - | 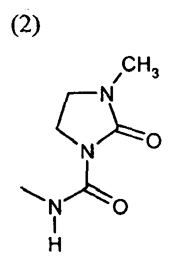 | logP = 2,13 ^{a)} |
| III-12 | (4) CF₃ | - | 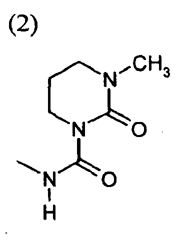 | |
| III-13 | (4) CF3 | - | 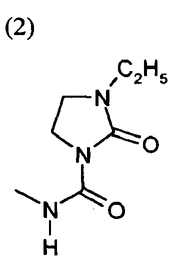 | |
| III-14 | (4) CF₃ | - | 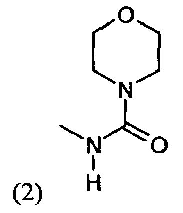 | |
| III-16 | (4) CF₃ | - | 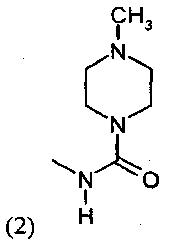 | |
| III-18 | (2) Cl | (4) Cl | 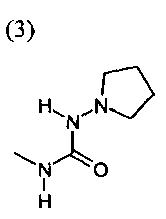 | logP = 1,16 ^{a)} |
| III-19 | (2) Cl | (4) Cl | 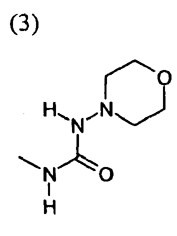 | logP = 1,03 ^{a)} |

### Ausgangsstoffe der Formel (IIIa):

### Beispiel (IIIa-1)

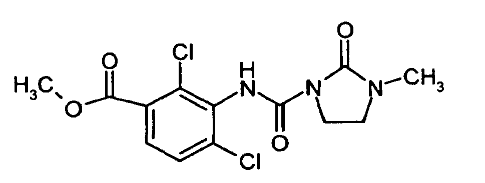

Eine Mischung aus 12,3 g (50 mMol) 2,4-Dichlor-3-isocyanato-benzoesäure-methylester, 5,0. g (50 mMol) 1-Methyl-2-oxo-imidazolidin, einigen Tropfen Triethylamin und 100 ml Acetonitril wird 18 Stunden bei Raumtemperatur (ca. 20°C) gerührt und anschließend unter vermindertem Druck eingeengt. Der Rückstand wird dann mit Diethylether digeriert und das kristalline Produkt durch Absaugen isoliert.
Man erhält 11,4 g (60 % der Theorie) 2,4-Dichlor-3-[[(3-methyl-2-oxo-1-imidazolidinyl)-carbonyl]-amino]-benzoesäure-methylester.
logP (pH=2,3): 1,94.

Analog zu Beispiel (IIIa-1) können beispielsweise auch die in der nachstehenden Tabelle 3 aufgeführten Verbindungen der allgemeinen Formel (III) hergestellt werden.

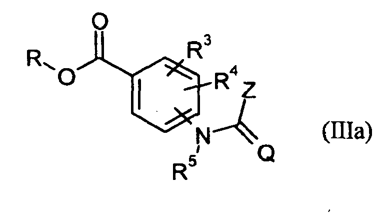

**Tabelle 3: Beispiele für die Verbindungen der Formel (IIIa)**

| Bsp.- Nr. | R | (Position) R³ | (Position) R⁴ | (Position) 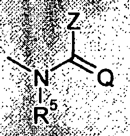 | Physikal. Daten |
|---|---|---|---|---|---|
| IIIa-3 | CH₃ | (2) Cl | (4) Cl | 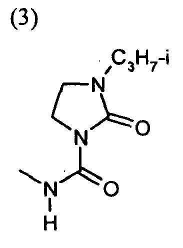 | |
| IIIa-4 | CH₃ | (2) Cl | (4) Cl | 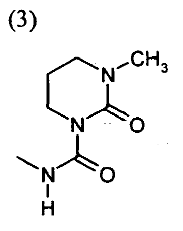 | |
| IIIa-5 | CH₃ | (2) Cl | (4) Cl | 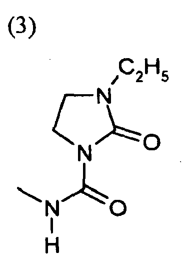 | |
| IIIa-6 | CH₃ | (2) Cl | (4) Cl | 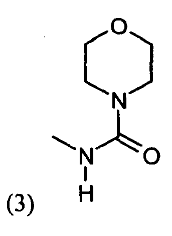 | logP = 1,34 ^{a)} |
| IIIa-8 | CH₃ | (2) Cl | (4) Cl | 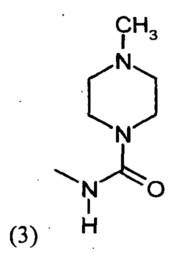 | logP = 0,50 ^{a)} |
| IIIa-11 | CH₃ | (4) CF₃ | - | 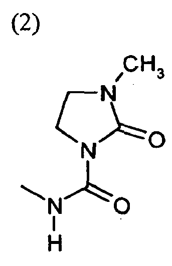 | logP = 2,76 ^{a)} |
| IIIa-12 | CH₃ | (4) CF₃ | - | 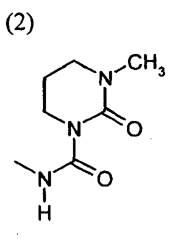 | |
| IIIa-13 | CH₃ | (4) CF₃ | - | 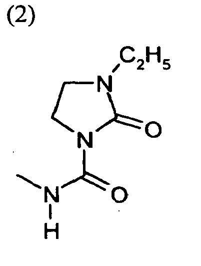 | |
| IIIa-14 | CH₃ (4) | CF₃ | - | 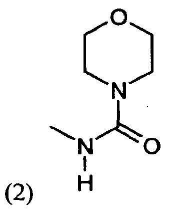 | |
| IIIa-16 | CH₃ | (4) CF₃ | - | 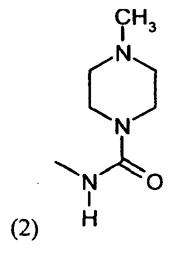 | |
| IIIa-18 | CH₃ | (2) Cl | (4) Cl | 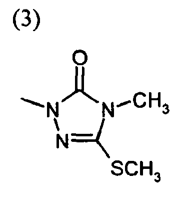 | |
| IIIa-19 | CH₃ | (2) Cl | (4) Cl | 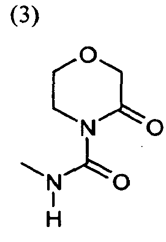 | |
| IIIa-20 | CH₃ | (2) Cl | (4) Cl | 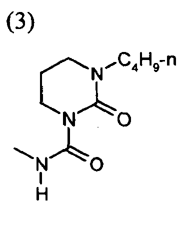 | logP = 3,45 ^{a)} |
| IIIa-21 | CH₃ | (2) Cl | (4) Cl | 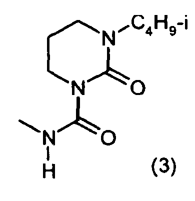 | logP = 3,43 ^{a)} |
| IIIa-22 | CH₃ | (2) Cl | (4) Cl | 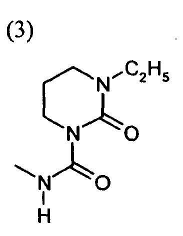 | |
| IIIa-23 | CH₃ | (2) Cl | (4) Cl | 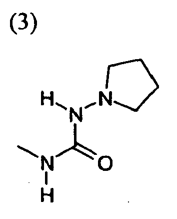 | logP = 1,55 ^{a)} |
| IIIa-24 | CH₃ | (2) Cl | (4) Cl | 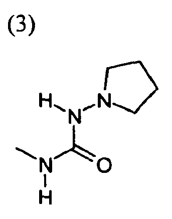 | logP = 1,80 ^{a)} |
| IIIa-25 | CH₃ | (2) Cl | (4) Cl | 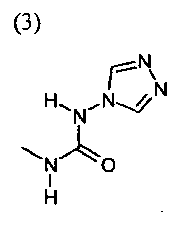 | logP = 0,93 ^{a)} |

### Anwendungsbeispiele:

### Beispiel A

### Pre-emergence-Test

| | |
|---|---|
| Lösungsmittel: | 5 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach 24 Stunden wird der Boden so mit der Wirkstoffzubereitung besprüht, daß die jeweils gewünschte Wirkstoffmenge pro Flächeneinheit ausgebracht wird. Die Wirkstoffkonzentration in der Spritzbrühe wird so gewählt, daß in 1000 Liter Wasser pro Hektar die jeweils gewünschte Wirkstoffmenge ausgebracht wird.

Nach drei Wochen wird der Schädigungsgrad der Pflanzen bonitiert in % Schädigung im Vergleich zur Entwicklung der unbehandelten Kontrolle. Es bedeuten:

| | |
|---|---|
| 0% = | keine Wirkung (wie unbehandelte Kontrolle) |
| 100 % = | totale Vernichtung |

In diesem Test zeigen beispielsweise die Verbindungen gemäß Herstellungsbeispiele 1-1, 1-2, 1-4 und 1-5 guter Verträglichkeit gegenüber Kulturpflanzen, wie z.B. Mais, starke Wirkung gegen Unkräuter.

### Beispiel B

### Post-emergence-Test

| | |
|---|---|
| Lösungsmittel: | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 bis 15 cm haben so, dass die jeweils gewünschten Wirkstoffmengen pro Flächeneinheit ausgebracht werden. Die Konzentration der Spritzbrühe wird so gewählt, dass in 1 0001 Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle

Es bedeuten:

| | |
|---|---|
| 0% = | keine Wirkung (wie unbehandelte Kontrolle) |
| 100 % = | totale Vernichtung |

In diesem Test zeigen beispielsweise die Verbindungen der Herstellungsbeispiele 1, 2, 4 bei guter Verträglichkeit gegenüber Kulturpflanzen, starke Wirkung gegen Unkräuter und Ungräser.

### Beispiel C

### Versuch in gesätem Reis (Gewächshaus)

| | |
|---|---|
| Lösungsmittel: | 5 Gewichtsteile Aceton |
| Emulgator: | 1 Gewichtsteil Alkylarylpolyglykolether |

Zur Herstellung einer zweckmäßigen Spritzzubereitung werden 1 Gewichtsteil Wirkstoff mit der angegebenen Menge Lösungsmittel und Emulgator versetzt und durch Vermischen mit Wasser auf die gewünschte Konzentration verdünnt.

Pflanzengefäße (Größe: 20 cm x 20 cm x 9 cm; Oberfläche: 1/200 Ar) werden mit boden aus einem Reisfeld gefüllt. Samen von Reis und Unkräutern werden in die feucht gehaltene Erde ausgesät. Im 1,5 - 2 Blattstadium von Reis wird die verdünnte Wirkstoffzubereitung in Form einer Spritzung (Blattbehandlung) ausgebracht.

Einen Tag nach der Behandlung werden die Testgefäße bis zu einer Wassertiefe von 3 cm überstaut. Anschließend werden die Testansätze unter Überflutungsbedingungen gehalten (Wassertiefe 3 cm).

4 Wochen nach der Wirkstoffapplikation wird der Schädigungsgrad der Pflanzen bonitiert in % Schädigung (bzw. Unkrautwirkung) im Vergleich zu einer unbehandelten Kontrolle.

Es bedeuten:

| | |
|---|---|
| 0% = | keine Wirkung (wie unbehandelte Kontrolle) |
| 100 % = | totale Vernichtung |

In diesem Test zeigen beispielsweise die Verbindungen der Herstellungsbeispiele 1, 2, 4, 16, 23 bei guter Verträglichkeit gegenüber Reis starke Wirkung gegen Unkräuter.

## Patentansprüche

1. Verbindungen der Formel (I) 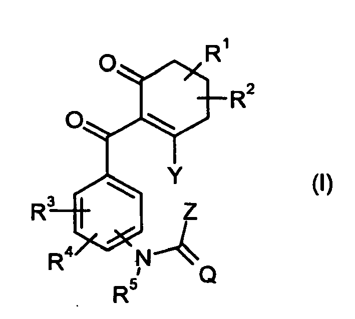 in welcher
Q für O (Sauerstoff) oder S (Schwefel) steht,
R¹ für Wasserstoff, Fluor, Chlor oder Brom, für jeweils gegebenenfalls durch Fluor, Chlor, Methoxy, Ethoxy, n- oder i-Propoxy, Methylthio, Ethylthio, n- oder i-Propylthio, Methylsulfinyl, Ethylsulfinyl, Methyl- sulfonyl oder Ethylsulfonyl substituiertes Methyl, Ethyl, n- oder i- Propyl, n-, i-, s- oder t-Butyl, Methylthio, Ethylthio, n- oder i-Propyl- thio, n-, i-, s- oder t-Butylthio, oder für Phenyl steht,
R² für Wasserstoff, Fluor, Chlor oder Brom, für jeweils gegebenenfalls durch Fluor oder Chlor substituiertes Methyl, Ethyl, n- oder i-Propyl, n-, i-, s- oder t-Butyl, oder gegebenenfalls auch zusammen mit R¹ für O (Sauerstoff), Ethan-1,2-diyl, Propan-1,3-diyl oder Butan-1,4-diyl steht,
R³ für Wasserstoff, Nitro, Cyano, Carboxy, Carbamoyl, Thiocarbamoyl, Fluor, Chlor, Brom, Iod, für jeweils gegebenenfalls durch Fluor, Chlor, Methoxy, Ethoxy, Methylthio, Ethylthio, Methylsulfinyl, Ethylsulfinyl, Methylsulfonyl oder Ethylsulfonyl substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethyl- thio, n- oder i-Propylthio, n-, i-, s- oder t-Butylthio, Methylsulfinyl, Ethylsulfinyl, n- oder i-Propylsulfinyl, Methylsulfonyl, Ethylsulfonyl, n- oder i-Propylsulfonyl, für Methylamino, Ethylamino, n- oder i- Propylamino, n-, i-, s- oder t-Butylamino, Dimethylamino, Diethyl- amino, Dimethylaminocarbonyl, Diethylaminocarbonyl, Dimethyl- aminosulfonyl oder Diethylaminosulfonyl steht,
R⁴ für Wasserstoff, Nitro, Cyano, Carboxy, Carbamoyl, Thiocarbamoyl, Fluor, Chlor, Brom, Iod, für jeweils gegebenenfalls durch Fluor, Chlor, Methoxy, Ethoxy, Methylthio, Ethylthio, Methylsulfinyl, Ethylsulfinyl, Methylsulfonyl oder Ethylsulfonyl substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethyl- thio, n- oder i-Propylthio, n-, i-, s- oder t-Butylthio, Methylsulfinyl, Ethylsulfinyl, n- oder i-Propylsulfinyl, Methylsulfonyl, Ethylsulfonyl, n- oder i-Propylsulfonyl, für Methylamino, Ethylamino, n- oder i- Propylamino, n-, i-, s- oder t-Butylamino, Dimethylamino, Diethyl- amino, Dimethylaminocarbonyl, Diethylaminocarbonyl, Dimethyl- aminosulfonyl oder Diethylaminosulfonyl steht,
R⁵ für Wasserstoff, für jeweils gegebenenfalls durch Cyano, Fluor, Chlor, Brom, Methoxy, Ethoxy, n- oder i-Propoxy substituiertes Methyl, Ethyl, n- oder i-Propyl, n-, i-, s- oder t-Butyl, n-, i-, s- oder t-Pentyl, Methoxy, Ethoxy, n- oder i-Propoxy, n-, i-, s- oder t-Butoxy, Methyl- thio, Ethylthio, n- oder i-Propylthio, n-, i-, s- oder t-Butylthio, Methyl- sulfinyl, Ethylsulfinyl, n- oder i-Propylsulfinyl, Methylsulfonyl, Ethylsulfonyl, Methylamino, Ethylamino, n- oder i-Propylamino, n-, i-, s- oder t-Butylamino, für Dimethylamino oder Diethylamino, für jeweils gegebenenfalls durch Fluor, Chlor und/oder Brom substitu- iertes Ethenyl, Propenyl, Butenyl, Pentenyl, Ethinyl, Propinyl, Butinyl oder Pentinyl, für jeweils gegebenenfalls durch Cyano, Fluor, Chlor, Brom, Methyl oder Ethyl substituiertes CyClopropyl, CyClobutyl, CyClopentyl, CyClohexyl, CyClopropylmethyl, CyClobutylmethyl, CyClopentylmethyl oder CyClohexylmethyl, oder für jeweils gege- benenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substitu- iertes Phenyl, Phenylthio, Phenylsulfinyl, Phenylsulfonyl, Naphthyl, Phenylmethyl, Phenylethyl, Naphthylmethyl oder Naphthylethyl oder für Gruppierung -C(Q)-Z steht,
Y für Hydroxy, Formyloxy, Fluor, Chlor oder Brom, für jeweils gege- benenfalls durch Cyano, Fluor, Chlor, Methoxy, Ethoxy, n- oder i- Propoxy, Methylthio, Ethylthio, n- oder i-Propylthio, Methylsulfinyl, Ethylsulfinyl, Methylsulfonyl oder Ethylsulfonyl substituiertes Methoxy, Ethoxy, n- oder i-Propoxy, Methylthio, Ethylthio, n- oder i- Propylthio, Methylsulfinyl, Ethylsulfinyl, Methylsulfonyl, Ethyl- sulfonyl, Acetyloxy, Propionyloxy, n- oder i-Butyroyloxy, Methoxy- carbonyloxy, Ethoxycarbonyloxy, n- oder i-Propoxycarbonyloxy, Methylaminocarbonyloxy, Ethylaminocarbonyloxy, n- oder i-Propyl- aminocarbonyloxy, Methylsulfonyloxy, Ethylsulfonyloxy, n- oder i- Propylsulfonyloxy, für jeweils gegebenenfalls durch Fluor, Chlor und/oder Brom substituiertes Propenyloxy, Butenyloxy, Propinyloxy oder Butinyloxy, für jeweils gegebenenfalls durch Nitro, Cyano, Fluor, Chlor, Brom, Methyl, Ethyl, n- oder i-Propyl, n-, i-, s- oder t- Butyl, Trifluormethyl, Methoxy, Ethoxy, n- oder i-Propoxy, Difluor- methoxy, Trifluormethoxy, Methylthio, Ethylthio, n- oder i-Propyl- thio, Difluormethylthio, Trifluormethylthio, Methylsulfinyl, Ethyl- sulfmyl, Trifluormethylsulfinyl, Methylsulfonyl, Ethylsulfonyl oder Trifluormethylsulfonyl substituiertes Phenyloxy, Phenylthio, Phenyl- sulfinyl, Phenylsulfonyl, Phenylcarbonyloxy, Phenylcarbonylmeth- oxy, Phenylsulfonyloxy, Phenylmethoxy, Phenylmethylthio, Phenyl- methylsulfinyl oder Phenylmethylsulfonyl steht, und
Z für Wasserstoff, Amino, Cyanoamino, Nitroamino, Hydroxyamino, Hydrazino,
für durch Cyano, Fluor, Chlor, Methoxy, Ethoxy, n- oder i-Propoxy, Methyl- thio, Ethylthio, n- oder i-Propylthio, Methylsulfinyl oder Ethylsulfonyl substituiertes Methyl,
für jeweils gegebenenfalls durch Fluor, Chlor und/oder Brom substituiertes Ethenyl, Propenyl, Butenyl, Pentenyl, Ethinyl, Propinyl, Butinyl, Pentinyl, Propenyloxy, Butenyloxy, Pentenyloxy, Propenylthio, Butenylthio, Pentenyl- thio, Propenylamino, Butenylamino, Pentenylamino, Propenyloxyamino, Butenyloxyamino, Pentenyloxyamino, Ethinyl, Propinyl, Butinyl, Pentinyl, Propinyloxy, Butinyloxy, Pentinyloxy, Propinylamino, Butinylamino oder Pentinylamino,
für jeweils gegebenenfalls durch Cyano, Fluor, Chlor, Brom, Methyl oder Ethyl substituiertes CyClopropyl, CyClobutyl, CyClopentyl, CyClohexyl, CyClopropyloxy, CyClobutyloxy, CyClopentyloxy, CyClohexyloxy, CyClo- propylamino, CyClobutylamino, CyClopentylamino, CyClohexylamino, CyClo- propylhydrazino, CyClobutylhydrazino, CyClopentylhydrazino, CyClohexyl- hydrazino, CyClopropylmethyl, CyClobutylmethyl, CyClopentylmethyl, CyClohexylmethyl, CyClopropylmethoxy, CyClobutylmethoxy, CyClopentyl- methoxy, CyClohexylmethoxy, CyClopropylmethylamino, CyClobuylmethyl- amino, CyClopentylmethylamino oder CyClohexylmethylamino,
für jeweils gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy, Trifluormethoxy, Methoxy- carbonyl, Ethoxycarbonyl, n- oder i-Propoxycarbonyl substituiertes Phenyl, Phenylcarbonyl, Phenoxy, Phenoxycarbonyl, Phenylthio, Phenylamino, Phenylhydrazino, Naphthyl, Naphthyloxy, Naphtylthio, Naphthylamino, Phenylmethyl, Phenylethyl, Phenylmethoxy, Phenylethoxy, Phenylmethyl- thio, Phenylethylthio, Phenylmethylamino, Phenylethylamino, Naphthyl- methyl, Naphthylethyl, Naphthylmethoxy, Naphthylethoxy, Naphthylmethyl- amino oder Naphthylethylamino, oder
für jeweils gegebenenfalls durch Nitro, Cyano, Fluor, Chlor, Brom, Methyl, Ethyl, n- oder i-Propyl, n-, i-, s- oder t-Butyl, Difluormethyl, Trifluormethyl, Dichlormethyl, Trichlormethyl, Chlordifluormethyl, Fluordichlormethyl, Methoxy, Ethoxy, n- oder i-Propoxy, n-, i-, s- oder t-Butoxy, Difluor- methoxy, Trifluormethoxy, Methylthio, Ethylthio, n- oder i-Propylthio, n-, i-, s- oder t-Butylthio, Difluormethylthio, Trifluormethylthio, Methoxycarbonyl, Ethoxycarbonyl, n- oder i-Propoxycarbonyl substituiertes monocyClisches oder bicyClisches HeterocyClyl, HeterocyClyloxy, HeterocyClylamino, die Gruppierung -N=(HeterocyClyl), HeterocyClylalkyl, HeterocyClylalkoxy oder HeterocyClylalkylamino aus der Reihe Furyl, Tetrahydrofuryl, Furyloxy, Tetrahydrofuryloxy, Furylamino, Tetrahydrofurylamino, Furylmethyl, Tetra- hydrofurylmethyl, Furylmethoxy, Tetrahydrofurylmethoxy, Furylmethyl- amino, Tetrahydrofurylmethylamino, Dioxolanyl, Dioxolanylmethyl, Di- oxolanylmethoxy, Dioxolanylmethylamino, Thienyl, Thienylamino, Thienyl- methyl, Thenylmethylamino, Dithiolanyl, Dithiolanylmethyl, Dithiolanyl- methoxy, Dithiolanylmethylamino, Pyrrolidinyl, Pyrrolidinylamino, Oxo- pyrrolidinyl, Pyrrolyl, Indolyl, Pyrrolylmethyl, Pyrazolyl, Pyrazolyloxy, Pyrazolylamino, Pyrazolylmethyl, Imidazolyl, Imidazolinyl, Imidazolyl- methyl, Imidazolinylmethyl, Oxoimidazolinyl, 2-Oxo-1,3-diaza-cyClopentyl, Oxazolyl, Oxazolylamino, Dihydrooxazolyl (Oxazolinyl), Tetrahydro- oxazolyl, (Oxazolidinyl), Isoxazolyl, Isoxazolylamino, Dihydroisoxazolyl (Isoxazolinyl), Tetrahydroisoxazolyl (Isoxazolidinyl), Tetrahydro-(2H)-1,2- oxazin-2-yl, Oxazolylmethyl, Thiazolyl, Thiazolylamino, Thiazolylmethyl, Dihydrothiazolyl (Thiazolinyl), Tetrahydrothiazolyl (Thiazolidinyl), Thiazolimino, Thiazolinylthio, Thiazolinylamino, Oxothiazolidinyl, Cyano- iminothiazolidinyl, Oxadiazolylamino, Thiadiazolylamino, Triazolylamino, Oxotriazolinyl, Thioxotriazolinyl, Oxotetrazolinyl, Oxotetrazolinylmethyl, Tetrazolylmethyl, Dioxanyl, Dioxanylmethyl, Dioxanylmethoxy, Dioxanyl- methylamino, Dithianyl, Dithianylmethyl, Dithianylmethoxy, Dithianyl- methylamino, Piperidinyl, Piperidinylamino, Oxopiperidinyl, 2-Oxo-1,3- diaza-cyClohexyl, 2-Oxo-1-aza-cyCloheptyl, 2-Oxo-1,3-diaza-cyCloheptyl, Morpholinyl, Morpholinylamino, Piperazinyl, Pyridinyl, Pyridinyloxy, Pyridinylthio, Pyridinylamino, 2-(1H)-Pyridinimino, Pyridinylmethyl, Pyridinylmethoxy, Pyrimidinyl, Pyrimidinyloxy, Pyrimidinylthio, Pyri- midinylamino, Pyrimidinylmethyl oder Pyrimidinylmethoxy steht, einschließlich aller möglichen tautomeren Formen der Verbindungen der all- gemeinen Formel (I) und der möglichen Salze der Verbindungen der allge- meinen Formel (I).

2. Verbindungen der Formel (I) gemäß Anspruch 1, **dadurch gekennzeichnet, dass**
R¹ für Wasserstoff, für jeweils gegebenenfalls durch Fluor oder Chlor substituiertes Methyl, Ethyl, n- oder i-Propyl, Methylthio, Ethylthio, n- oder i-Propylthio, oder für Phenyl steht,
R² für Wasserstoff, für jeweils gegebenenfalls durch Fluor oder Chlor substituiertes Methyl, Ethyl, n- oder i-Propyl, oder gegebenenfalls auch zusammen mit R¹ für Ethan-1,2-diyl, Propan-1,3-diyl oder Butan-1,4-diyl steht,
R³ für Wasserstoff, Nitro, Cyano, Fluor, Chlor, Brom, Iod, Methyl, Ethyl, n- oder i-Propyl, Difluormethyl, Trifluormethyl, Dichlormethyl, Tri- chlormethyl, Methoxymethyl, Methylthiomethyl, Methylsulfinyl- methyl, Methylsulfonylmethyl, Methoxy, Ethoxy, Difluormethoxy, Trifluormethoxy, Methylthio, Ethylthio, Methylsulfinyl, Ethylsulfinyl, Methylsulfonyl, Ethylsulfonyl oder Dimethylaminosulfonyl steht,
R⁴ für Wasserstoff, Nitro, Cyano, Fluor, Chlor, Brom, Iod, Methyl, Ethyl, n- oder i-Propyl, Difluormethyl, Trifluormethyl, Dichlormethyl, Tri- chlormethyl, Methoxymethyl, Methylthiomethyl, Methylsulfinyl- methyl, Methylsulfonylmethyl, Methoxy, Ethoxy, Difluormethoxy, Trifluormethoxy, Methylthio, Ethylthio, Methylsulfinyl, Ethylsulfinyl, Methylsulfonyl, Ethylsulfonyl oder Dimethylaminosulfonyl,
R⁵ für Wasserstoff, für jeweils gegebenenfalls durch Cyano, Fluor, Chlor, Methoxy oder Ethoxy substituiertes Methyl, Ethyl, n- oder i-Propyl, n- oder i-Butyl, für Methoxy, Ethoxy, n- oder i-Propoxy, Methylthio, Ethylthio, n- oder i-Propylthio, Methylsulfinyl, Ethylsulfinyl, n- oder i-Propylsulfinyl, Methylsulfonyl, Ethylsulfonyl, Methylamino, Ethyl- amino, n- oder i-Propylamino oder Dimethylamino, für jeweils gege- benenfalls durch Fluor und/oder Chlor substituiertes Propenyl, Butenyl, Propinyl oder Butinyl, für jeweils gegebenenfalls durch Cyano, Fluor, Chlor oder Methyl substituiertes CyClopropyl, CyClo- butyl, CyClopentyl, CyClohexyl, CyClopropylmethyl, CyClobutyl- methyl, CyClopentylmethyl oder CyClohexylmethyl, oder für jeweils gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluor- methoxy substituiertes Phenyl, Phenylthio, Phenylsulfinyl, Phenyl- sulfonyl, Phenylmethyl oder Phenylethyl, oder für Gruppierung -C(Q)-Z steht,
Y für Hydroxy, für jeweils gegebenenfalls durch Fluor und/oder Chlor substituiertes Methoxy, Ethoxy, n- oder i-Propoxy, Methylthio, Ethyl- thio, n- oder i-Propylthio, Methylsulfinyl, Ethylsulfinyl, Methyl- sulfonyl, Ethylsulfonyl, Acetyloxy, Propionyloxy, n- oder i-Butyroyl- oxy, Methoxycarbonyloxy, Ethoxycarbonyloxy, n- oder i-Propoxy- carbonyloxy, Methylaminocarbonyloxy, Ethylaminocarbonyloxy, n- oder i-Propylaminocarbonyloxy, Methylsulfonyloxy, Ethylsulfonyl- oxy, n- oder i-Propylsulfonyloxy, für jeweils gegebenenfalls durch Fluor, Chlor und/oder Brom substituiertes Propenyloxy, Butenyloxy, Propinyloxy oder Butinyloxy, für jeweils gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Di- fluormethoxy, Trifluormethoxy, Methylthio, Ethylthio, n- oder i- Propylthio, Difluormethylthio, Trifluormethylthio, Methylsulfinyl, Ethylsulfinyl, Trifluormethylsulfinyl, Methylsulfonyl, Ethylsulfonyl oder Trifluormethylsulfonyl substituiertes Phenyloxy, Phenylthio, Phenylsulfinyl, Phenylsulfonyl, Phenylcarbonyloxy, Phenylcarbonyl- methoxy, Phenylsulfonyloxy, Phenylmethoxy, Phenylmethylthio, Phenylmethylsulfinyl oder Phenylmethylsulfonyl steht, und
Z für Amino, Cyanoamino, Hydrazino,
für durch Cyano, Fluor, Chlor, Methoxy, Ethoxy, n- oder i-Propoxy, Methylthio, Ethylthio, n- oder i-Propylthio, Methylsulfinyl, Ethylsulfinyl, n- oder i-Propylsulfinyl, Methylsulfonyl oder Ethylsulfonyl substituiertes Methyl,
für jeweils gegebenenfalls durch Fluor und/oder Chlor substituiertes Ethenyl, Propenyl, Butenyl, Ethinyl, Propinyl, Butinyl, Propenyloxy, Butenyloxy, Propenylthio, Butenylthio, Propenylamino, Butenylamino, Propenyloxyamino, Butenyloxyamino, Ethinyl, Propinyl, Butinyl, Propinyloxy, Butinyloxy, Propinylamino oder Butinylamino,
für jeweils gegebenenfalls durch Cyano, Fluor, Chlor oder Methyl substituiertes CyClopropyl, CyClobutyl, CyClopentyl, CyClohexyl, CyClopropyloxy, CyClobutyloxy, CyClopentyloxy, CyClohexyloxy, CyClopropylamino, CyClobutylamino, CyClopentylamino, CyClohexylamino, CyClopentylhydrazino, CyClohexylhydrazino, CyClopropylmethyl, CyClobutylmethyl, CyClopentylmethyl, CyClohexylmethyl, CyClopropylmethoxy, CyClobutylmethoxy, CyClopentylmethoxy, CyClohexylmethoxy, CyClopropylmethylamino, CyClobuylmethylamino, CyClopentylmethylamino oder CyClohexylmethylamino,
für jeweils gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy, Trifluormethoxy, Methoxycarbonyl, Ethoxycarbonyl, n- oder i-Propoxycarbonyl substituiertes Phenyl, Phenylcarbonyl, Phenoxy, Phenoxycarbonyl, Phenylthio, Phenylamino, Phenylhydrazino, Phenylmethyl, Phenylethyl, Phenylmethoxy, Phenylethoxy, Phenylmethylthio, Phenylethylthio, Phenylmethylamino oder Phenylethylamino, oder für jeweils gegebenenfalls durch Nitro, Cyano, Fluor, Chlor, Brom, Methyl, Ethyl, n- oder i-Propyl, n-, i-, s- oder t-Butyl, Difluormethyl, Trifluormethyl, Dichlormethyl, Trichlormethyl, Chlordifluormethyl, Fluordichlormethyl, Methoxy, Ethoxy, n- oder i-Propoxy, Difluormethoxy, Trifluormethoxy, Methylthio, Ethylthio, n- oder i-Propylthio, Difluormethylthio, Trifluormethylthio, Methoxycarbonyl, Ethoxycarbonyl, n- oder i-Propoxycarbonyl substituiertes monocyClisches oder bicyClisches HeterocyClyl, HeterocyClyloxy, HeterocyClylamino, die Gruppierung -N=(HeterocyClyl), HeterocyClylalkyl, HeterocyClylalkoxy oder HeterocyClylalkylamino aus der Reihe Furyl, Tetrahydrofuryl, Furyloxy, Tetrahydrofuryloxy, Furylamino, Tetrahydrofurylamino, Furylmethyl, Tetrahydrofurylmethyl, Furylmethoxy, Tetrahydrofurylmethoxy, Furylmethylamino, Tetrahydrofurylmethylamino, Dioxolanyl, Dioxolanylmethyl, Dioxolanylmethoxy, Dioxolanylmethylamino, Thienyl, Thienylmethyl, Dithiolanyl, Dithiolanylmethyl, Dithiolanylmethoxy, Dithiolanylmethylamino, Pyrrolidinyl, Pyrrolidinylamino, Oxopyrrolidinyl, Pyrrolyl, Indolyl, Pyrrolylmethyl, Pyrazolyl, Pyrazolyloxy, Pyrazolylamino, Pyrazolylmethyl, Imidazolyl, Imidazolinyl, Imidazolylmethyl, Imidazolinylmethyl, Oxoimidazolinyl, 2-Oxo-1,3-diaza-cyClopentyl, Oxazolyl, Oxazolylmethyl, Dihydrooxazolyl (Oxazolinyl), Tetrahydrooxazolyl (Oxazolidinyl), Isoxazolyl, Dihydroisoxazolyl (Isoxazolinyl), Tetrahydroi-(2H)-1,2-oxazin-z-yl, Tetrahydroisoxazolyl (Isoxazolidinyl), Thiazolyl, Thiazolylmethyl, Dihydrothiazolyl (Thiazolinyl), Tetrahydrothiazolyl (Thiazolidinyl), Thiazolimino, Oxothiazolidinyl, Cyanoiminothiazolidinyl, Oxadiazolylamino, Thiadiazolylamino, Oxotriazolinyl, Oxatetrazolinylmethyl, Tetrazolylmethyl, Oxotetrazolinyl, Oxotetrazolinylmethyl, Tetrazolylmethyl, Dioxanyl, Dioxanylmethyl, Dioxanylmethoxy, Dioxanylmethylamino, Dithianyl, Dithianylmethyl, Dithianylmethoxy, Dithianylmethylamino, Triazolylamino, Piperidinyl, Piperidinylamino, 2-(1H)-Pyridinimino, Oxopiperidinyl, 2-Oxo-1,3-diaza-cyClohexyl, 2-Oxo-1-azacyCloheptyl, 2-Oxo-1,3-diaza-cyCloheptyl, Morpholinyl, Morpholinylamino, Piperazinyl, Pyridinyl, Pyridinyloxy, Pyridinylamino, Pyridinylmethyl, Pyridinylmethoxy, Pyrimidinyl, Pyrimidinyloxy, Pyrimidinylmethyl oder Pyrimidinylmethoxy steht.

3. Verbindungen der Formel (I) gemäß Anspruch 1 oder 2, **dadurch gekennzeichnet, dass**
R¹ und R² für Wasserstoff stehen,
R³ für Wasserstoff, Fluor, Chlor, Methyl, Ethyl, Trifluormethyl, Trichlormethyl, Methoxy, Ethoxy, Methylsulfonyl oder Ethylsulfonyl steht,
R⁴ für Wasserstoff, Fluor, Chlor, Methyl, Ethyl, Trifluormethyl, Trichlormethyl, Methoxy, Ethoxy, Methylsulfonyl oder Ethylsulfonyl steht,
R⁵ für Wasserstoff steht, und
Y für Hydroxyl steht.

4. Verbindungen der Formel (I-2) 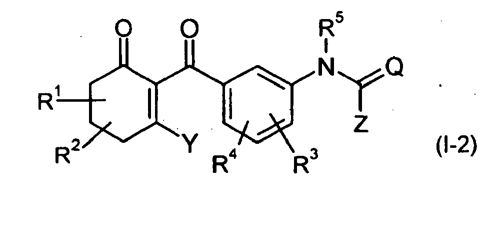 in welcher
Q, R¹, R², R³, R⁴, R⁵ und Z eine der in einem der Ansprüche 1 bis 3 angegebenen Bedeutungen haben.

5. Verfahren zum Herstellen von Verbindungen der Formel (I) gemäß einem der Ansprüche 1 bis 3 und der Formel (1-2) gemäß Anspruch 4, **dadurch gekennzeichnet, dass** man CyClohexandione der allgemeinen Formel 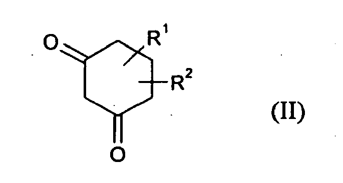 in welcher
R¹ und R² die in einem der Ansprüche 1 bis 3 angegebene Bedeutung haben,
mit substituierten Benzoesäuren der allgemeinen Formel (III), 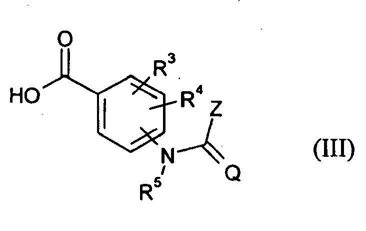 in welcher
Q, R³, R⁴, R⁵ und Z die in einem der Ansprüche 1 bis 3 angegebene Be- deutung haben,
oder mit reaktionsfähigen Derivaten hiervon, gegebenenfalls in Gegenwart eines Dehydratisierungsmittels, gegebenenfalls in Gegenwart eines oder mehrerer Reaktionshilfsmittel und gegebenenfalls in Gegenwart eines oder mehrerer Verdünnungsmittel umsetzt,
und gegebenenfalls im Anschluß daran an den so erhaltenen Verbindungen der Formel (I) im Rahmen der Substituentendefinition auf übliche Weise elektrophile oder nuCleophile Substitutionsreaktionen bzw. Oxidations- oder Reduktionsreaktionen durchführt oder die Verbindungen der Formel (I) auf übliche Weise in Salze überführt.

6. Herbizide Mittel, **gekennzeichnet durch** den Gehalt mindestens einer Verbindung der Formel (I) gemäß einem der Ansprüche 1 bis 3 und üblichen Streckmitteln.

7. Verwendung von mindestens einer Verbindung gemäß einem der Ansprüche 1 bis 3 oder eines Mittels gemäß Anspruch 6 zur Bekämpfung von unerwünschten Pflanzen.

8. Verfahren zum Bekämpfen von unerwünschten Pflanzen, **dadurch gekennzeichnet, dass** man mindestens eine Verbindung gemäß einem der Ansprüche 1 bis 3 oder ein Mittel gemäß Anspruch 6 auf die unerwünschten Pflanzen und/oder ihren Lebensraum einwirken lässt.

## Claims

1. Compounds of the formula (I) 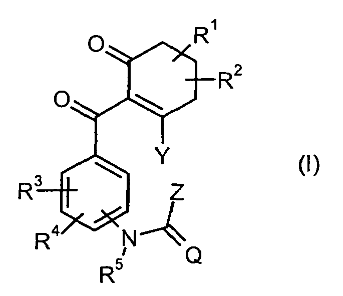 in which
Q represents O (oxygen) or S (sulphur),
R¹ represents hydrogen, fluorine, chlorine or bromine, represents in each case optionally fluorine-, chlorine-, methoxy-, ethoxy-, n- or i- propoxy-, methylthio-, ethylthio-, n- or i-propylthio-, methylsulph- inyl-, ethylsulphinyl-, methylsulphonyl- or ethylsulphonyl- substituted methyl, ethyl, n- or i-propyl, n-, i-, s- or t-butyl, methylthio, ethylthio, n- or i-propylthio, n-, i-, s- or t-butylthio, or represents phenyl,
R² represents hydrogen, fluorine, chlorine or bromine, represents in each case optionally fluorine- or chlorine-substituted methyl, ethyl, n- or i-propyl, n-, i-, s- or t-butyl, or optionally together with R¹ also represents O (oxygen), ethane-1,2-diyl, propane-1,3-diyl or butane- 1,4-diyl,
R³ represents hydrogen, nitro, cyano, carboxyl, carbamoyl, thiocarbamoyl, fluorine, chlorine, bromine, iodine, represents in each case optionally fluorine-, chlorine-, methoxy-, ethoxy-, methylthio-, ethylthio-, methylsulphinyl-, ethylsulphinyl-, methylsulphonyl- or ethylsulphonyl-substituted methyl, ethyl, n- or i-propyl, n-, i-, s- or t-butyl, methoxy, ethoxy, n- or i-propoxy, n-, i-, s- or t-butoxy, methylthio, ethylthio, n- or i-propylthio, n-, i-, s- or t- butylthio, methylsulphinyl, ethylsulphinyl, n- or i-propylsulphinyl, methylsulphonyl, ethylsulphonyl, n- or i-propylsulphonyl, represents methylamino, ethylamino, n- or i-propylamino, n-, i-, s- or t- butylamino, dimethylamino, diethylamino, dimethylaminocarbonyl, diethylaminocarbonyl, dimethylaminosulphonyl or diethylaminosulphonyl,
R⁴ represents hydrogen, nitro, cyano, carboxyl, carbamoyl, thiocarbamoyl, fluorine, chlorine, bromine, iodine, represents in each case optionally fluorine-, chlorine-, methoxy-, ethoxy-, methylthio-, ethylthio-, methylsulphinyl-, ethylsulphinyl-, methylsulphonyl- or ethylsulphonyl-substituted methyl, ethyl, n- or i-propyl, n-, i-, s- or t-butyl, methoxy, ethoxy, n- or i-propoxy, n-, i-, s- or t-butoxy, methylthio, ethylthio, n- or i-propylthio, n-, i-, s- or t- butylthio, methylsulphinyl, ethylsulphinyl, n- or i-propylsulphinyl, methylsulphonyl, ethylsulphonyl, n- or i-propylsulphonyl, represents methylamino, ethylamino, n- or i-propylamino, n-, i-, s- or t- butylamino, dimethylamino, diethylamino, dimethylaminocarbonyl, diethylaminocarbonyl, dimethylaminosulphonyl or diethylaminosulphonyl,
R⁵ represents hydrogen, represents in each case optionally cyano-, fluorine-, chlorine-, bromine-, methoxy-, ethoxy-, n- or i-propoxy- substituted methyl, ethyl, n- or i-propyl, n-, i-, s- or t-butyl, n-, i-, s- or t-pentyl, methoxy, ethoxy, n- or i-propoxy, n-, i-, s- or t-butoxy, methylthio, ethylthio, n- or i-propylthio, n-, i-, s- or t-butylthio, methylsulphinyl, ethylsulphinyl, n- or i-propylsulphinyl, methyl- sulphonyl, ethylsulphonyl, methylamino, ethylamino, n- or i-propyl- amino, n-, i-, s- or t-butylamino, represents dimethylamino or diethylamino, represents in each case optionally fluorine-, chlorine- and/or bromine-substituted ethenyl, propenyl, butenyl, pentenyl, ethinyl, propinyl, butinyl or pentinyl, represents in each case optionally cyano-, fluorine-, chlorine-, bromine-, methyl- or ethyl- substituted cyClopropyl, cyClobutyl, cyClopentyl, cyClohexyl, cyClopropylmethyl, cyClobutylmethyl, cyClopentylmethyl or cyClohexylmethyl, or represents in each case optionally nitro-, cyano-, fluorine-, chlorine-, bromine-, methyl-, ethyl-, n- or i-propyl- , n-, i-, s- or t-butyl-, trifluoromethyl-, methoxy-, ethoxy-, n- or i- propoxy-, difluoromethoxy- or trifluoromethoxy-substituted phenyl, phenylthio, phenylsulphinyl, phenylsulphonyl, naphthyl, phenylmethyl, phenylethyl, naphthylmethyl or naphthylethyl or represents the grouping -C(Q)-Z,
Y represents hydroxyl, formyloxy, fluorine, chlorine or bromine, represents in each case optionally cyano-, fluorine-, chlorine-, methoxy-, ethoxy-, n- or i-propoxy-, methylthio-, ethylthio-, n- or i- propylthio-, methylsulphinyl-, ethylsulphinyl-, methylsulphonyl- or ethylsulphonyl-substituted methoxy, ethoxy, n- or i-propoxy, methylthio, ethylthio, n- or i-propylthio, methylsulphinyl, ethylsulphinyl, methylsulphonyl, ethylsulphonyl, acetyloxy, propionyloxy, n- or i-butyroyloxy, methoxycarbonyloxy, ethoxycarbonyloxy, n- or i-propoxycarbonyloxy, methylaminocarbonyloxy, ethylaminocarbonyloxy, n- or i- propylaminocarbonyloxy, methylsulphonyloxy, ethylsulphonyloxy, n- or i-propylsulphonyloxy, represents in each case optionally fluorine-, chlorine- and/or bromine-substituted propenyloxy, butenyloxy, propinyloxy or butinyloxy, represents in each case optionally nitro-, cyano-, fluorine-, chlorine-, bromine-, methyl-, ethyl-, n- or i-propyl-, n-, i-, s- or t-butyl-, trifluoromethyl-, methoxy-, ethoxy-, n- or i-propoxy-, difluoromethoxy-, trifluoromethoxy-, methylthio-, ethylthio-, n- or i-propylthio-, difluoromethylthio-, trifluoromethylthio-, methylsulphinyl-, ethyl- sulphinyl-, trifluoromethylsulphinyl-, methylsulphonyl-, ethylsulphonyl- or trifluoromethylsulphonyl-substituted phenyloxy, phenylthio, phenylsulphinyl, phenylsulphonyl, phenylcarbonyloxy, phenylcarbonylmethoxy, phenylsulphonyloxy, phenylmethoxy, phenylmethylthio, phenylmethylsulphinyl or phenylmethylsulphonyl, and
Z represents hydrogen, amino, cyanoamino, nitroamino, hydroxyamino, hydrazino,
represents cyano-, fluorine-, chlorine-, methoxy-, ethoxy-, n- or i- propoxy-, methylthio-, ethylthio-, n- or i-propylthio-, methyl- sulphinyl- or ethylsulphonyl-substituted methyl,
represents in each case optionally fluorine-, chlorine- and/or bromine-substituted ethenyl, propenyl, butenyl, pentenyl, ethinyl, propinyl, butinyl, pentinyl, propenyloxy, butenyloxy, pentenyloxy, propenylthio, butenylthio, pentenylthio, propenylamino, butenylamino, pentenylaminb, propenyloxyamino, butenyloxyamino, pentenyloxyamino, ethinyl, propinyl, butinyl, pentinyl, propinyloxy, butinyloxy, pentinyloxy, propinylamino, butinylamino or pentinylamino,
represents in each case optionally cyano-, fluorine-, chlorine-, bromine-, methyl- or ethyl-substituted cyClopropyl, cyClobutyl, cyClopentyl, cyClohexyl, cyClopropyloxy, cyClobutyloxy, cyClopentyloxy, cyClohexyloxy, cyClopropylamino, cyClobutylamino, cyClopentylamino, cyClohexylamino, cyClopropylhydrazino, cyClobutylhydrazino, cyClopentylhydrazino, cyClohexylhydrazino, cyClopropylmethyl, cyClobutylmethyl, cyClopentylmethyl, cyClohexylmethyl, cyClopropylmethoxy, cyClobutylmethoxy, cyClopentylmethoxy, cyClohexylmethoxy, cyClopropylmethylamino, cyClobutylmethylamino, cyClopentylmethylamino or cyClohexylmethylamino,
represents in each case optionally nitro-, cyano-, fluorine-, chlorine-, bromine-, methyl-, ethyl-, n- or i-propyl-, n-, i-, s- or t-butyl-, trifluoromethyl-, methoxy-, ethoxy-, n- or i-propoxy-, difluoromethoxy-, trifluoromethoxy-, methoxycarbonyl-, ethoxycarbonyl-, n- or i-propoxycarbonyl-substituted phenyl, phenylcarbonyl, phenoxy, phenoxycarbonyl, phenylthio, phenylamino, phenylhydrazino, naphthyl, naphthyloxy, naphtylthio, naphthylamino, phenylmethyl, phenylethyl, phenylmethoxy, phenylethoxy, phenylmethylthio, phenylethylthio, phenylmethylamino, phenylethylamino, naphthylmethyl, naphthylethyl, naphthylmethoxy, naphthylethoxy, naphthylinethylamino or naphthylethylamino, or represents in each case optionally nitro-, cyano-, fluorine-, chlorine-, bromine-, methyl-, ethyl-, n- or i-propyl-, n-, i-, s- or t-butyl-, difluoromethyl-, trifluoromethyl-, dichloromethyl-, trichloromethyl-, chlorodifluoromethyl-, fluorodichloromethyl-, methoxy-, ethoxy-, n- or i-propoxy-, n-, i-, s- or t-butoxy-, difluoromethoxy-, trifluoro- methoxy-, methylthio-, ethylthio-, n- or i-propylthio-, n-, i-, s- or t- butylthio-, difluoromethylthio-, trifluoromethylthio-, methoxy- carbonyl-, ethoxycarbonyl-, n- or i-propoxycarbonyl-substituted monocyClic or bicyClic heterocyClyl, heterocyClyloxy, heterocyClyl- amino, the grouping -N=(heterocyClyl), heterocyClylalkyl, hetero- cyClylalkoxy or heterocyClylalkylamino from the group consisting of furyl, tetrahydrofuryl, furyloxy, tetrahydrofuryloxy, furylamino, tetrahydrofurylamino, furylmethyl, tetrahydrofurylmethyl, furylmethoxy, tetrahydrofurylmethoxy, furylmethylamino, tetrahydrofurylmethylamino, dioxolanyl, dioxolanylmethyl, dioxolanylmethoxy, dioxolanylmethylamino, thienyl, thienylamino, thienylmethyl, thenylmethylamino, dithiolanyl, dithiolanylmethyl, dithiolanylmethoxy, dithiolanylmethylamino, pyrrolidinyl, pyrrolidinylamino, oxopyrrolidinyl, pyrrolyl, indolyl, pyrrolylmethyl, pyrazolyl, pyrazolyloxy, pyrazolylamino, pyrazolylmethyl, imidazolyl, imidazolinyl, imidazolylmethyl, imidazolinylmethyl, oxoimidazolinyl, 2-oxo-1,3-diaza-cyClopentyl, oxazolyl, oxazolylamino, dihydrooxazolyl (oxazolinyl), tetrahydrooxazolyl, (oxazolidinyl), isoxazolyl, isoxazolylamino, dihydroisoxazolyl (isoxazolinyl), tetrahydroisoxazolyl (isoxazolidinyl), tetrahydro-(2H)-1,2-oxazin-2-yl, oxazolylmethyl, thiazolyl, thiazolylamino, thiazolylmethyl, dihydrothiazolyl (thiazolinyl), tetrahydrothiazolyl (thiazolidinyl), thiazolimino, thiazolinylthio, thiazolinylamino, oxothiazolidinyl, cyanoiminothiazolidinyl, oxadiazolylamino, thiadiazolylamino, triazolylamino, oxotriazolinyl, thioxotriazolinyl, oxotetrazolinyl, oxotetrazolinylinethyl, tetrazolylmethyl, dioxanyl, dioxanylmethyl, dioxanylmethoxy, dioxanylmethylamino, dithianyl, dithianylmethyl, dithianylmethoxy, dithianylmethylamino, piperidinyl, piperidinylamino, oxopiperidinyl, 2-oxo-1,3-diaza-cyClohexyl, 2- oxo-1-aza-cyCloheptyl, 2-oxo-1,3-diaza-cyCloheptyl, morpholinyl, morpholinylamino, piperazinyl, pyridinyl, pyridinyloxy, pyridinylthio, pyridinylamino, 2-(1H)-pyridinimino, pyridinylmethyl, pyridinylmethoxy, pyrimidinyl, pyrimidinyloxy, pyrimidinylthio, pyrimidinylamino, pyrimidinylmethyl or pyrimidinylmethoxy,
inCluding all possible tautomeric forms of the compounds of the general formula (I) and the possible salts of the compounds of the general formula (I).

2. Compounds of the formula (I) according to Claim 1, **characterized in that**
R¹ represents hydrogen, represents in each case optionally fluorine- or chlorine-substituted methyl, ethyl, n- or i-propyl, methylthio, ethyl- thio, n- or i-propylthio, or represents phenyl,
R² represents hydrogen, represents in each case optionally fluorine- or chlorine-substituted methyl, ethyl, n- or i-propyl, or together with R¹ optionally also represents ethane-1,2-diyl, propane-1,3-diyl or butane-1,4-diyl,
R³ represents hydrogen, nitro, cyano, fluorine, chlorine, bromine, iodine, methyl, ethyl, n- or i-propyl, difluoromethyl, trifluoromethyl, dichloromethyl, trichloromethyl, methoxymethyl, methylthiomethyl, methylsulphinylmethyl, methylsulphonylmethyl, methoxy, ethoxy, difluoromethoxy, trifluoromethoxy, methylthio, ethylthio, methylsulphinyl, ethylsulphinyl, methylsulphonyl, ethylsulphonyl or dimethylaminosulphonyl,
R⁴ represents hydrogen, nitro, cyano, fluorine, chlorine, bromine, iodine, methyl, ethyl, n- or i-propyl, difluoromethyl, trifluoromethyl, dichloromethyl, trichloromethyl, methoxymethyl, methylthiomethyl, methylsulphinylmethyl, methylsulphonylmethyl, methoxy, ethoxy, difluoromethoxy, trifluoromethoxy, methylthio, ethylthio, methyl- sulphinyl, ethylsulphinyl, methylsulphonyl, ethylsulphonyl or dimethylaminosulphonyl,
R⁵ represents hydrogen, represents in each case optionally cyano-, fluorine-, chlorine-, methoxy- or ethoxy-substituted methyl, ethyl, n- or i-propyl, n- or i-butyl, represents methoxy, ethoxy, n- or i- propoxy, methylthio, ethylthio, n- or i-propylthio, methylsulphinyl, ethylsulphinyl, n- or i-propylsulphinyl, methylsulphonyl, ethylsulphonyl, methylamino, ethylamino, n- or i-propylamino or dimethylamino, represents in each case optionally fluorine- and/or chlorine-substituted propenyl, butenyl, propinyl or butinyl, represents in each case optionally cyano-, fluorine-, chlorine- or methyl-substituted cyClopropyl, cyClobutyl, cyClopentyl, cyClohexyl, cyClopropylmethyl, cyClobutylmethyl, cyClopentylmethyl or cyClohexylmethyl, or represents in each case optionally nitro-, cyano-, fluorine-, chlorine-, bromine-, methyl-, ethyl-, n- or i-propyl- , n-, i-, s- or t-butyl-, trifluoromethyl-, methoxy-, ethoxy-, n- or i- propoxy-, difluoromethoxy- or trifluoromethoxy-substituted phenyl, phenylthio, phenylsulphinyl, phenylsulphonyl, phenylmethyl or phenylethyl, or represents the grouping -C(Q)-Z,
Y represents hydroxyl, represents in each case optionally fluorine- and/or chlorine-substituted methoxy, ethoxy, n- or i-propoxy, methylthio, ethylthio, n- or i-propylthio, methylsulphinyl, ethylsulphinyl, methylsulphonyl, ethylsulphonyl, acetyloxy, propionyloxy, n- or i-butyroyloxy, methoxycarbonyloxy, ethoxycarbonyloxy, n- or i-propoxycarbonyloxy, methylaminocarbonyloxy, ethylaminocarbonyloxy, n- or i- propylaminocarbonyloxy, methylsulphonyloxy, ethylsulphonyloxy, n- or i-propylsulphonyloxy, represents in each case optionally fluorine-, chlorine- and/or bromine-substituted propenyloxy, butenyloxy, propinyloxy or butinyloxy, represents in each case optionally nitro-, cyano-, fluorine-, chlorine-, bromine-, methyl-, ethyl-, n- or i-propyl-, n-, i-, s- or t-butyl-, trifluoromethyl-, methoxy-, ethoxy-, n- or i-propoxy-, difluoromethoxy-, trifluoromethoxy-, methylthio-, ethylthio-, n- or i-propylthio-, difluoromethylthio-, trifluoromethylthio-, methylsulphinyl-, ethylsulphinyl-, trifluoromethylsulphinyl-, methylsulphonyl-, ethylsulphonyl- or trifluoromethylsulphonyl-substituted phenyloxy, phenylthio, phenylsulphinyl, phenylsulphonyl, phenylcarbonyloxy, phenylcarbonylmethoxy, phenylsulphonyloxy, phenylmethoxy, phenylmethylthio, phenylmethylsulphinyl or phenylmethyl- sulphonyl, and
Z represents amino, cyanoamino, hydrazino,
represents cyano-, fluorine-, chlorine-, methoxy-, ethoxy-, n- or i- propoxy-, methylthio-, ethylthio-, n- or i-propylthio-, methylsulphinyl-, ethylsulphinyl-, n- or i-propylsulphinyl-, methylsulphonyl- or ethylsulphonyl-substituted methyl, represents in each case optionally fluorine- and/or chlorine- substituted ethenyl, propenyl, butenyl, ethinyl, propinyl, butinyl, propenyloxy, butenyloxy, propenylthio, butenylthio, propenylamino, butenylamino, propenyloxyamino, butenyloxyamino, ethinyl, propinyl, butinyl, propinyloxy, butinyloxy, propinylamino or butinylamino,
represents in each case optionally cyano-, fluorine-, chlorine- or methyl-substituted cyClopropyl, cyClobutyl, cyClopentyl, cyClohexyl, cyClopropyloxy, cyClobutyloxy, cyClopentyloxy, cyClohexyloxy, cyClopropylamino, cyClobutylamino, cyClopentylamino, cyClohexyl- amino, cyClopentylhydrazino, cyClohexylhydrazino, cyClopropyl- methyl, cyClobutylmethyl, cyClopentylmethyl, cyClohexylmethyl, cyClopropylmethoxy, cyClobutylmethoxy, cyClopentylmethoxy, cyClohexylmethoxy, cyClopropylmethylamino, cyClobuylmethylamino, cyClopentylmethylamino or cyClohexylmethylamino,
represents in each case optionally nitro-, cyano-, fluorine-, chlorine-, bromine-, methyl-, ethyl-, n- or i-propyl-, n-, i-, s- or t-butyl-, trifluoromethyl-, methoxy-, ethoxy-, n- or i-propoxy-, difluoro- methoxy-, trifluoromethoxy-, methoxycarbonyl-, ethoxycarbonyl-, n- or i-propoxycarbonyl-substituted phenyl, phenylcarbonyl, phenoxy, phenoxycarbonyl, phenylthio, phenylamino, phenylhydrazino, phenylmethyl, phenylethyl, phenylmethoxy, phenylethoxy, phenylmethylthio, phenylethylthio, phenylmethylamino or phenylethylamino, or represents in each case optionally nitro-, cyano-, fluorine-, chlorine-, bromine-, methyl-, ethyl-, n- or i-propyl-, n-, i-, s- or t-butyl-, difluoromethyl-, trifluoromethyl-, dichloromethyl-, trichloromethyl-, chlorodifluoromethyl-, fluorodichloromethyl-, methoxy-, ethoxy-, n- or i-propoxy-, difluoromethoxy-, trifluoromethoxy-, methylthio-, ethylthio-, n- or i-propylthio-, difluoromethylthio-, trifluoromethylthio-, methoxycarbonyl-, ethoxycarbonyl-, n- or i- propoxycarbonyl-substituted monocyClic or bicyClic heterocyClyl, heterocyClyloxy, heterocyClylamino, the grouping -N=(heterocyClyl), heterocyClylalkyl, heterocyClylalkoxy or heterocyClylalkylamino from the group consisting of furyl, tetrahydrofuryl, furyloxy, tetrahydrofuryloxy, furylamino, tetrahydrofurylamino, furylmethyl, tetrahydrofurylmethyl, furylmethoxy, tetrahydrofurylmethoxy, furylmethylamino, tetrahydrofurylmethylamino, dioxolanyl, dioxolanylmethyl, dioxolanylmethoxy, dioxolanylmethylamino, thienyl, thienylmethyl, dithiolanyl, dithiolanylmethyl, dithiolanylmethoxy, dithiolanylmethylamino, pyrrolidinyl, pyrroli- dinylamino, oxopyrrolidinyl, pyrrolyl, indolyl, pyrrolylmethyl, pyrazolyl, pyrazolyloxy, pyrazolylamino, pyrazolylmethyl, imidazolyl, imidazolinyl, imidazolylmethyl, imidazolinylmethyl, oxoimidazolinyl, 2-oxo-1,3-diaza-cyClopentyl, oxazolyl, oxazolylmethyl, dihydrooxazolyl (oxazolinyl), tetrahydrooxazolyl (oxazolidinyl), isoxazolyl, dihydroisoxazolyl (isoxazolinyl), tetrahydro-(2H)-1,2-oxazin-z-yl, tetrahydroisoxazolyl (isoxazolidinyl), thiazolyl, thiazolylmethyl, dihydrothiazolyl (thiazolinyl), tetrahydrothiazolyl (thiazolidinyl), thiazolimino, oxothiazolidinyl, cyanoiminothiazolidinyl, oxadiazolylamino, thiadiazolylamino, oxotriazolinyl, oxatetrazolinylmethyl, tetrazolylmethyl, oxotetrazolinyl, oxotetrazolinylmethyl, tetrazolylmethyl, dioxanyl, dioxanylmethyl, dioxanylmethoxy, dioxanylmethylamino, dithianyl, dithianylmethyl, dithianylmethoxy, dithianylmethylamino, triazolylamino, piperidinyl, piperidinylamino, 2-(1H)-pyridinimino, oxopiperidinyl, 2-oxo-1,3- diaza-cyClohexyl, 2-oxo-1-aza-cyCloheptyl, 2-oxo-1,3-diaza- cyCloheptyl, morpholinyl, morpholinylamino, piperazinyl, pyridinyl, pyridinyloxy, pyridinylamino, pyridinylmethyl, pyridinylmethoxy, pyrimidinyl, pyrimidinyloxy, pyrimidinylmethyl or pyrimidinylmethoxy.

3. Compounds of the formula (I) according to Claim 1 or 2, **characterized in that**
R¹ and R² represent hydrogen,
R³ represents hydrogen, fluorine, chlorine, methyl, ethyl, trifluoromethyl, trichloromethyl, methoxy, ethoxy, methylsulphonyl or ethylsulphonyl,
R⁴ represents hydrogen, fluorine, chlorine, methyl, ethyl, trifluoromethyl, trichloromethyl, methoxy, ethoxy, methylsulphonyl or ethylsulphonyl,
R⁵ represents hydrogen, and
Y represents hydroxyl.

4. Compounds of the formula (I-2) 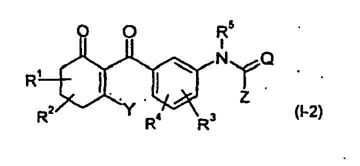 in which
Q, R¹, R², R³, R⁴, R⁵ and Z have one of the meanings given in any of Claims 1 to 3.

5. Process for preparing compounds of the formula (I) according to any of Claims 1 to 3 and of the formula (I-2) according to Claim 4, **characterized in that** cyClohexanediones of the general formula 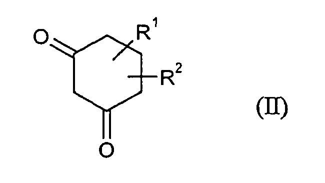 in which
R¹ and R² have the meanings given in any of Claims 1 to 3,
are reacted with substituted benzoic acids of the general formula (III) 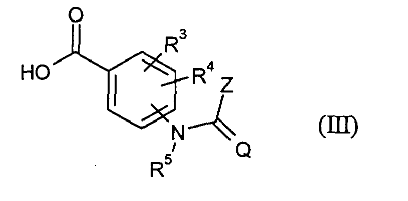 in which
Q, R³, R⁴, R⁵ and Z have the meanings given in any of Claims 1 to 3,
or with reactive derivatives thereof,
if appropriate in the presence of a dehydrating agent, if appropriate in the presence of one or more reaction auxiliaries and if appropriate in the presence of one or more diluents,
and, if appropriate, electrophilic or nuCleophilic substitutions and/or oxidations or reductions within the scope of the definition of the substituents are subsequently carried out in a customary manner on the resulting compounds of the formula (I), or the compounds of the formula (I) are converted in a customary manner into salts.

6. Herbicidal compositions, **characterized in that** they comprise at least one compound of the formula (I) according to any of Claims 1 to 3 and the customary extenders.

7. Use of at least one compound according to any of Claims 1 to 3 or of a composition according to Claim 6 for controlling undesirable plants.

8. Method for controlling undesirable plants, **characterized in that** at least one compound according to any of Claims 1 to 3 or a composition according to Claim 6 is allowed to act on the undesirable plants and/or their habitat.

## Revendications

1. Composés de la formule (I) 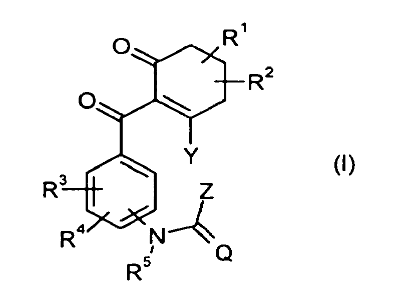 dans laquelle
Q représente 0 (oxygène) ou S (soufre),
R¹ représente l'hydrogène, le fluor, le chlore ou le brome ; un groupe méthyle, éthyle, n- ou i-propyle, n-, i-, s- ou t-butyle, méthylthio, éthylthio, n- ou i-propylthio, n-, i-, s- ou t-butylthio à chaque fois éventuellement substitué par du fluor, du chlore, un groupe méthoxy, éthoxy, n- ou i-propoxy, méthylthio, éthylthio, n- ou i-propylthio, méthylsulfinyle, éthylsulfinyle, méthylsulfonyle ou éthylsulfonyle ; ou le groupe phényle.
R² représente l'hydrogène, le fluor, le chlore ou le brome ; un groupe méthyle, éthyle, n- ou i-propyle, n-, i-, s- ou t-butyle à chaque fois éventuellement substitué par du fluor ou du chlore ; ou représente éventuellement également avec R¹ 0 (oxygène), un groupe éthane 1,2-diyle, propane-1,3-diyle ou butane-1,4-diyle,
R³ représente l'hydrogène, un groupe nitro, cyano, carboxy, carbamoyle, thiocarbamoyle, le fluor, le chlore, le brome, l'iode ; un groupe méthyle, éthyle, n- ou i-propyle, n-, i-, s- ou t-butyle, méthoxy, éthoxy, n-, ou i-propoxy, n-, i-, s-, ou t-butoxy, méthylthio, éthylthio, n- ou i-propylthio, n-, i-, s- ou t-butylthio, méthylsulfinyle, éthylsulfinyle, n- ou i-propylsuifinyle, méthylsulfonyle, éthylsulfonyle, n- ou i-propylsulfonyle à chaque fois éventuellement substitué par le fluor, le chlore, un groupe méthoxy, éthoxy, méthythio, éthylthio, méthylsulfinyle, éthylsulfinyle, méthylsulfonyle ou éthylsulfonyle ; un groupe méthylamino, éthylamino, n- ou i-propylamino, n-, i-, s- ou t-butylamino, diméthylamino, diéthylamino, diméthylaminocarbonyle, diéthylaminocarbonyle, diméthylaminosulfonyle ou diéthylaminosulfonyle,
R⁴ représente l'hydrogène, un groupe nitro, cyano, carboxy, carbamoyle, thiocarbamoyle, le fluor, le chlore, le brome, l'iode ; un groupe méthyle, éthyle, n- ou i-propyle, n-, i-, s- ou t-butyle, méthoxy, éthoxy, n- ou i-propoxy, n-, i-, s-, ou t-butoxy, méthylthio, éthylthio, n- ou i-propylthio, n-, i-, s- ou t-butylthio, méthylsulfinyle, éthylsulfinyle, n- ou i-propylsulfinyle, méthylsulfonyle, éthylsulfonyle, n- ou i-propylsulfonyle à chaque fois éventuellement substitué par du fluor, du chlore, un groupe méthoxy, éthoxy, méthythio, éthylthio, méthylsulfinyle, éthylsulfinyle, méthylsulfonyle ou éthylsulfonyle ; un groupe méthylamino, éthylamino, n- ou i-propylamino, n-, i-, s- ou t-butylamino, diméthylamino, diéthylamino, diméthylaminocarbonyle, diéthylaminocarbonyle, diméthylaminosulfonyle ou diéthylaminosulfonyle,
R⁵ représente l'hydrogène ; un groupe méthyle, éthyle, n- ou i-propyle, n-, i-, s- ou t-butyle, , n-, i-, s-, ou t-pentyle, méthoxy, éthoxy, n- ou i-propxy, n-, i-, s-, ou t-butoxy, méthylthio, éthylthio, n- ou i-propylthio, n-, i-, s-, ou t-butylthio, méthylsulfinyle, éthylsulfinyle, n- ou i-propylsulfinyle, méthylsulfonyle, éthylsulfonyle, méthylamino, éthylamino, n- ou i-propylamino, n-, i-, s- ou t-butylamino à chaque fois éventuellement substitué par un groupe cyano, du fluor, du chlore, du brome, un groupe méthoxy, éthoxy, n- ou i-propoxy ; un groupe diméthylamino ou diéthylamino ; un groupe éthényle, propényle, butényle, pentényle, éthinyle, propinyle, butinyle ou pentinyle à chaque fois éventuellement substitué par du fluor, du chlore et/ou du brome ; un groupe cyClopropyle, cyClobutyle, cyClopentyle, cyClohexyle, cyClopropylméthyle, cyClobutylméthyle, cyClopentylméthyle ou cyClohexylméthyle à chaque fois éventuellement substitué par un groupe cyano, du fluor, du chlore, du brome, un groupe méthyle ou éthyle ; ou un groupe phényle, phénylthio, phénylsulfinyle, phénylsulfonyle, naphtyle, phénylméthyle, phényléthyle, naphtylméthyle ou naphtyléthyle à chaque fois éventuellement substitué par un groupe nitro, cyano, du fluor, du chlore, du brome, un groupe méthyle, éthyle, n- ou i-propyle, n-, i-, s- ou t-butyle, trifluorométhyle, méthoxy, éthoxy, n- ou i-prpoxy, difluorométhoxy ou trifluorométhoxy ; ou un groupement -C(Q)-Z,
Y représente un groupe hydroxy, formyloxy, le fluor, le chlore ou le brome ; un groupe méthoxy, éthoxy, n- ou i-propxy, méthylthio, éthylthio, n- ou i-propylthio, méthylsulfinyle, éthylsulfinyle, méthylsulfonyle, éthylsulfonyle, acétyloxy, propionyloxy, n- ou i-butyroyloxy, méthoxycarbonyloxy, éthoxycarbonyloxy, n- ou i-propoxycarbonyloxy, méthylaminocarbonyloxy, éthylaminocarbonyloxy, n- ou i-propylaminocarbonyloxy, méthylsulfonyloxy, éthylsulfonyloxy, n- ou i-propylsulfonyloxy à chaque fois éventuellement substitué par un groupe cyano, du fluor, du chlore, un groupe méthoxy, éthoxy, n- ou i-propoxy, méthylthio, éthylthio, n- ou i-propylthio, méthylsulfinyle, éthylsulfinyle, méthylsulfonyle ou éthylsulfonyle ; un groupe propényloxy, butényloxy, propinyloxy ou butinyloxy à chaque fois éventuellement substitué par du fluor, du chlore et/ou du brome ; un groupe phényloxy, phénylthio, phénylsulfinyle, phénylsulfonyle, phénylcarbonyloxy, phénylcarbonylméthoxy, phénylsulfonyloxy, phénylméthoxy, phénylméthylthio, phénylméthylsulfinyle ou phénylméthylsulfonyle à chaque fois éventuellement substitué par un groupe nitro, cyano, du fluor, du chlore, du brome, un groupe méthyle, éthyle, n- ou i-propyle, n-, i-, s- ou t-butyle, trifluorométhyle, méthoxy, éthoxy, n- ou i-propoxy, difluorométhoxy, trifluorométhoxy, méthylthio, éthylthio, n- ou i-propylthio, difluorométhylthio, trifluorométhylthio, méthylsulfinyle, éthylsulfinyle, trifluorométhylsulfinyle, méthylsulfonyle, éthylsulfonyle, ou trifluorométhylsulfonyle, et
Z représente l'hydrogène, un groupe amino, cyanoamino, nitroamino, hydroxyamino, hydrazino, un groupe méthyle substitué par un groupe cyano, du fluor, du chlore, un groupe méthoxy, éthoxy, n- ou i-propoxy, méthylthio, éthylthio, n- ou i-propylthio, méthylsulfinyle ou éthylsulfinyle,
un groupe éthényle, propényle, butényle, pentényle, éthinyle, propinyle, butinyle, pentinyle, propényloxy, butényloxy, pentényloxy, propénylthio, buténylthio, penténylthio, propénylamino, buténylamino, penténylamino, propényloxyamino, butényloxyamino, pentényloxyamino, éthinyle, propinyle, butinyle, pentinyle, propinyloxy, butinyloxy, pentinyloxy, propinylamino, butinylamino ou pentinylamino à chaque fois éventuellement substitué par du fluor, du chlore et/ou du brome,
un groupe cyClopropyle, cyClobutyle, cyClopentyle, cyClohexyle, cyClopropyloxy, cyClobutyloxy, cyClopentyloxy, cyClohexyloxy, cyClopropylamino, cyClobutylamino, cyClopentylamino, cyClohexylamino, cyClopropylhydrazino, cyClobutylhydrazino, cyClopentylhydrazino, cyClohexylhydrazino, cyClopropylméthyle, cyClobutylméthyle, cyClopentylméthyle, cyClohexyméthyle, cyClopropylméthoxy, cyClobutylméthoxy, cyClopentylméthoxy, cyClohexylméthoxy, cyClopropylméthylamino, cyClobutylméthylamino, cyClopentylméthylamino ou cyClohexylméthylamino à chaque fois éventuellement substitué par un groupe cyano, du fluor, du chlore, du brome, un groupe méthyle ou éthyle,
un groupe phényle, phénylcarbonyle, phénoxy, phénoxycarbonyle, phénylthio, phénylamino, phénylhydrazino, naphtyle, naphtyloxy, naphtylthio, naphthylamino, phénylméthyle, phényléthyle, phénylméthoxy, phényléthoxy, phénylméthylthio, phényléthylthio, phénylméthylamino, phényléthylamino, naphtylméthyle, naphtyléthyle, naphtylméthoxy, naphtyléthoxy, naphtylméthylamino ou naphtyléthylamino à chaque fois éventuellement substitué par un groupe nitro, cyano, du fluor, du chlore, du brome, un groupe méthyle, éthyle, n- ou i-propyle, n-, i-, s-, ou t-butyle, trifluorométhyle, méthoxy, éthoxy, n- ou i-propoxy, difluorométhoxy, trifluorométhoxy, méthoxycarbonyle, éthoxycarbonyle, n- ou i-propoxycarbonyle, ou
un groupe hétérocyClyle, hétérocyClyloxy, hétérocyClylamino, monocyClique ou bicyClique à chaque fois éventuellement substitué par un groupe nitro, cyano, du fluor, du chlore, du brome, un groupe méthyle, éthyle, n- ou i-propyle, n-, i-, s- ou t-butyle, difluorométhyle, trifluorométhyle, dichlorométhyle, trichlorométhyle, chlorodifluorométhyle, fluorodichlorométhyle, méthoxy, éthoxy, n- ou i-propoxy, n-, i-, s- ou t-butoxy, difluorométhoxy, trifluorométhoxy, méthylthio, éthylthio, n- ou i-propylthio, n-, i-, s- ou t-butylthio, difluorométhylthio, trifluorométhylthio, méthoxycarbonyle, éthoxycarbonyle, n- ou t-propoxycarbonyle, le groupement -N=(hétérocyClyle), hétérocyClylalkyle, hétérocyClylalcoxy ou hétérocyClylalkylamino de la série des groupes furyle, tétrahydrofuryle, furyloxy, tétrahydrofuryloxy, furylamino, tétrahydrofurylamino, furylméthyle, tétrahydrofurylméthyle, furylméthoxy, tétrahydrofurylméthoxy, furylméthylamino, tétrahydrofurylméthylamino, dioxolanyle, dioxolanylméthyle, dioxoanylméthoxy, dioxolanylméthylamino, thiényle, thiénylamino, thiénylméthyle, thiénylméthylamino, dithiolanyle, dithiolanylméthyle, dithiolanylméthoxy, dithiolanylméthylamino, pyrrolidinyle, pyrrolidinylamino, oxopyrrolidinyle, pyrrolyle, indolyle, pyrrolylméthyle, pyrazolyle, pyrazolyloxy, pyrazolylamino, pyrazolylméthyle, imidazolyle, imidazolinyle, imidazolylméthyle, oxoimidazolinyle, 2-oxo-1,3-diaza-cyClopentyle, oxazolyle, oxazolylamino, dihydrooxazolyle (oxazolinyle), tétrahydrooxazolyle, (oxazolidinyle), isoxazolyle, isoxazolylamino, dihydroisooxazolyle (isoxazolinyle), tétrahydroisoxazolyle (isoxazolidinyle), tétrahydro-(2H)-1,2-oxazin-2-yle, oxazolylméthyle, thiazolyle, thiazolylamino, thiazolylméthyle, dihydrothiazolyle (thiazolinyle), tétrahydrothiazolyle (thiazolidinyle), thiazolimino, thiazolinylthio, thiazolinylamino, oxothiazolidinyle, cyanoiminothiazolidinyle, oxadiazolylamino, thiadiazolylamino, triazolylamino, oxotriazolinyle, thioxotriazolinyle, oxatétrazolinyle, oxotétrazolinylméthyle, tétrazolylméthyle, dioxanyle, dioxanylméthyle, dioxanylméthoxy, dioxanylméthylamino, dithianyle, dithianylméthyle, dithianylméthoxy, dithianylméthylamino, pipéridinyle, pipéridinylamino, oxopipéridinyle, 2-oxo-1,3-diaza-cyClohexyle, 2-oxo-1-aza-cyCloheptyle, 2-oxo-1,3-diaza-cyCloheptyle, morpholinyle, morpholinylamino, pipérazinyle, pyridinyle, pyridinyloxy, pyridinylthio, pyridinylamino, 2-(1H)-pyridinimino, pyridinylméthyle, pyridinylméthoxy, pyrimidinyle, pyrimidinyloxy, pyrimidinylthio, pyrimidinylamino, pyrimidinylméthyle ou pyrimidinylméthoxy,
comprenant toutes les formes tautomères possibles des composés de la formule générale (I) et les sels possibles des composés de la formule générale (I).

2. Composés de la formule (I) selon la revendication 1, **caractérisés en ce que**
R¹ représente l'hydrogène, un groupe méthyle, éthyle, n- ou i-propyle, méthylthio, éthylthio, n- ou i-propylthio à chaque fois éventuellement substitué par du fluor ou du chlore; ou un groupe phényle,
R² représente l'hydrogène, un groupe méthyle, éthyle, n- ou i-propyle à chaque fois éventuellement substitué par du fluor ou du chlore, ou représente éventuellement également avec R¹ un groupe éthan-1,2-diyle, propan-1,3-diyle ou butan-1,4-diyle,
R³ représente l'hydrogène, un groupe nitro, cyano, le fluor, le chlore, le brome, l'iode, un groupe méthyle, éthyle, n- ou i-propyle, difluorométhyle, trifluorométhyle, dichlorométhyle, trichlorométhyle, méthoxyméthyle, méthylthiométhyle, méthylsulfinylméthyle, méthylsulfonylméthyle, méthoxy, éthoxy, difluorométhoxy, trifluorométhoxy, méthythio, éthylthio, méthylsulfinyle, éthylsulfinyle, méthylsulfonyle, éthylsulfonyle ou diméthylaminosulfonyle,
R⁴ représente l'hydrogène, un groupe nitro, cyano, le fluor, le chlore, le brome, l'iode, un groupe méthyle, éthyle, n- ou i-propyle, difluorométhyle, trifluorométhyle, dichlorométhyle, trichlorométhyle, méthoxyméthyle, méthylthiométhyle, méthylsulfinylméthyle, méthylsulfonylméthyle, méthoxy, éthoxy, difluorométhoxy, trifluorométhoxy, méthylthio, éthylthio, méthylsulfinyle, éthylsulfinyle, méthylsulfonyle, éthylsulfonyle ou diméthylaminosulfonyle,
R⁵ représente l'hydrogène, un groupe méthyle, éthyle, n- ou i-propyle, n-, i-butyle à chaque fois éventuellement substitué par un groupe cyano, du fluor, du chlore, un groupe méthoxy ou éthoxy ; un groupe méthoxy, éthoxy, n- ou i-propoxy, méthylthio, éthylthio, n- ou i-propylthio, méthylsulfinyle, éthylsulfinyle, n- ou i-propylsulfinyle, méthylsulfonyle, éthylsulfonyle, méthylamino, éthylamino, n- ou i-propylamino ou diméthylamino ; un groupe propényle, butényle, propinyle ou butinyle à chaque fois éventuellement substitué par du fluor et/ou du chlore ; un groupe cyClopropyle, cyClobutyle, cyClopentyle, cyClohexyle, cyClopropylméthyle, cyClobutylméthyle, cyClopentylméthyle ou cyClohexylméthyle à chaque fois éventuellement substitué par un groupe cyano, du fluor, du chlore ou un groupe méthyle ; ou un groupe phényle, phénylthio, phénylsulfinyle, phénylsulfonyle, phénylméthyle ou phényléthyle à chaque fois éventuellement substitué par un groupe nitro, cyano, du fluor, du chlore, du brome, un groupe méthyle, éthyle, n- ou i-propyle, n-, i-, s- ou t-butyle, trifluorométhyle, méthoxy, éthoxy, n- ou i-propoxy, difluorométhoxy ou trifluorométhoxy ; ou le groupement -C(Q)-Z,
Y représente un groupe hydroxy ; un groupe méthoxy, éthoxy, n- ou i-propoxy, méthylthio, éthylthio, n- ou i-propylthio, méthylsulfinyle, éthylsulfinyle, méthylsulfonyle, éthylsulfonyle, acétyloxy, propionyloxy, n- ou i-butyroyloxy, méthoxycarbonyloxy, éthoxycarbonyloxy, n- ou i-propoxycarbonyloxy, méthylaminocarbonyloxy, éthylaminocarbonyloxy, n- ou i-propylaminocarbonyloxy, méthylsulfonyloxy, éthylsulfonyloxy, n- ou i-propylsulfonyloxy à chaque fois éventuellement substitué par du fluor et/ou du chlore; un groupe propényloxy, butényloxy, propinyloxy ou butinyloxy à chaque fois éventuellement substitué par du fluor, du chlore et/ou du brome ; un groupe phényloxy, phénylthio, phénylsulfinyle, phénylsulfonyle, phénylcarbonyloxy, phénylcarbonylméthoxy, phénylsulfonyloxy, phénylméthoxy, phénylméthylthio, phénylméthylsulfinyle ou phénylméthylsulfonyle à chaque fois éventuellement substitué par un groupe nitro, cyano, du fluor, du chlore, du brome, un groupe méthyle, éthyle, n- ou i-propyle, n-, i-, s- ou t-butyle, trifluorométhyle, méthoxy, éthoxy, n- ou i-propoxy, difluorométhoxy, trifluorométhoxy, méthylthio, éthylthio, n- ou i-propylthio, difluorométhylthio, trifluorométhylthio, méthylsulfinyle, éthylsulfinyle, trifluorométhylsulfinyle, méthylsulfonyle, éthylsulfonyle ou trifluorométhylsulfonyle, et
Z représente un groupe amino, cyanoamino, hydrazino,
un groupe méthyle substitué par un groupe cyano, du fluor, du chlore, un groupe méthoxy, éthoxy, n- ou i-propoxy, méthylthio, éthylthio, n- ou i-propylthio, méthylsulfinyle, éthylsulfinyle, n- ou i-propylsulfinyle, méthylsulfonyle ou éthylsulfonyle,
un groupe éthényle, propényle, butényle, , éthinyle, propinyle, butinyle, propényloxy, butényloxy, propénylthio, buténylthio, propénylamino, buténylamino, propényloxyamino, butényloxyamino, éthinyle, propinyle, butinyle, propinyloxy, butinyloxy, propinylamino ou butinylamino à chaque fois éventuellement substitué par du fluor et/ou du chlore,
un groupe cyClopropyle, cyClobutyle, cyClopentyle, cyClohexyle, cyClopropyloxy, cyClobutyloxy, cyClopentyloxy, cyClohexyloxy, cyClopropylamino, cyClobutylamino, cyClopentylamino, cyClohexylamino, cyClopentylhydrazino, cyClohexylhydrazino, cyClopropylméthyle, cyClobutylméthyle, cyClopentylméthyle, cyClohexyméthyle, cyClopropylméthoxy, cyClobutylméthoxy, cyClopentylméthoxy, cyClohexylméthoxy, cyClopropylméthylamino, cyClobutylméthylamino, cyClopentylméthylamino ou cyClohexylméthylamino à chaque fois éventuellement substitué par un groupe cyano, du fluor, du chlore ou un groupe méthyle,
un groupe phényle, phénylcarbonyle, phénoxy, phénoxycarbonyle, phénylthio, phénylamino, phénylhydrazino, phénylméthyle, phényléthyle, phénylméthoxy, phényléthoxy, phénylméthylthio, phényléthylthio, phénylméthylamino ou phényléthylamino à chaque fois éventuellement substitué par un groupe nitro, cyano, du fluor, du chlore, du brome, un groupe méthyle, éthyle, n- ou i-propyle, n-, i-, s-, ou t-butyle, trifluorométhyle, méthoxy, éthoxy, n- ou i-propoxy, difluorométhoxy, trifluorométhoxy, méthoxycarbonyle, éthoxycarbonyle, n- ou i-propoxycarbonyle, ou
un groupe hétérocyClyle, hétérocyClyloxy, hétérocyClylamino, monocyClique ou bicyClique à chaque fois éventuellement substitué par un groupe nitro, cyano, du fluor, du chlore, du brome, un groupe méthyle, éthyle, n- ou i-propyle, n-, i-, s- ou t-butyle, difluorométhyle, trifluorométhyle, dichlorométhyle, trichlorométhyle, chlorodifluorométhyle, fluorodichlorométhyle, méthoxy, éthoxy, n- ou i-propoxy, difluorométhoxy, trifluorométhoxy, méthylthio, éthylthio, n- ou i-propylthio, difluorométhylthio, trifluorométhylthio, méthoxycarbonyle, éthoxycarbonyle, n- ou t-propoxycarbonyle ; le groupement -N=(hétérocyClyle), hétérocyClylalkyle, hétérocyClylalcoxy ou hétérocyClylalkylamino de la série des groupes furyle, tétrahydrofuryle, furyloxy, tétrahydrofuryloxy, furylamino, tétrahydrofurylamino, furylméthyle, tétrahydrofurylméthyle, furylméthoxy, tétrahydrofurylméthoxy, furylméthylamino, tétrahydrofurylméthylamino, dioxolanyle, dioxolanylméthyle, dioxoanylméthoxy, dioxolanylméthylamino, thiényle, thiénylméthyle, dithiolanyle, dithiolanylméthyle, dithiolanylméthoxy, dithioanylméthylamino, pyrrolidinyle, pyrrolidinylamino, oxopyrrolidinyle, pyrrolyle, indolyle, pyrrolylméthyle, pyrazolyle, pyrazolyloxy, pyrazolylamino, pyrazolylméthyle, imidazolyle, imidazolinyle, imidazolylméthyle, imidazolinylméthyle, oxoimidazolinyle, 2-oxo-1,3-diaza-cyClopentyle, oxazolyle, oxazolylméthyle, dihydrooxazolyle (oxazolinyle), tétrahydroxazolyle (oxazolidinyle), isoxazolyle, dihydroisoxazolyle (isoxazolinyle), tétrahydroi-(2H)-1,2-oxazin-z-yle, tétrahydroixoxazolyle (isoxazolidinyle), thiazolyle, thiazolylméthyle, dihydrothiazolyle (thiazolinyle), tétrahydrothiazolyle (thiazolidinyle), thiazolimino, thiazolinylthio, thiazolinylamino, oxothiazolidinyle, cyanoiminothiazolidinyle, oxadiazolylamino, thiadiazolylamino, oxotriazolinyle, oxatétrazolinylméthyle, tétrazolylméthyle, oxotétrazolinyle, oxotétrazolinylméthyle, tétrazolylméthyle, dioxanyle, dioxanylméthyle, dioxanylméthoxy, dioxanylméthylamino, dithianyle, dithianylméthyle, dithianylméthoxy, dithianylméthylamino, triazolylamino, pipéridinyle, pipéridinylamino, 2-(1H)-pyridinimino, oxopipéridinyle, 2-oxo-1,3-diazacyClohexyle, 2-oxo-1-azacyCloheptyle, 2-oxo-1,3-diaza-cyCloheptyle, morpholinyle, morpholinylamino, pipérazinyle, pyridinyle, pyridinyloxy, pyridinylamino, pyridinylméthyle, pyridinylméthoxy, pyrimidinyle, pyrimidinyloxy, pyrimidinylméthyle ou pyrimidinylméthoxy.

3. Composés de la formule (I) selon la revendication 1 ou 2, **caractérisés en ce que**
R¹ et R² représentent l'hydrogène,
R³ représente l'hydrogène, le fluor, le chlore, un groupe méthyle, éthyle, trifluorométhyle, trichlorométhyle, méthoxy, éthoxy, méthylsulfonyle ou éthylsulfonyle,
R⁴ représente l'hydrogène, le fluor, le chlore, un groupe méthyle, éthyle, trifluorométhyle, trichlorométhyle, méthoxy, éthoxy, méthylsulfonyle ou éthylsulfonyle,
R⁵ représente l'hydrogène, et
Y représente un groupe hydroxyle.

4. Composés de la formule (1-2) 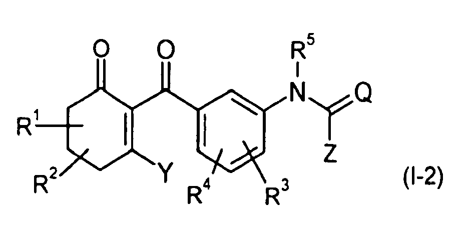 dans laquelle Q, R¹, R², R³, R⁴, R⁵ et Z ont une des significations indiquées dans l'une quelconque des revendications 1 à 3.

5. Procédé pour la préparation de composés de la formule (I) selon l'une quelconque des revendications 1 à 3 et de la formule (I-2) selon la revendication 4, **caractérisé en ce que** l'on fait réagir une cyClohexanedione de la formule générale 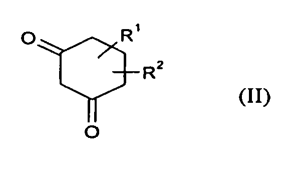 dans laquelle
R¹ et R² ont la signification indiquée dans l'une des revendications 1 à 3,
avec des acides benzoïques substitués de la formule générale (III), 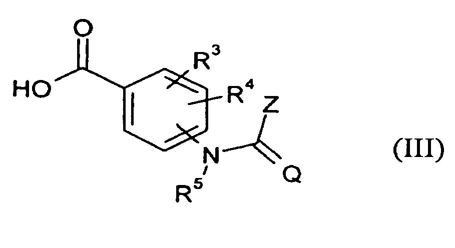 dans laquelle
Q, R³, R⁴, R⁵ et Z ont la signification indiquée dans l'une quelconque des revendications 1 à 3,
où avec des dérivés de ceux-ci capables de réagir, éventuellement en présence d'un agent de déshydratation, éventuellement en présence d'un ou plusieurs auxiliaires de réaction et éventuellement en présence d'un ou plusieurs agents diluants,
et **en ce que** l'on réalise éventuellement ensuite des réactions de substitution électrophiles ou nuCléophiles Classiques dans le cadre de la définition des substituants respectivement des réactions d'oxydation ou de réduction sur les composés de la formule (I) ainsi obtenus ou **en ce que** l'on transforme les composés de la formule (I) de manière Classique en sels.

6. Agents herbicides, **caractérisés en ce qu'**ils contiennent au moins un composé de la formule (I) selon l'une quelconque des revendications 1 à 3 et des diluants Classiques.

7. Utilisation d'au moins un composé selon l'une quelconque des revendications 1 à 3 ou d'un agent selon la revendication 6 pour combattre des plantes non souhaitées.

8. Procédé pour combattre des plantes non souhaitées, **caractérisé en ce que** l'on laisse agir au moins un composé selon l'une quelconque des revendications 1 à 3 ou un agent selon la revendication 6 sur les plantes non souhaitées ou leur biotope.
